# EUROPEAN PATENT SPECIFICATION

(11) **EP 1 543 324 B1**
(45) Date of publication and mention of the grant of the patent: **21.11.2007**
(21) Application number: 03724564.4
(22) Date of filing: 12.05.2003
(51) Int. Cl.: G01N 33/53

(54) **METHODS AND COMPOSITIONS FOR GRAFTING FUNCTIONAL LOOPS INTO A PROTEIN**
VERFAHREN UND ZUSAMMENSETZUNGEN ZUR IMPLANTATION VON FUNKTIONALEN SCHLEIFEN IN EIN PROTEIN
PROCEDES ET COMPOSITIONS POUR LA GREFFE DE BOUCLES FONCTIONNELLES DANS UNE PROTEINE

(30) Priority: 12.06.2002 US 170387
(43) Date of publication of application: 22.06.2005
(73) Proprietor: GENENCOR INTERNATIONAL, INC., Palo Alto, California 94304 (US)
(72) Inventor: MURRAY, Christopher, J., Soquel, CA 95073 (US)
(74) Representative: Kiddle, Simon John
(86) International application number: PCT/US2003/015017
(87) International publication number: WO 2003/105753

(56) References cited:
- WO-A-02/08779
- US-A- 5 834 247
- ROSS-MACDONALD PETRA ET AL: "A multipurpose transposon system for analyzing protein production, localization, and function in Saccharomyces cerevisiae" PROCEEDINGS OF THE NATIONAL ACADEMY OF SCIENCES OF USA, NATIONAL ACADEMY OF SCIENCE, WASHINGTON, DC, US, vol. 94, no. 1, 1997, pages 190-195, XP002148084 ISSN: 0027-8424
- HAYES FINBARR ET AL: "Insertion mutagenesis as a tool in the modification of protein function. Extended substrate specificity conferred by pentapeptide insertions in the OMEGA-loop of TEM-1 beta-lactamase" JOURNAL OF BIOLOGICAL CHEMISTRY, vol. 272, no. 46, 14 November 1997 (1997-11-14), pages 28833-28836, XP002349313 ISSN: 0021-9258
- HAYES F ET AL: "Pentapeptide scanning mutagenesis: encouraging old proteins to execute unusual tricks" TRENDS IN MICROBIOLOGY, ELSEVIER SCIENCE LTD., KIDLINGTON, GB, vol. 8, no. 12, December 2000 (2000-12), pages 571-577, XP002241076 ISSN: 0966-842X
- GALARNEAU A ET AL: "Beta-lactamase protein fragment complementation assays as in vivo and in vitro sensors of protein protein interactions" NATURE BIOTECHNOLOGY, NATURE PUBLISHING, US, vol. 20, no. 6, June 2002 (2002-06), pages 619-622, XP002279639 ISSN: 1087-0156
- LU Z ET AL: "EXPRESSION OF THIOREDOXIN RANDOM PEPTIDE LIBRARIES ON THE ESCHERICHIA COLI CELL SURFACE AS FUNCTIONAL FUSIONS TO FLAGELLIN: A SYSTEM DESIGNED FOR EXPLORING PROTEIN-PROTEIN INTERACTIONS" BIO/TECHNOLOGY, NATURE PUBLISHING CO. NEW YORK, US, vol. 13, April 1995 (1995-04), pages 366-372, XP002033346 ISSN: 0733-222X
- CHIANG S L ET AL: "Construction of a mariner-based transposon for epitope-tagging and genomic targeting" GENE: AN INTERNATIONAL JOURNAL ON GENES AND GENOMES, ELSEVIER SCIENCE PUBLISHERS, BARKING, GB, vol. 296, no. 1-2, 21 August 2002 (2002-08-21), pages 179-185, XP004386954 ISSN: 0378-1119
- VERSHAVSKY A.: 'CODOMINANCE AND TOXINS:A PATH TO DRUGS OF NEARLY UNLIMITED SELECTIVITY' PNAS vol. 92, no. 9, April 1995, pages 3663 - 3667, XP002920219
- VOSS S. AND SKERRA A.: 'Mutagenesis of a flexible loop in streptavidin leads to higher affinity for the Strep-tag II peptide and improved performance in recombinant protein purification' PROTEIN ENGINEERING vol. 10, no. 8, 1997, pages 975 - 982, XP002149254

## Description

### FIELD OF THE INVENTION

The present invention provides targeted enzymes that bind to targets better than the corresponding pre-targeted enzymes bind the target under like conditions, methods of making targeted enzymes, and pharmaceutical compositions comprising targeted enzymes.

### BACKGROUND OF THE INVENTION

Enzymes conjugated or fused to a targeting moiety have many diagnostic and therapeutic uses. For example, most homogeneous drug detection immunoassays utilize an enzyme conjugated to a drug metabolite. *See, e.g.,* Rubinstein, et al., Biochem. Biophys, Res. Commun. 47:846 (1972). More recently, Legendre, *et al.* describe an updated version of the homogenous immunoassay. Legendre et al., 1999, Nat. Biotechnol. 17:67-72; Yamada et al., 1995, J. Biol. Chem. 270:5687. Ross-MacDonald describe the use of a transposon system for epitope tagging proteins in yeast. Ross-MacDonald et al., 1997, Proc. Natl. Acad. Sci. USA, 94: 190-195.

In the therapeutic arena, antibody-enzyme conjugates have been studied and described. However, from these studies, several deficiencies have become apparent. Some of these deficiencies include: slow diffusion into tumors, slow clearance from circulation; non-specific binding to other tissues; elicitation of an immune response; difficulties in production, conjugation or expression; and the required coupling between targeting and catalytic function.

One type of approach that has been used is antibody-directed enzyme prodrug therapy (ADEPT). ADEPT and similar procedures are multistep methods designed to increase the selectivity of antitumor agents. *See, e.g.,* Philpott et al., J Immunol 111:921 (1973), Bagshawe et al., Curr Opin Immunol 11:579 (1999), Niculescu-Duvaz et al., Anticancer Drug Des 14:517 (1999), Chari Adv Drug Deliv Rev 31:89 (1998), Syrigos & Epenetos Anticancer Res 19:605 (1999), Sherwood, Advanced Drug Del. Rev. 22:269 (1996) and Niculescu-Duvaz & Springer, Advanced Drug Del. Rev. 26:151 (1997). Immunogenicity of the antibody-enzyme conjugate, however, is a key limitation of existing ADEPT approaches.

Another limitation of existing ADEPT methods is the long half-live of the antibody-enzyme conjugate in the circulation. In general, antibodies have long half-lives in the circulation and this property is conferred to the antibody-enzyme conjugates. The antibody-enzyme conjugate must be removed from non-tumor sites of the body before the prodrug can be administered to prevent drug activation in other tissues. One method for removing excess antibody-enzyme conjugate requires the administration of a second antibody which is typically directed against the enzyme portion of the antibody-enzyme conjugate. *See* Kerr et al., Bioconjug Chem 4:353 (1993). Another method to accelerate clearance of excess enzyme is to construct a fusion protein between a single chain antibody fragment (scFv) and an enzyme that can be rapidly cleared through the kidney due to its relatively low molecular weight and because it lacks the Fc-binding portion of the corresponding antibody. *See* Siemers et al., 1997, Bioconjug Chem 8:510-9.

In response to the shortcomings of ADEPT, other strategies for specifically activating a prodrug at a target site in a subject have been developed. In gene-directed enzyme prodrug therapy (GDEPT), the gene that encodes the prodrug activating enzyme is delivered to the tumor. For a recent review, see Niculescu-Duvaz et al., Anticancer Drug Des 14:517 (1999). However, the utility of GDEPT is severely limited by the requirement that a safe and effective method be developed of introducing the required gene into the tumor to be treated. In another approach, antitumor agents are terminally coupled to a targeting agent. For reviews of this technology, *see* Torchilin, Eur JPharm Sci 11 Suppl 2:S81 (2000), and Frankel et al., Clin Cancer Res 6:326 (2000). These approaches suffer from some of the same shortcomings as ADEPT. In particular, these therapeutics can be immunogenic, and the combined size of the targeting moiety, the enzyme and the linker (if one is used) can cause them to have a prohibitively long half-life in the circulation of the subject.

Thus, there remains in the art a need for targeted enzymes that are relatively easy to create, bind with high affinity to a target, exhibit catalytic activity when bound to the target, and when utilized in a subject, have the physicochemical properties of rapid diffusion, low immunogenicity and rapid clearance. This invention meets these and other needs.

### SUMMARY OF THE INVENTION

The present invention describes the surprising generation of a targeted enzyme that has catalytic activity while bound to a target that the pre-targeted enzyme binds with lower affinity, and methods for making such targeted enzymes. It has unexpectedly been discovered that one or more peptides can be inserted into a protein without inactivating the protein. For example, one or more peptides can be inserted into one or more sites of an enzyme such that the modified enzyme binds to a target better than the pre-modified enzyme binds to the target and retains significant enzymatic activity. The enzymes of the current invention may be used in therapy or diagnostics.

In one aspect, the present invention provides a method of imparting a function to a protein, comprising
a) inserting a peptide into a protein having a measurable property,
b) selecting the protein with the inserted peptide if it has the measurable property,
c) replacing the inserted peptide in the selected protein with a functional peptide having the function, wherein the protein with the functional peptide has the measurable property and the detectable function.

In another aspect, the present invention provides a method of imparting a function to a protein, comprising
a) inserting a peptide into the protein at a random position, wherein the protein has a measurable property,
b) selecting the protein with the inserted peptide if it has the measurable property,
c) replacing the inserted peptide in the protein with a functional peptide having the function, wherein the protein with the functional peptide has the measurable property and the function.

In another aspect, the present invention provides a method of imparting a function to a protein, comprising
a) selecting a protein having an inserted peptide and a measurable property from a library of proteins, said library comprising proteins that have an inserted peptide that a corresponding unmodified protein does not have, and said unmodified protein has the measurable property, and
b) replacing the inserted peptide in the selected protein with a peptide having a function, wherein the protein with the functional peptide has the measurable property and the function.

In another aspect, the present invention provides a method of imparting a new function to a protein, comprising
a) selecting a protein having an inserted peptide and a measurable property from a library of proteins, said library comprising proteins that have an inserted peptide that a corresponding unmodified protein does not have, and said unmodified protein has the measurable property,
b) identifying the point of insertion of the peptide in the selected protein, and
c) inserting a peptide having a function into the unmodified protein at the point of insertion identified in (b), wherein the protein with the functional peptide has the measurable property and the function.

In another aspect, the present invention provides a method of making a protein with a function, comprising
a) inserting a peptide into a protein having a measurable property,
b) selecting the protein with the inserted peptide if it has the measurable property,
c) identifying the point of insertion of the peptide in the protein of step (b), and
d) inserting a functional peptide into the protein at the point of insertion identified in step (c), wherein the protein with the functional peptide has the measurable property and the function.

In another aspect, the present invention provides a method of making a protein with a function, comprising
a) inserting a foreign peptide into a protein having a measurable property,
b) selecting the protein with the inserted foreign peptide if it has the measurable property,
c) identifying the point of insertion of the foreign peptide in the protein of step (b),
d) repeating steps (a)-(c), as necessary, to identify a plurality of points of insertion, and
e) inserting at least one functional peptide into the protein at at least one of the points of insertion identified in steps (a)-(d), wherein the protein with the one or more inserted functional peptide has the measurable property and the function.

In another aspect, the present invention provides a method of making a targeted enzyme, comprising
a) making a modified enzyme by inserting a peptide into an unmodified enzyme, wherein the unmodified enzyme has a measurable enzymatic activity,
b) selecting the modified enzyme if it has the measurable enzymatic activity, and
c) replacing the inserted peptide in the modified enzyme with a targeting peptide that binds a target, wherein the enzyme with the targeting peptide has the measurable enzymatic activity and binds the target.

In another aspect, the present invention provides a method of making a targeted enzyme, comprising
a) making a modified enzyme by inserting a peptide into an unmodified enzyme at a randomly determined position, wherein the unmodified enzyme has a measurable enzymatic activity,
b) selecting the modified enzyme if it has the measurable enzymatic activity, and
c) replacing the inserted peptide in the modified enzyme with a targeting peptide that binds a target, wherein the enzyme with the targeting peptide has the measurable enzymatic activity and binds the target.

In another aspect, the present invention provides a method of making a targeted enzyme, comprising
a) selecting an enzyme having an inserted peptide and a measurable enzymatic activity from a library of enzymes, said library comprising enzymes that have an inserted peptide that a corresponding unmodified enzyme does not have, and said unmodified enzyme has the measurable enzymatic activity, and
b) replacing the inserted peptide in the selected modified enzyme with a binding peptide that binds a target, wherein the enzyme with the binding peptide has the measurable enzymatic activity and binds the target.

In another aspect, the present invention provides a method of making a targeted enzyme, comprising
a) selecting an enzyme having an inserted peptide and a measurable enzymatic activity from a library of enzymes, said library comprising enzymes that have an inserted peptide that a corresponding unmodified enzyme does not have, and said unmodified enzyme has the measurable enzymatic activity,
b) identifying the point of insertion of the peptide in the selected modified enzyme, and
c) inserting a binding peptide that binds a target into the unmodified enzyme at the point of insertion identified in (b), wherein the enzyme with the binding peptide has the measurable enzymatic activity and the function.

In another aspect, the present invention provides a method of making a targeted enzyme, comprising
a) inserting a foreign peptide into an unmodified enzyme having a measurable enzymatic activity,
b) selecting the enzyme with the inserted foreign peptide if it has the measurable enzymatic activity,
c) identifying the point of insertion of the foreign peptide in the enzyme of step (b), and
d) inserting a binding peptide into the unmodified enzyme at the point of insertion identified in step (c), wherein the binding peptide binds a target and the enzyme with the functional peptide has the measurable enzymatic activity and binds the target.

In another aspect, the present invention provides a method of making a targeted enzyme, comprising
a) inserting a foreign peptide into an enzyme having a measurable enzymatic activity,
b) selecting the enzyme with the inserted foreign peptide if it has the measurable enzymatic activity,
c) identifying the point of insertion of the foreign peptide in the enzyme of step (b),
d) repeating steps (a)-(c), as necessary, to identify a plurality of points of insertion, and
e) inserting at least one binding peptide into the enzyme at at least one of the points of insertion identified in steps (a)-(d), wherein the enzyme with the one or more inserted binding peptides has the measurable property and binds the target.

In another aspect, the present invention provides a method of making a targeted enzyme, comprising
a) creating a library of modified enzymes by inserting a library of peptides into an enzyme, wherein the enzyme has an enzymatic activity, and
b) selecting a modified enzyme from the library of modified enzymes that has the enzymatic activity and binds to a target better than the unmodified enzyme binds to the target,
wherein the modified enzyme selected in (b) is the targeted enzyme.

In one embodiment, the peptide, foreign peptide or library of peptides is inserted into a pre-selected site in the protein or enzyme. In another embodiment, the peptide, foreign peptide or library of peptides is inserted into a random site in the enzyme.

In another embodiment the modified enzyme is a targeted enzyme. In a more particularly defined embodiment, the targeted enzyme is useful in targeted enzyme prodrug therapy.

The compositions and methods of the present invention offer several advantages over previously available compositions and methods. The targeted enzymes of the invention can be smaller than similar enzymes conjugated or fused to an antibody or antibody fragment, thus, when administered to a subject, targeted enzymes not bound to their targets are more quickly eliminated from the subject's system. Their reduced size also makes them less immunogenic, and allows them greater access to their target sites. The methods of the present invention for making targeted enzymes are superior to previously known methods because, in one aspect, they allow for selection of a modified enzyme that is about the same size as, but binds to a target better than, the unmodified enzyme without knowing the three-dimensional structure of the enzyme.

### BRIEF DESCRIPTION OF THE FIGURES

Figure 1 presents the sequence of the p99 β-lactamase of *E. cloacae.*
Figure 2 presents a scheme for the creation of a targeted loop library.
Figure 3 presents a scheme for creating targeted enzymes using Phoenix mutagenesis.
Figure 4 presents a scheme for creating targeted enzyme using iterative assembly.
Figure 5 presents a diagram of plasmid pTDS004.
Figure 6 illustrates a scheme for modifying a variation-tolerant sequence of a pre-targeted enzyme
Figure 7 illustrates a scheme for the random recombination of pre-selected repetoires.
Figure 8 presents a diagram of plasmid pCB04WT.

### DETAILED DESCRIPTION OF THE INVENTION

Unless defined otherwise, all technical and scientific terms used herein have the same meaning as commonly understood by one of ordinary skill in the art to which this invention belongs. All references are incorporated by reference for all purposes. Although any methods and materials similar or equivalent to those described herein can be used in the practice or testing of the present invention, the preferred methods and materials are described. For purposes of the present invention, the following terms are defined below.

All single-stranded nucleic acid sequences are written from 5' to 3', unless otherwise indicated. The top strand of each double-stranded nucleic acid sequence is written from 5' to 3' and the bottom strand from 3' to 5', unless otherwise indicated. All peptide sequences are written N-terminus to C-terminus, unless otherwise indicated. Standard one-letter amino acid and nucleic acid abbreviations are used throughout, unless otherwise indicated. In an amino acid sequence, "X" indicates a position that can be occupied by any amino acid residue, preferably a naturally-occurring amino acid residue.

The term "targeted enzyme" refers to an enzyme exhibiting catalytic activity that comprises a substrate recognition site and has been modified from a pre-targeted enzyme to comprise one or more targeting sites, each targeting site comprising one or more variant sequences, and to bind to a target with higher affinity than the corresponding pre-targeted enzyme binds the target under like conditions. Targeted enzymes of the invention include modified enzymes that bind to a target that the corresponding pre-targeted enzyme does not bind to under like conditions. Targeted enzymes of the invention also include modified enzymes that bind to a target with about 10-fold, 10²-fold, 10³-fold, 10⁴-fold, 10⁵-fold or higher affinity than the corresponding pre-targeted enzyme under like conditions. Targeted enzymes of the invention do not include enzymes with a targeting site that consists entirely of a polypeptide or other target-binding molecule that is attached to the N- or C-terminus of the pre-targeted enzyme (*e.g.*, as in a histidine tagged protein or a fusion protein), a targeted enzyme whose only target is a monoclonal antibody, or a targeted enzyme made by increasing or optimizing the binding of a pre-targeted enzyme to a substrate of a reaction catalyzed by the pre-targeted enzyme. However, a targeted enzyme of the invention can be further modified to include a polypeptide or other targeting molecule that is attached to the N- or C-terminus. A targeted enzyme also can be further modified to change or optimize binding to a substrate of a reaction catalyzed by the targeted enzyme.

The term "pre-targeted enzyme" refers to a protein having a catalytic activity and comprising a substrate recognition site and a variation-tolerant sequence. The protein can be, *e.g.,* a naturally-occurring, modified, artificial, chimeric or fusion protein.

The termn "target" refers to any entity a protein can be made to bind.

The term "targeting site" refers to a portion of a targeted enzyme that binds a target. A targeting site comprises one or more variant sequences. It does not consist solely of a protein binding domain copied from another protein and introduced into the targeted enzyme, does not consist entirely of a variant sequence in a protein-binding domain of the pre-targeted enzyme, and does not consist entirely of a substrate recognition site.

The term "variant sequence" refers to one or more contiguous amino acid residues derived from, but not identical to, a variation-tolerant sequence of a pre-targeted enzyme. A variant sequence is derived from a variation-tolerant sequence in that the variant sequence differs from its corresponding variation-tolerant sequence by the insertion, deletion, substitution or replacement of one or more amino acid residues of the variation-tolerant sequence. Thus, a variant sequence has 0% or more, but less than 100%, sequence identity to the corresponding variation-tolerant sequence, and can be shorter, the same length, or longer than the variation-tolerant sequence.

The term "variation-tolerant sequence" refers to one or more contiguous amino acid residues in an enzyme that can be modified to a different sequence without inactivating the catalytic activity of the enzyme. A variation-tolerant sequence can be, for example, one or more amino acid residues that can be replaced by one or more different amino acid residues, or two amino acid residues that can be separated by the insertion of one or more amino acid residues.

The term "substrate recognition site" refers to the amino acid residues of an enzyme that contact a substrate of a reaction catalyzed by the enzyme.

The term "protein binding domain" refers to the amino acid residues of a protein that contact one or more amino acid residues of a second protein wherein said protein binding domain is not a substrate recognition site.

A "repertoire of variant sequences" is a plurality of variant sequences each of which can be used to modify the same variant sequence of a pre-targeted enzyme.

A "recombinant library" is a plurality of proteins that are derived from the same pre-targeted enzyme. The members of a recombinant library share the same constant segments but they contain different combinations of variant sequences.

A "measurable enzymatic activity" is an enzymatic activity that can be detected using any method known in the art. A protein with a measurable enzymatic activity is one that exhibits said enzymatic activity at a level that can be detected using methods known in the art.

Unless otherwise noted, the term "protein" is used interchangeably here with the terms "peptide" and "polypeptide," and refers to a molecule comprising two or more amino acid residues joined by a peptide bond.

The terms "cell", "cell line", and "cell culture" can be used interchangeably and all such designations include progeny. Thus, the words "transformants" or "transformed cells" include the primary transformed cell and cultures derived from that cell without regard to the number of transfers. All progeny may not be precisely identical in DNA content, due to deliberate or inadvertent mutations. Mutant progeny that have the same functionality as screened for in the originally transformed cell are included in the definition of transformants. The cells can be prokaryotic or eukaryotic.

The term "control sequences" refers to DNA sequences necessary for the expression of an operably linked coding sequence in a particular host organism. The control sequences that are suitable for procaryotes, for example, include a promoter, optionally an operator sequence, a ribosome binding site, positive retroregulatory elements (see, *e.g.*, U.S. Pat. No. 4,666,848, incorporated herein by reference), and possibly other sequences. Eucaryotic cells are known to utilize promoters, polyadenylation signals, and enhancers.

The term "expression clone" refers to DNA sequences containing a desired coding sequence and control sequences in operable linkage, so that hosts transformed with these sequences are capable of producing the encoded proteins. The term "expression system" refers to a host transformed with an expression clone. To effect transformation, the expression clone may be included on a vector; however, the relevant DNA may also be integrated into the host chromosome.

The term "gene" refers to a DNA sequence that comprises control and coding sequences necessary for the production of a protein or polypeptide.

The term "operably linked" refers to the positioning of the coding sequence such that control sequences will function to drive expression of the protein encoded by the coding sequence. Thus, a coding sequence "operably linked" to control sequences refers to a configuration wherein the coding sequences can be expressed under the direction of a control sequence.

The term "oligonucleotide" as used herein is defined as a molecule comprised of two or more deoxyribonucleotides or ribonucleotides. The exact size will depend on many factors, which in turn depends on the ultimate function or use of the oligonucleotide. Oligonucleotides can be prepared by any suitable method, including, for example, cloning and restriction of appropriate sequences and direct chemical synthesis by a method such as the phosphotriester method of Narang et al., 1979, Meth. Enzymol. 68:90-99; the phosphodiester method of Brown et al., 1979, Meth. Enzymol. 68:109-151; the diethylphosphoramidite method of Beaucage et al., 1981, Tetrahedron Lett. 22:1859-1862; and the solid support method of U.S. Pat. No. 4,458,066, each incorporated herein by reference. A review of synthesis methods is provided in Goodchild, 1990, Bioconjugate Chemistry 1(3):165-187, incorporated herein by reference.

The term "primer" as used herein refers to an oligonucleotide which is capable of acting as a point of initiation of synthesis when placed under conditions in which primer extension is initiated. Synthesis of a primer extension product that is complementary to a nucleic acid strand is initiated in the presence of the requisite four different nucleoside triphosphates and a DNA polymerase in an appropriate buffer at a suitable temperature. A "buffer" includes cofactors (such as divalent metal ions) and salt (to provide the appropriate ionic strength), adjusted to the desired pH.

A primer that hybridizes to the non-coding strand of a gene sequence (equivalently, is a subsequence of the coding strand) is referred to herein as an "upstream" or "forward" primer. A primer that hybridizes to the coding strand of a gene sequence is referred to herein as an "downstream" or "reverse" primer.

The terms "restriction endonucleases" and "restriction enzymes" refer to enzymes, typically bacterial in origin, which cut double-stranded DNA at or near a specific nucleotide sequence.

Families of amino acid residues having similar side chains have been defined in the art. These families include amino acids with basic side chains (*e.g.,* lysine, arginine, histidine), acidic side chains (*e.g.*, aspartic acid, glutamic acid), uncharged polar side chains (*e.g.*, asparagine, glutamine, serine, threonine, tyrosine), nonpolar side chains (*e.g*., alanine, valine, leucine, isoleucine, proline, phenylalanine, methionine, tryptophan, cysteine, glycine), beta-branched side chains (e.g., threonine, valine, isoleucine) and aromatic side chains (e.g., tyrosine, phenylalanine, tryptophan, histidine). Standard three-letter or one-letter amino acid abbreviations are used herein.

The peptides, polypeptides and proteins of the invention include those comprising one or more non-classical amino acids. Non-classical amino acids include but are not limited to the D-isomers of the common amino acids, α-amino isobutyric acid, 4-aminobutyric acid (4-Abu), 2-aminobutyric acid (2- Abu), 6-amino hexanoic acid (Ahx), 2-amino isobutyric acid (2-Aib), 3-amino propionoic acid, ornithine, norleucine, norvaline, hydroxyproline, sarcosine, citrulline, cysteic acid, t-butylglycine, t-butylalanine, phenylglycine, cyclohexylalanine, β-alanine, fluoro-amino acids, designer amino acids such as β-methyl amino acids, Cα-methyl amino acids, Nα-methyl amino acids, and amino acid analogs in general.

As used herein, a "point mutation" in an amino acid sequence refers to either a single amino acid substitution, a single amino acid insertion or single amino acid deletion. A point mutation preferably is introduced into an amino acid sequence by a suitable codon change in the encoding DNA. Individual amino acids in a sequence are represented herein as AN, wherein A is the standard one letter symbol for the amino acid in the sequence, and N is the position in the sequence. Mutations within an amino acid sequence are represented herein as A₁ NA₂, wherein A₁ is the standard one letter symbol for the amino acid in the unmutated protein sequence, A₂ is the standard one letter symbol for the amino acid in the mutated protein sequence, and N is the position in the amino acid sequence. For example, a G46D mutation represents a change from glycine to aspartic acid at amino acid position 46. The amino acid positions are numbered based on the full-length sequence of the protein from which the region encompassing the mutation is derived. Representations of nucleotides and point mutations in DNA sequences are analogous.

As used herein, a "chimeric" protein refers to a protein whose amino acid sequence represents a fusion product of subsequences of the amino acid sequences from at least two distinct proteins. A chimeric protein preferably is not produced by direct manipulation of amino acid sequences, but, rather, is expressed from a "chimeric" gene that encodes the chimeric amino acid sequence.

The term "host immune response" refers to a response of a host organism's immune system to contact with an immunogenic substance. Specific aspects of a host immune response can include, *e.g.,* antibody production or formation, T cell activation, monocyte activation or granulocyte activation. Each of these aspects can be detected and/or measured using standard *in vivo* or *in vitro* methods.

The term "Ab" or "antibody" refers to polyclonal and monoclonal antibodies, an entire immunoglobulin or antibody or any functional fragment of an immunoglobulin molecule that binds to the target antigen. Examples of such functional entities include complete antibody molecules, antibody fragments, such as Fv, single chain Fv, complementarity determining regions (CDRs), V_{L} (light chain variable region), V_{H} (heavy chain variable region), and any combination of those or any other functional portion of an immunoglobulin peptide capable of binding to target antigen.

The terms "dox" and "doxorubicin" refer to the drug commonly known by that name and any derivative thereof. Derivatives may be made for a variety of purposes including, but not limited to, conjugating to a linker or pro-part of a prodrug, increased efficacy, increased binding, decreased toxicity, etc. The CAS Registry Number for Doxorubicin is 25316409. The molecular formula is C₂₇H₂₉NO₁₁AHCl and its molecular weight is 580 Daltons.

The term "PEG" and polyethylene glycol" refer to the compounds commonly known by the name and comprising the general chemical formula (C₂H₄O)ₙAH₂O. The CAS Number for PEG is 25322-68-3. As is well known in the art, PEG is typically provided in mixtures of differing molecular weights. For example, PEG-8000 is a mixture of polyethylene glycols that have an average molecular weight of 8,000 Daltons.

The term "prodrug" refers to a compound that is converted via one or more enzymatically catalyzed steps into an active compound that has an increased pharmacological activity relative to the prodrug. A prodrug can comprise a pro-part or inactive moiety and a drug or active drug. Optionally, the prodrug also contains a linker. For example, the prodrug can be cleaved by an enzyme to release an active drug. In a more specific example, prodrug cleavage by the targeted enzyme releases the active drug into the vicinity of the target bound to the targeted enzyme. "Pro-part" and "inactive moiety" refer to the inactive portion of the prodrug after it has been converted. For example, if a prodrug comprises PEG molecule linked by a peptide to an active drug, the pro-part is the PEG moiety with or without a portion of the peptide linker. "Linker" refers to the means connecting the pro-part of a prodrug to the active drug of a prodrug. Typically, but not essentially, the linker is a peptide cleavable by the targeted enzyme, however, it can be any moiety that joins the drug to the propart. The term "drug" and "active drug" refer to the active moieties of a prodrug. After cleavage by a targeted enzyme, the active drug acts therapeutically upon the targeted tumor, cell, infectious agent or other agent of disease. In another example, the prodrug is chemically modified by the activating enzyme, for example, by oxidation, reduction, phosphorylation, dephosphorylation, the addition of a moiety, or the like. In another example, the prodrug is converted into an intermediate compound by the enzyme. The intermediate compound is converted to the active compound either spontaneously, through contact with other proteins or molecules in the subject, through contact with one or more enzymes native to the subject, or through contact with one or more additional activating enzymes administered to the subject.

The term "Serum albumin" refers to the commonly known blood protein of the same name. "BSA" refers to bovine serum albumin and "HSA" refers to human serum albumin.

The term "constant segment" refers to a part of the sequence of the pre-targeted enzyme that shares high homology (> 80% homology) among all members of the recombinant library.

The term "% sequence homology" is used interchangeably herein with the terms "% homology," "% sequence identity" and "% identity" and refers to the level of amino acid sequence identity between two or more peptide sequences, when aligned using a sequence alignment program. For example, as used herein, 80% homology means the same thing as 80% sequence identity determined by a defined algorithm, and accordingly a homologue of a given sequence has greater than 80% sequence identity over a length of the given sequence. Exemplary levels of sequence identity include, but are not limited to, 60, 70, 80, 85, 90, 95, 98% or more sequence identity to a given sequence

Exemplary computer programs which can be used to determine identity between two sequences include, but are not limited to, the suite of BLAST programs, *e.g.,* BLASTN, BLASTX, and TBLASTX, BLASTP and TBLASTN, publicly available on the Internet at http://www.ncbi.nlm.nih.gov/BLAST/". *See also* Altschul et al., 1990, J. Mol. Biol. 215: 403-10 (with special reference to the published default setting, *i.e.,* parameters w=4, t=17) and Altschul et al., 1997, Nucleic Acids Res., 25:3389-3402. Sequence searches are typically carried out using the BLASTP program when evaluating a given amino acid sequence relative to amino acid sequences in the GenBank Protein Sequences and other public databases. The BLASTX program is preferred for searching nucleic acid sequences that have been translated in all reading frames against amino acid sequences in the GenBank Protein Sequences and other public databases. Both BLASTP and BLASTX are run using default parameters of an open gap penalty of 11.0, and an extended gap penalty of 1.0, and utilize the BLOSUM-62 matrix. *See* Altschul, *et al.,* 1997.

A preferred alignment of selected sequences in order to determine "% identity" between two or more sequences, is performed using for example, the CLUSTAL-W program in MacVector version 6.5, operated with default parameters, including an open gap penalty of 10.0, an extended gap penalty of 0.1, and a BLOSUM 30 similarity matrix.

"Hit density" is the fraction of useful clones in the library.

"Hapaxomer" is a restriction endonuclease that generates unique ends. *See* Berger, S. L. Anal Biochem 222:1 (1994).

### TARGETED ENZYMES

In one aspect, the present invention provides targeted enzymes that exhibit a catalytic activity, comprise a substrate recognition site and have been modified from a pretargeted enzyme to comprise one or more targeting sites, each targeting site comprising one or more variant sequences, and to bind to a target with higher affinity than the corresponding pre-targeted enzyme binds the target under like conditions. In one embodiment, the targeted enzyme of the invention differs from the corresponding pre-targeted enzyme only at the location of the variation-tolerant sequence or sequences of the pre-targeted enzyme.

Targeted enzymes of the invention include modified enzymes that bind to a target that the corresponding pre-targeted enzyme does not bind to under like conditions. For example, the present invention provides a targeted β-lactamase enzyme that binds to streptavidin under conditions where the corresponding pre-targeted β-lactamase does not bind to streptavidin. Targeted enzymes of the invention also include modified enzymes that bind to a target with about 2-fold, 3-fold, 5-fold, 10-fold, 10²-fold, 10³-fold, 10⁴-fold, 10⁵-fold, 10⁶-fold or higher affinity than the corresponding pre-targeted enzyme under like conditions. Targeted enzymes of the invention do not include enzymes with only one targeting site that consists solely of a polypeptide or other target-binding molecule that is attached to the N- or C-terminus of the pre-targeted enzyme (e.g., as in a histidine tagged protein or a fusion protein), a targeted enzyme whose only target is a monoclonal antibody, or a targeted enzyme made by increasing or optimizing the binding of a pre-targeted enzyme to a substrate of a reaction catalyzed by the pre-targeted enzyme. However, a targeted enzyme of the invention can be further modified to include a polypeptide or other targeting molecule that is attached to the NB or C-terminus. A targeted enzyme can also be further modified to change or optimize binding to a substrate of a reaction catalyzed by the targeted enzyme.

In one aspect, the targeted enzymes of the invention comprise one or more targeting sites, *e.g*.,one, two, three, four, five, six, seven, eight, nine, ten or more targeting sites, each of which comprises one or more variant sequences, *e.g.*, one, two, three, four, five, six, seven, eight, nine, ten or more variant sequences. The presence of the targeting site or sites in the targeted enzyme allows the targeted enzyme to binds to a target with higher affinity than the corresponding pre-targeted enzyme binds the target under like conditions.

The targeted enzyme can, for example, bind to target with a K_{d} of about 100 µM or less, 10 µM or less, 1 µM or less, 100 nM or less, about 90 nM or less, about 80 nM or less, about 70 nM or less, about 60 nM or less, about 50 nM or less, about 40 nM or less, about 30 nM or less, about 20 nM or less, about 10 nM or less, about 5 nM or less, about 1 nM or less or about 0.1 nM or less.

In one embodiment, each of the variant sequences is separated from its neighboring variant sequences by one or more constant segments in the primary sequence of the enzyme, but is close to each of the other variant sequences in the folded protein. This arrangement simplifies recombination as one can introduce recombination sites into the constant segments. Furthermore, such an arrangement reduces the chance of direct interaction between the different variable segments.

Variation-tolerant sequences can be, for example, single amino acids, or can sequences that are less than about 100, 90, 80, 70, 60, 50, 40, 30, 20, 10 or 5 amino acid residues in length. A variation tolerant sequence may be a loop of the folded protein, *e.g.*, a solvent accessible loop.

Variant sequences can be, for example, between zero and about 50 amino acid residues. In one embodiment, a variant sequence ranges from about zero to about 20, zero to about 14, zero to ten, or three to 20 amino acid residues in length. "Zero" amino acid residues refers to a situation where a variation-tolerant sequence has been deleted.

The targeting site of the targeted enzyme does not consist solely of the substrate recognition site of the pre-targeted enzyme. Preferably, the targeting site does not overlap with a catalytic site in the tertiary structure of the pre-targeted enzyme. As such, in one embodiment, the targeting site is at least about 1, 2, 3, 4, 5, 6, 7, 8, or 9 angstroms from the pre-targeted enzyme's catalytic site.

As discussed above, the targeted enzymes of the invention exhibit catalytic activity. Generally, the catalytic activity of the targeted enzyme corresponds to the catalytic activity of the corresponding pre-targeted enzyme. As such, the catalytic activity of the targeted enzyme is qualitatively that of the corresponding pre-targeted enzyme. Once a targeted enzyme is generated, however, its catalytic activity can be further modified (*e.g*, optimized or changed).

Any enzyme can serve as the pre-targeted enzyme for purposes of the present invention. In one embodiment, a corresponding pre-targeted enzyme is selected that has a catalytic activity that one desires to have in a targeted enzyme. In another embodiment, a pre-targeted enzyme is selected that converts a substrate into a desired product. In another embodiment, the substrate lacks a property that the product possesses. In another embodiment the property is a chemical or physical property. In another embodiment, the substrate does not cause an effect in a subject that the product causes. In another embodiment, the substrate is a nutrient of a diseased cell, tissue or organ. In another embodiment, the effect is a physiological effect. In another embodiment, the physiological effect is death of a cell. In another embodiment, the substrate is a prodrug and the product is an active drug.

Targeted enzymes may be used for therapeutic administration, *e.g.,* as part of targeted enzyme prodrug therapy applications. It is known that macromolecules with molecular weights below about 45,000 Daltons are rapidly cleared from the circulation by glomerular filtration of the kidney. *See* also Greenwald et al., Crit Rev Ther Drug Carrier Syst 17:101 (2000). A targeted enzyme may have a molecular weight that allows its removal from the circulation of a mammalian host via glomerular filtration. It is noted that in addition to having a shorter half-life in the circulation, smaller targeted enzymes diffuse more quickly than antibody-enzyme conjugates into certain types of targets, *e.g.,* a tumor mass. For *in vivo* applications, targeted enzymes are also preferred that have a relatively small size, preferably smaller than about 45kD, have a high specific activity, are highly active under physiological relevant conditions (*e.g*., between about 25-40E C and pH about 5.5 to about 7.5), and that are subject to minimal interference in the treated subject from inhibitors, enzyme substrates, or endogenous enzyme systems. In other aspects, the targeted enzyme has a molecular weight greater than 5 kD but less than 10 kD, 15 kD, 20 kD, 25 kD, 30 kD, 35 kD, 40 kD, 45 kD, 50 kD, 55 kD, 60 kD, 75 kD, 100kD, 150 kD, 200 kD, 250 kD, 300 kD, 350 kD, 400 kD, 450 kD or 500 kD.

For some embodiments, enzymes are preferred that are highly active in diseased cells with altered physiological states, for example, in cancer cells with lowered pH. Of particular interest are enzymes that can be used to activate a prodrug in a therapeutic setting. A large number of enzymes with different catalytic modes of action have been used to activate prodrugs. *See, e.g.,* Melton & Knox Enzyme-prodrug strategies for cancer therapy (1999) and Bagshawe et al., Curr Opin Immunol 11:579 (1999). These enzymes can be modified utilizing, for example, the methods of the present invention to incorporate targeting capability into the protein while retaining the ability of these enzymes to activate a prodrug. In another embodiment, enzymes that generate a toxic agent from a metabolite are modified to include a targeting site. While not a targeted enzyme as the term is utilized herein, Christofidou-Solomidou et al., Am J Physiol Lung Cell Mol Physiol 278:L794 (2000), for example, describes the use of glucose oxidase, which generates hydrogen peroxide from glucose, as an immune-targeted enzyme.

Examples of types of pre-targeted enzyme that can be used to make the targeted enzymes of the present invention include, but are not limited to, proteases, carboxypeptidases, β-lactamases, asparaginases, oxidases, hydrolases, lyases, lipases, cellulases, amylases, aldolases, phospatases, kinases, tranferases, polymerases, nucleases, nucleotidases, laccases, reductases, and the like. *See, e.g.,* co-pending U.S. Pat. App. Ser. No. 09/954,385, filed September 12, 2001, incorporated herein by reference in its entirety. As such, targeted enzymes of the invention can, for example, exhibit protease, carboxypeptidase, β-lactamase, asparaginase, oxidase, hydrolase, lyase, lipase, cellulase, amylase, aldolase, phospatase, kinase, tranferase, polymerase, nuclease, nucleotidase, laccase or reductase activity, or the like. A particular example of a type of enzyme that can be used are enzymes that can activate a prodrug, discussed below.

Examples of specific pre-targeted enzymes that can be used to make the targeted enzymes of the present invention include, but are not limited to, Class A, B, C, or D β-lactamase, β-galactosidase, *see* Benito et al., FEMS Microbiol. Lett. 123:107 (1994), fibronectin, glucose oxidate, glutathione S-transferase, *see* Napolitano et al., Chem. Biol. 3:359 (1996) and tissue plasminogen activator, *see* Smith et al., J. Biol. Chem. 270:30486 (1995).

In one embodiment, the targeted enzyme is not a laccase. In another embodiment, the targeted enzyme is not a bilirubin oxidase. In another embodiment, the targeted enzyme is not a phenol oxidase. In another embodiment, the targeted enzyme is not a catechol oxidase. In another embodiment, the targeted enzyme is not capable of catalyzing redox reactions wherein the electron donor is a phenolic compound and the electron acceptor is molecular oxygen or hydrogen peroxide.

In another embodiment, the catalytic activity of the targeted enzyme is not significantly different from the catalytic activity of the pre-targeted enzyme. That is, the variant sequence or sequences does not significantly increase or decrease the catalytic activity of the enzyme. In another embodiment, the catalytic activity of the targeted enzyme is between about 1% and about 100% of the catalytic activity of the pre-targeted enzyme. It is contemplated that the variant sequence or sequences can, in fact, result in a targeted enzyme that exhibits greater than 100% of the catalytic activity of the pre-targeted enzyme, for example, up to about 125%, 150%, 175%, 200%, 250%, 300%, 400% or 500%. In another embodiment, the catalytic activity of the targeted enzyme is between about 10% and about 100% of the catalytic activity of the pre-targeted enzyme. In another embodiment, the catalytic activity of the targeted enzyme is between about 20% and about 100% of the catalytic activity of the pre-targeted enzyme. In another embodiment, the catalytic activity of the targeted enzyme is between about 30% and about 100% of the catalytic activity of the pre-targeted enzyme. In another embodiment, the catalytic activity of the targeted enzyme is between about 40% and about 100% of the catalytic activity of the pre-targeted enzyme. In another embodiment, the catalytic activity of the targeted enzyme is between about 50% and about 100% of the catalytic activity of the pre-targeted enzyme. In another embodiment, the catalytic activity of the targeted enzyme is between about 60% and about 100% of the catalytic activity of the pre-targeted enzyme. In another embodiment, the catalytic activity of the targeted enzyme is between about 70% and about 100% of the catalytic activity of the pre-targeted enzyme. In another embodiment, the catalytic activity of the targeted enzyme is between about 80% and about 100% of the catalytic activity of the pre-targeted enzyme. In another embodiment, the catalytic activity of the targeted enzyme is between about 90% and about 100% of the catalytic activity of the pre-targeted enzyme.

In another embodiment, the catalytic activity of the targeted enzyme is not significantly affected by the binding of the target. That is, the targeted enzyme bound to the target has about the same catalytic activity as the targeted enzyme that is not bound to the target. In another embodiment, the catalytic activity of the targeted enzyme bound to the target is between about 10% and about 500% of the catalytic activity of the targeted enzyme not bound to the target. In another embodiment, the catalytic activity of the targeted enzyme bound to the target is between about 20% and about 450% of the catalytic activity of the targeted enzyme not bound to the target. In another embodiment, the catalytic activity of the targeted enzyme bound to the target is between about 30% and about 400% of the catalytic activity of the targeted enzyme not bound to the target. In another embodiment, the catalytic activity of the targeted enzyme bound to the target is between about 40% and about 350% of the catalytic activity of the targeted enzyme not bound to the target. In another embodiment, the catalytic activity of the targeted enzyme bound to the target is between about 50% and about 300% of the catalytic activity of the targeted enzyme not bound to the target. In another embodiment, the catalytic activity of the targeted enzyme bound to the target is between about 60% and about 250% of the catalytic activity of the targeted enzyme not bound to the target. In another embodiment, the catalytic activity of the targeted enzyme bound to the target is between about 70% and about 200% of the catalytic activity of the targeted enzyme not bound to the target. In another embodiment, the catalytic activity of the targeted enzyme bound to the target is between about 80% and about 150% of the catalytic activity of the targeted enzyme not bound to the target. In another embodiment, the catalytic activity of the targeted enzyme bound to the target is between about 90% and about 125% of the catalytic activity of the targeted enzyme not bound to the target. In another embodiment, the catalytic activity of the targeted enzyme bound to the target is between about 95% and about 110% of the catalytic activity of the targeted enzyme not bound to the target. In another embodiment, the catalytic activity of the targeted enzyme bound to the target is between about 60% and about 165% of the catalytic activity of the targeted enzyme not bound to the target. In another embodiment, the catalytic activity of the targeted enzyme bound to the target is about 100% of the catalytic activity of the targeted enzyme not bound to the target.

In another aspect the present invention provides a targeted enzyme that, while bound to a target, exhibits a catalytic activity of greater than about, *e.g.*, 1%, 10%, 20%, 30%, 40%, 50%, 60%, 70%, 80%, 90%, 100%, 150%, 200%, 250%, 500%, 750%, 1,000%, 1,500%, 2,000%, 2,500% or 5,000% relative to the catalytic activity of the pre-targeted enzyme.

In another aspect, the present invention provides a targeted enzyme that is a milieu-dependent targeted enzyme,

In another aspect, the present invention provides a targeted enzyme that is a multifunctional polypeptide.

### PRE-TARGETED ENZYMES

The pre-targeted enzyme used to make the targeted enzyme can be any enzyme, fragment of an enzyme, or derivative of an enzyme that has a catalytic activity and one or more variation-tolerant sequences and that does not, under like conditions, specifically bind a target that is bound by the targeted enzyme. Methods of identifying variation-tolerant sequences in an enzyme are taught below. The pre-targeted enzyme can have, *e.g.,* more than one activity. For example, the pre-targeted enzyme can have more than one catalytic activity, or one or more catalytic activities and one or more binding activities. In another embodiment, the pre-targeted enzyme is a naturally-occurring enzyme. In another embodiment, it is a mutated or otherwise genetically engineered protein. In another embodiment, it is a chimeric or fusion protein. In another embodiment, it is an artificially created enzyme.

In one embodiment, a pre-targeted enzyme is selected that has been modified or evolved to become more or less active in response to a stimulus, which then can be used to affect the activity of a targeted enzyme derived from it. In another embodiment, the stimulus is one that can be controlled, allowing the activity of the enzyme to be controlled. In another embodiment, the stimulus is pH. Many solid tumors have reduced internal pH compared to healthy tissue and this difference could be exploited to activate a targeted enzyme derived from the pre-targeted enzyme selectively at the tumor site. In another embodiment, the enzyme is activated by elevated or reduced temperature. Such temperature differences between various tissues can occur naturally or they can be induced, for instance, with microwaves. Temperature and pH serve as mere examples stimuli that can be used to selectively activate a pre-targeted enzyme.

In another the pre-targeted enzyme is a milieu-dependent targeted enzyme.

In another embodiment, the pre-targeted enzyme is a multifunctional polypeptide.

### Source of pre-targeted enzyme

In one embodiment, the pre-targeted enzyme is derived from a natural source of an enzyme, including, but not limited to, bacteria, archaea, plants, fungi or animals. In another embodiment, the pre-targeted enzyme is an enzyme from a species that the targeted enzyme will be used in. In another embodiment, the pre-targeted enzyme is a mammalian enzyme or a catalytically active fragment of a mammalian enzyme. In another embodiment, the pre-targeted enzyme is a primate enzyme or a catalytically active fragment of a primate enzyme. In another embodiment, the pre-targeted enzyme is a human enzyme or a catalytically active fragment of a human enzyme. In another embodiment, the pre-targeted enzyme is a human enzyme or catalytically active fragment of a human enzyme that has been genetically engineered or modified. In another embodiment, the pre-targeted enzyme is a fusion or chimeric protein comprising all or a portion of a human enzyme.

In one embodiment, the pre-targeted enzyme is not a laccase. For example, in one embodiment, the pre-targeted enzyme is not a bilirubin oxidase, a phenol oxidase, a catechol oxidase or an enzyme capable of catalyzing redox reactions wherein the electron donor is a phenolic compound and the electron acceptor is molecular oxygen or hydrogen peroxide.

A significant hurdle to existing chronic ADEPT protocols is that antibody-enzyme conjugates elicit an immune response in the subject Such a response precludes repeated treatment because, paradoxically, the immune system clears the antibody-enzyme conjugates from the circulation before the conjugates can reach their targets. Recently, significant progress has been made in generating human or humanized antibodies. However, this does not overcome the problem of immunogenicity of the enzyme attached to the antibody in the antibody-enzyme conjugate.

In another embodiment the prodrug is designed so that it is not activated or is slowly activated by the native human enzyme, yet possesses favorable pharmacological properties, *e.g.*, tissue distribution, half-life or toxicity. In another embodiment, a human pre-targeted enzyme is modified to selectively activate the prodrug. This modification can be accomplished using a combination of structure-based engineering, directed evolution, and chemical modification. As will be appreciated by one of skill in the art, the modification should be done in a way that minimizes the risk of introducing novel immunological epitopes into the targeted enzyme. There are methods available to test the modified enzyme for the presence of epitopes, which enables one to choose modifications that avoid the introduction of epitopes yet lead to the desired catalytic properties of the enzyme. For example, see U.S. Patent 5,750,356, WO 99/53038, WO 98/5296 and WO 99/61916.

In another embodiment, a targeted enzyme for use in a human subject is derived from a pre-targeted enzyme from a non-human source. In another embodiment, the pre-targeted enzyme is not immunogenic in a human subject.

As described in more detail below, disclosed herein is a method of treating a subject comprising administering a targeted enzyme and a prodrug that is a substrate of the targeted enzyme to a subject. Pre-targeted enzymes that are useful in this aspect include, but are not limited to alkaline phosphatase useful for converting phosphate-containing prodrugs into free drugs, arylsulfatase useful for converting sulfate-containing prodrugs into free drugs, cytosine deaminase useful for converting non-toxic 5-fluorocytosine into the anti-cancer drug, 5-fluorouracil, proteases, such as serine proteases, thermolysins, subtilisins, carboxypeptidases and cathepsins (such as cathepsins B and L), that are useful for converting peptide-containing prodrugs into free drugs, D-alanylcarboxypeptidases, useful for converting prodrugs that contain D-amino acid substituents, carbohydrate-cleaving enzymes such as β-galactosidase and neuraminidase useful for converting glycosylated prodrugs into free drugs, β-lactamase useful for converting drugs derivatized with β-lactams into free drugs, and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as abzymes, can be used to convert the prodrugs of the invention into free active drugs *(see, e.g.,* R. J. Massey, Nature, 328, pp. 457-458 (1987)).

Described in detail below are particular representative, non-limiting classes of targeted enzymes of the invention. Following the teaching provided herein, any other enzyme or enzyme class of interest can also be utilized in a similar fashion to produce targeted enzymes as those described below:

### β-lactamases

In one embodiment, the present invention provides a targeted β-lactamase (BLA) enzyme. In another embodiment, the targeted BLA enzyme comprises a substrate recognition site and a targeting site that binds a target, wherein the targeting site comprises one or more variant sequences derived from one or more variation-tolerant sequences.

In another embodiment, the variation-tolerant sequence is selected from the group consisting of loop A, loop B, loop C, loop D and loop E, as they are defined below.

In another embodiment, the targeted BLA enzyme has a specific activity greater than about 0.01 U/pmol against nitrocefin using the assay described below in the Examples. In another embodiment, the specific activity is greater than about 0.1 U/pmol. In another embodiment, the specific activity is greater than about 1 U/pmol. Preferably, these specific activities refer to the specific activity of the targeted BLA when bound to a target.

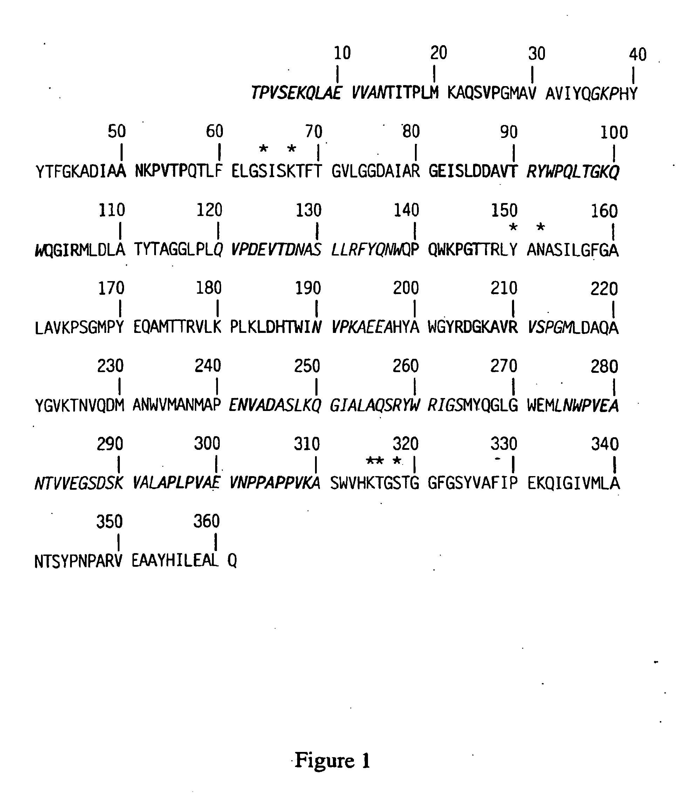

BLA enzymes are widely distributed in both gram-negative and gram-positive bacteria. BLA sequences are well known. A representative example of a BLA sequence is depicted in Figure 1. BLA enzymes vary in specificity, but have in common that they hydrolyze β-lactams, producing substituted β-amino acids. Thus, they confer resistance to antibiotics containing β-lactams. Because BLA enzymes are not endogenous to mammals, they are subject to minimal interference from inhibitors, enzyme substrates, or endogenous enzyme systems (unlike proteases; see below), and therefore are particularly well-suited for therapeutic administration. BLA enzymes are further well-suited to the therapeutic methods of the present invention because of their small size (BLA from *E. cloacae* is a monomer of 43 kD; BLA from E. *coli* is a monomer of 30 kD) and because they have a high specific activity against their substrates and have optimal activity at neutral pH and 37E C. *See* Melton et al., Enzyme-Prodrug Strategies for Cancer Therapy, Kluwer Academic/Plenum Publishers, New York (1999).

The β-lactamases have been divided into four classes based on their sequences. *See* Thomson et al., 2000, Microbes and Infection 2:1225-35. The serine β-lactamases are subdivided into three classes: A (penicillinases), C (cephalosporinases) and D (oxacillnases). Class B β-lactamases are the zinc-containing or metallo β-lactamases. Any class of BLA can be utiized to generate a targeted enzyme of the invention.

In one embodiment, the present invention provides a targeted β-lactamase that comprises the sequence YXN at its substrate recognition site (throughout, "X" refers to any amino acid residue). In another embodiment, the targeted β-lactamase comprises the sequence RLYANASI at its active site. In another embodiment, the targeted β-lactamase comprises a sequence at its active site that differs from the sequence RLYANASI by one, two or three amino acid residues. Preferably, the differences are the substitution of conservative amino acid residues. However, insertions, deletions and non-conservative amino acid substitutions also are included.

In one embodiment, the present invention provides a targeted β-lactamase that comprises the sequence KTXS at its substrate recognition site. In another embodiment, the targeted β-tactamase comprises the sequence VHKTGSTG at its active site. In another embodiment, the targeted β-lactamase comprises sequence at its active site that differs from the sequence VHKTGSTG by one, two or three amino acid residues. Preferably, the differences are the substitution of conservative amino acid residues. However, insertions, deletions and non-conservative amino acid substitutions also are included.

In one embodiment, the present invention provides a targeted β-lactamase that comprises the sequences YXN and KTXS at its substrate recognition site. In another embodiment, the targeted β-lactamase comprises the sequences VHKTGSTG and RLYANASI at its active site. In another embodiment, the targeted β-lactamase comprises sequences at its active site that differ from the sequences RLYANASI and VHKTGSTG by one, two or three amino acid residues. Preferably, the differences are the substitution of conservative amino acid residues. However, insertions, deletions and non-conservative amino acid substitutions also are included.

In one embodiment, the pre-targeted enzyme corresponding to a targeted enzyme of the present invention is a β-lactamase comprising the amino acid sequence of Figure 1. In such an embodiment, the targeted β-lactamase of the invention can be 50%, 60%, 70%, 80%, 90%, 95%, 98% or more (but not 100%) identical to the sequence depicted in Figure 1. In certain embodiments, the amino acid sequence of the targeted β-lactamase enzyme differs from the amino acid sequence depicted in Figure 1 only within the variation-tolerant sequence or sequences of the enzyme.

In other embodiments, the amino acid sequence of the β-lactamase pre-targeted enzyme is 50%, 60%, 70%, 80%, 90%, 95%, 98% or more identical to the sequence of Figure 1, and the targeted enzyme of the invention is derived from, but not identical to this sequence. In one such embodiment, the targeted enzyme differs from the β-lactamase pre-targeted enzyme only within the variation-tolerant sequence or sequences of the enzyme.

In another embodiment, a nucleic acid encoding the pre-targeted enzyme hybridizes to a nucleic acid complementary to a nucleic acid encoding the amino acid sequence of Figure 1 under highly stringent conditions. The highly stringent conditions can be, for example, hybridization to filter-bound DNA in 0.5 M NaHPO₄, 7% sodium dodecyl sulfate (SDS), 1 mM EDTA at 65E C, and washing in 0.1xSSC/0.1 % SDS at 68E C (Ausubel et al., eds., 1989, Current Protocols in Molecular Biology, Vol. I, Green Publishing Associates, Inc., and John Wiley & Sons, Inc., New York, at p. 2.10.3). Other highly stringent conditions can be found in, for example, Current Protocols in Molecular Biology, at pages 2.10.1-16 and Molecular Cloning: A Laboratory Manual, 2d ed., Sambrook et al. (eds.), Cold Spring Harbor Laboratory Press, 1989, pages 9.47-57. In another embodiment, a nucleic acid encoding the pre-targeted enzyme hybridizes to a nucleic acid complementary to a nucleic acid encoding the amino acid sequence of Figure 1 under moderately stringent conditions. The moderately stringent conditions can be, for example, washing in 0.2xSSC/0.1% SDS at 42E C (Ausubel et al., 1989, *supra).* Other moderately stringent conditions can be found in, for example, Current Protocols in Molecular Biology, Vol. I, Ausubel et al. (eds.), Green Publishing Associates, Inc., and John Wiley & Sons, Inc., 1989, pages 2.10.1-16 and Molecular Cloning: A Laboratory Manual, 2d ed., Sambrook et al. (eds.), Cold Spring Harbor Laboratory Press, 1989, pages 9.47-57.

In another embodiment the invention provides a method of treating a subject by administering to the subject a targeted BLA enzyme and a prodrug that is converted by the BLA into an active drug. Examples of suitable prodrugs for this embodiment are provided in, *e.g.,* Melton et al., Enzyme-Prodrug Strategies for Cancer Therapy, Kluwer Academic/Plenum Publishers, New York (1999), Bagshaw et al., Current Opinion in Immunology 11:579-83 (1999) and Kerr et al., Bioconjugate Chem. 9:255-59 (1998).

### TARGETS

The targets bound by the Targeted enzymes of the present invention can be any substance or composition to which a molecule can be made to bind.

In one aspect, the target is a surface. In one embodiment, the surface is a biological surface. In another embodiment, the biological surface is a surface of an organ. In another embodiment, the biological surface is a surface of a tissue. In another embodiment, the biological surface is a surface of a cell. In another embodiment, the biological surface is a surface of a diseased organ, tissue or cell. In another embodiment, the biological surface is a surface of a normal or healthy organ, tissue or cell. In another embodiment, the surface is a macromolecule in the interstitial space of a tissue. In another embodiment, the biological surface is the surface of a virus or pathogen. In another embodiment, the surface is a non-biological surface. In another embodiment, the non-biological surface is a surface of a medical device. In another embodiment, the medical device is a therapeutic device. In another embodiment, the therapeutic device is an implanted therapeutic device. In another embodiment, the medical device is a diagnostic device. In another embodiment, the diagnostic device is a well or tray.

Sources of cells or tissues include human, animal, bacterial, fungal, viral and plant. Tissues are complex targets and refer to a single cell type, a collection of cell types or an aggregate of cells generally of a particular kind. Tissue may be intact or modified. General classes of tissue in humans include but are not limited to epithelial tissue, connective tissue, nerve tissue, and muscle tissue.

In another aspect, the target is a cancer-related target. The cancer-related target can be any target that a composition of the invention binds to as part of the diagnosis, detection or treatment of a cancer or cancer-associated condition in a subject, for example, a cancerous cell, tissue or organ, a molecule associated with a cancerous cell, tissue or organ, or a molecule, cell, tissue or organ that is associated with a cancerous cell, tissue or organ (e.g., a tumor-bound diagnostic or therapeutic molecule administered to a subject or to a biopsy taken from a subject, or a healthy tissue, such as vasculature, that is associated with cancerous tissue). Examples of cancer-related targets are provided in U.S. Pat. No. 6,261,535.

The cancer-related target can be related to any cancer or cancer-associated condition. Examples of types of cancers include carcinomas, sarcomas, myelomas, leukemias, lymphomas and mixed type cancers.

In one embodiment, the cancer is a bone cancer, for example, Ewing's sarcoma, osteosarcoma and rhabdomyosarcoma and other soft-tissue sarcomas. In another embodiment, the cancer is a brain tumor, for example, oligodendroglioma, ependymoma, menengioma, lymphoma, schwannoma or medulloblastoma. In another embodiment, the cancer is breast cancer, for example, ductal carcinoma *in situ* of the breast. In another embodiment, the cancer is an endocrine system cancer, for example, adrenal, pancreatic, parathyroid, pituitary and thyroid cancers. In another embodiment, the cancer is a gastrointestinal cancer, for example, anal, colorectal, esophogeal, gallbladder, gastric, liver, pancreatic, and small intestine cancers. In another embodiment, the cancer is a gynecological cancer, for example, cervical, endometrial, uterine, fallopian tube, gestational trophoblastic disease, choriocarcinoma, ovarian, vaginal, and vulvar cancers. In another embodiment, the cancer is a head and neck cancer, for example, laryngeal, oropharyngeal, parathryroid or thyroid cancer. In another embodiment, the cancer is a leukemic cancer, for example, acute lymphocytic leukemia, acute myelogenous leukemia, chronic lymphocytic leukemia, chronic myelogenous leukemia, hairy cell leukemia, or a myeloproliferative disorder. In another embodiment, the cancer is a lung cancer, for example, a mesothelioma, non-small cell small cell lung cancer. In another embodiment, the cancer is a lymphoma, for example, AIDS-related lymphoma, cutaneous T cell lymphoma/mucosis fungoides, Hodgkin's disease, or non-Hodgkin's disease. In another embodiment, the cancer is metastatic cancer. In another embodiment, the cancer is a myeloma, for example, a multiple myeloma. In another embodiment, the cancer is a pediatric cancer, for example, a brain tumor, Ewing's sarcoma, leukemia (*e.g.,* acute lymphocytic leukemia or acute myelogenous leukemia), liver cancer, a lymphoma (*e.g.,* Hodgkin's lymphoma or non-Hodgkin's lymphoma), neuroblastoma, retinoblastoma, a sarcoma (e.g., osteosarcoma or rhabdomyosarcoma), or Wilms' Tumor. In another embodiment, the cancer is penile cancer. In another embodiment, the cancer is prostate cancer. In another embodiment, the cancer is a sarcoma, for example, Ewing's sarcoma, osteosarcoma, rhabdomyosarcoma and other soft-tissue sarcomas. In another embodiment, the cancer is a skin cancer, for example, cutaneous T cell lymphoma, mycosis fungoides, Kaposi's sarcoma or melanoma. In another embodiment, the cancer is testicular cancer. In another embodiment, the cancer is thyroid cancer, for example, papillary, follicular, medullary, or anaplastic or undifferentiated thyroid carcinoma. In another embodiment, the cancer is urinary tract cancers, for example, bladder, kidney or urethral cancers. In another embodiment, the cancer or cancer-related condition is ataxia-telangiectasia, carcinoma of unknown primary origin, Li-Fraumeni syndrome, or thymoma.

In another aspect, the cancer-related target is a molecule associated with a cancerous cell or tissue. In one embodiment, the molecule is a tumor or tumor stroma antigen, for example, GD2, Lewis-Y, 72 kd glycoprotein (gp72, decay-accelerating factor, CD55, DAF, C3/C5 convertases), CO17-1A (EpCAM, 17-1A, EGP-40), TAG-72, CSAg-P (CSAp), 45kd glycoprotein, HT-29 ag, NG2, A33 (43kd gp), 38kd gp, MUC-1, CEA, EGFR (HER1), HER2, HER3, HER4, HN-1 ligand, CA125, syndecan-1, Lewis X, PgP, FAP stromal Ag (fibroblast activation protein), EDG receptors (endoglin receptors), ED-B, laminin-5 (gamma2), cox-2 (+LN-5), PgP (P-glycoprotein), alphaVbeta3 integrin, alphaVbeta5, integrin, uPAR (urokinase plasminogen activator receptor), endoglin (CD105), folate receptor osteopontin (EDG 1,3), p97 (melanotransferrin), farnesyl transferase or a molecule in an apoptotic pathway (*e.g.,* a death receptor, fas, caspase or bc1-2) or a lectin.

In another aspect, the target is a hematopoietic cell. Hematopoietic cells encompass hematopoietic stem cells (HSCs), erythrocytes, neutrophils, monocytes, platelets, mast cells, eosinophils, basophils, B and T cells, macrophages, and natural killer cells. In one embodiment, the HSC has a surface antigen expression profile of CD34⁺ Thy-1⁺, and preferably CD34⁺ Thy-1⁺ Lin⁻. Lin⁻ refers to a cell population selected on the basis of the lack of expression of at least one lineage specific marker. Methods for isolating and selecting HSCs are well known in the art and reference is made to U.S. Patent Nos. 5,061,620, 5,677,136, and 5,750,397.

In another aspect, the target is a molecule. In one embodiment, the molecule is an organic molecule. In another embodiment, the molecule is a biological molecule. In another embodiment, the biological molecule is a cell-associated molecule. In another embodiment, the cell-associated molecule is associated with the outer surface of a cell. In another embodiment, the cell-associated molecule is part of the extracellular matrix. In another embodiment, the cell-associated molecule is associated with the outer surface of a cell is a protein. In another embodiment, the protein is a receptor. In another embodiment, the cell-associated molecule is specific to a type of cell in a subject. In another embodiment, the type of cell is a diseased cell. In another embodiment, the diseased cell is a cancer cell. In another embodiment, the diseased cell is an infected cell. Other molecules that can serve as targets according to the invention include, but are not limited to, proteins, peptides, nucleic acids, carbohydrates, lipids, polysaccharides, glycoproteins, hormones, receptors, antigens, antibodies, toxic substances, metabolites, inhibitors, drugs, dyes, nutrients and growth factors.

Non-limiting examples of protein and chemical targets encompassed by the invention include chemokines and cytokines and their receptors. Cytokines as used herein refer to any one of the numerous factors that exert a variety of effects on cells, for example inducing growth or proliferation. Non-limiting examples include interleukins (IL), IL-2, IL-3, IL-4 IL-6, IL-10, IL-12, IL-13, IL-14 and IL-16; soluble IL-2 receptor; soluble IL-6 receptor; erythropoietin (EPO); thrombopoietin (TPO); granulocyte macrophage colony stimulating factor (GM-CSF); stem cell factor (SCF); leukemia inhibitory factor (LIF); interferons; oncostatin M (OM); the immunoglobulin superfamily; tumor necrosis factor (TNF) family, particularly TNF-α; TGFβ; and IL-1α; and vascular endothelial growth factor (VEGF) family, particularly VEGF (also referred to in the art as VEGF-A), VEGF-B, VEGF-C, VEGF-D and placental growth factor (PLGF). Cytokines are commercially available from several vendors including Amgen (Thousand Oaks, CA), Immunex (Seattle, WA) and Genentech (South San Francisco, CA). Particularly preferred are VEGF and TNF-α. Antibodies against TNF-α show that blocking interaction of the TNF-α with its receptor is useful in modulating over-expression of TNF-α in several disease states such as septic shock, rheumatoid arthritis, or other inflammatory processes. VEGF is an angiogenic inducer, a mediator of vascular permeability, and an endothelial cell specific mitogen. VEGF has also been implicated in tumors. Targeting members of the VEGF family and their receptors may have significant therapeutic applications, for example blocking VEGF may have therapeutic value in ovarian hyper stimulation syndrome (OHSS). Reference is made to N. Ferrara et al., (1999) Nat. Med. 5:1359 and Gerber et al., (1999) Nat. Med. 5:623. Other preferred targets include cell-surface receptors, such as T-cell receptors.

Chemokines are a family of small proteins that play an important role in cell trafficking and inflammation. Members of the chemokine family include, but are not limited to, IL-8, stomal-derived factor-1(SDF-1), platelet factor 4, neutrophil activating protein-2 (NAP-2) and monocyte chemo attractant protein-1 (MCP-1).

Other protein and chemical targets include, but are not limited to: immunoregulation modulating proteins, such as soluble human leukocyte antigen (HLA, class I and/or class II, and non-classical class I HLA (E, F and G)); surface proteins, such as soluble T or B cell surface proteins; human serum albumin; arachadonic acid metabolites, such as prostaglandins, leukotrienes, thromboxane and prostacyclin; IgE, auto or alloantibodies for autoimmunity or allo- or xenoimmunity, Ig Fc receptors or Fc receptor binding factors; G-protein coupled receptors; cell-surface carbohydrates; angiogenesis factors; adhesion molecules; ions, such as calcium, potassium, magnesium, aluminum, and iron; fibril proteins, such as prions and tubulin; enzymes, such as proteases, aminopeptidases, kinases, phosphatases, DNAses, RNAases, lipases, esterases, dehydrogenases, oxidases, hydrolases, sulphatases, cyclases, transferases, transaminases, carboxylases, decarboxylases, superoxide dismutase, and their natural substrates or analogs; hormones and their corresponding receptors, such as follicle stimulating hormone (FSH), leutinizing hormone (LH), thyroxine (T4 and T3), apolipoproteins, low density lipoprotein (LDL), very low density lipoprotein (VLDL), cortisol, aldosterone, estriol, estradiol, progesterone, testosterone, dehydroepiandrosterone (DHBA) and its sulfate (DHEA-S); peptide hormones, such as renin, insulin, calcitonin, parathyroid hormone (PTH), human growth hormone (hGH), vasopressin and antidiuretic hormone (AD), prolactin, adrenocorticotropic hormone (ACTH), LHRH, thyrotropin-releasing hormone (THRH), vasoactive intestinal peptide (VIP), bradykinin and corresponding prohormones; catechcolamines such as adrenaline and metabolites; cofactors including atrionatriutic factor (AdF), vitamins A, B, C, D, E and K, and serotonin; coagulation factors, such as prothrombin, thrombin, fibrin, fibrinogen, Factor VIII, Factor IX, Factor XI, and von Willebrand factor; plasminogen factors, such as plasmin, complement activation factors, LDL and ligands thereof, and uric acid; compounds regulating coagulation, such as hirudin, hirulog, hementin, hepurin, and tissue plasminigen activator (TPA); nucleic acids for gene therapy; compounds which are enzyme antagonists; and compounds binding ligands, such as inflammation factors; and receptors and other proteins that bind to one or more of the preceding molecules.

Non-human derived targets include without limitation drugs, especially drugs subject to abuse, such as cannabis, heroin and other opiates, phencyclidine (PCP), barbiturates, cocaine and its derivatives, and benzadiazepine; toxins, such as heavy metals like mercury and lead, arsenic, and radioactive compounds; chemotherapeutic agents, such as paracetamol, digoxin, and free radicals; bacterial toxins, such as lipopolysaccharides (LPS) and other gram negative toxins, *Staphylococcus* toxins, Toxin A, Tetanus toxins, Diphtheria toxin and Pertussis toxins; plant and marine toxins; snake and other venoms, virulence factors, such as aerobactins, or pathogenic microbes; infectious viruses, such as hepatitis, cytomegalovirus (CMV), herpes simplex virus (HSV types 1, 2 and 6), Epstein-Barr virus (EBV), varicella zoster virus (VZV), human immunodeficiency virus (HIV-1, -2) and other retroviruses, adenovirus, rotavirus, influenzae, rhinovirus, parvovirus, rubella, measles, polio, pararyxovirus, papovavirus, poxvirus and picornavirus, prions, plasmodia tissue factor, protozoans, such as *Entamoeba histolitica,* Filaria, Giardia, Kalaazar, and toxoplasma; bacteria, gram-negative bacteria responsible for sepsis and nosocomial infections such as E. *coli, Acynetobacter, Pseudomonas, Proteus* and *Klebsiella,* also gram-positive bacteria such as *Staphylococcus, Streptococcus, Meningococcus* and *Llycobacteria, Chlamydiae Legionnella* and Anaerobes; fungi such as *Candida, Pneumocystis, Aspergillus,* and *Mycoplasma.*

In one aspect the target includes an enzyme such as proteases, aminopeptidases, kinases, phosphatases, DNAses, RNAases, lipases, esterases, dehydrogenases, oxidases, hydrolases, sulphatases, cellulases, cyclases, transferases, transaminases, carboxylases, decarboxylases, superoxide dismutase, and their natural substrates or analogs. Particularly preferred enzymes include hydrolases, particularly alpha/beta hydrolases; serine proteases, such as subtilisins, and chymotrypsin serine proteases; cellulases; and lipases.

In another embodiment, the target is a non-biological material. In another embodiment, the non-biological material is a fabric. In another embodiment, the fabric is a natural fabric. In another embodiment, the fabric is cotton. In another embodiment, the fabric is silk. In another embodiment, the fabric is wool. In another embodiment, the fabric is a non-natural fabric. In another embodiment, the fabric is nylon. In another embodiment, the fabric is rayon. In another embodiment, the fabric is polyester. In another embodiment, the non-biological material is a plastic. In another embodiment, the non-biological material is a ceramic. In another embodiment, the non-biological material is a metal. In another embodiment, the non-biological material is rubber.

In another embodiment the target is a microcircuit. This circuit can be in its finished form or in any stage of circuit manufacturing. The targeted enzyme can be used to remove or deposit a compound onto the circuit, for example, an n-type dopant (e.g., arsenic, phosphorus, antimony, titanium or other donor atom species) or a p-type dopant (e.g., boron, aluminum, gallium, indium or other acceptor atom species). *See, e.g.,* van Zant, 2000, Microchip Fabrication, McGraw-Hill, New York, incorporated herein by reference in its entirety.

In another embodiment, the target is not an antibody (e.g., a polyclonal antibody, a monoclonal antibody, an scFv, or another antigen-binding fragment of an antibody).

### TARGETED ENZYME PRODRUG THERAPY

Disclosed herein is a method of treating a subject by administering a targeted enzyme and a prodrug, wherein the targeted enzyme is specifically localized to a portion of the subject's body where it converts the prodrug into an active drug. Examples of enzyme/prodrug/active drug combinations are found in, *e.g.,* Bagshawe et al., Current Opinions in Immunology, 11:579-83 (1999); Wilman, "Prodrugs In Cancer Chemotherapy," Biochemical Society Transactions, 14, pp. 375-82 (615th Meeting, Belfast 1986) and V. J. Stella et al., "Prodrugs: A Chemical Approach To Targeted Drug Delivery," Directed Drug Delivery, R. Borchardt et al. (ed), pp.247-67 (Humana Press 1985). In one embodiment, the prodrug is a peptide. Examples of peptides as prodrugs can be found in Trouet et al., Proc Natl Acad Sci USA 79:626 (1982), and Umemoto et al., Int J Cancer 43:677 (1989). These and other reports show that peptides are sufficiently stable in blood. Another advantage of peptide-derived prodrugs is their amino acid sequences can be chosen to confer suitable pharmacological properties like half-life, tissue distribution, and low toxicity to the active drugs. Most reports of peptide-derived prodrugs relied on relatively nonspecific activation of the prodrug by, for instance, lysosomal enzymes. Recently, it was reported that a peptide-drug conjugate was specifically cleaved by prostate specific antigen (PSA) at a tumour site. *See* DeFeo-Jones et al., Nat Med 6:1248 (2000). This report shows the activation of peptide prodrugs at the tumor site is an efficient way to increase the selectivity of an anticancer agent.

The prodrug can be one that is converted to an active drug in more than one step. For example, the prodrug can be converted to a precursor of an active drug by the targeted enzyme. The precursor can be converted into the active drug by, for example, the catalytic activity of one or more additional targeted enzymes, the catalytic activities of one or more non-targeted enzymes administered to the subject, the catalytic activity of one or more enzymes naturally present in the subject or at the target site in the subject (e.g., a protease, a phosphatase, a kinase or a polymerase), by a drug that is administered to the subject, or by a chemical process that is not enzymatically catalyzed *(e.g.,* oxidation, hydrolysis, isomerization, epimerization).

### Drugs

Most studies involving prodrugs are generated after programs with existing drugs are found to be problematic. In particular anticancer drugs were generally characterized by a very low therapeutic index. By converting these drugs into prodrugs with reduced toxicity and then selectively activating them in the diseased tissue, the therapeutic index of the drug was significantly reduced. *See, e.g.,* Melton et al., Enzyme-prodrug strategies for cancer therapy (1999), and Niculescu-Duvaz et al., Anticancer Drug Des 14:517 (1999).

This invention allows one of skill in the art to evolve the specificity of an enzyme to accommodate even structures that would be poor substrates for naturally occurring enzymes. Thus, prodrugs can be designed even though the drugs were otherwise not amenable to a prodrug strategy.

Curnis et al., Nat Biotechnol 18:1185 (2000) showed the cytokine TNFα, when selectively targeted towards tumor vasculature, exhibited a strong antitumor effect. Otherwise, systemic delivery of TNFa is hampered by its toxicity. Other cytokines are likely to have similar limitations. The present invention enables the design of cytokine-based prodrugs that are selectively activated in diseased tissue by a targeted enzyme.

A number of studies have been performed with toxins coupled to targeting agents (usually antibodies or antibody fragments). *See, e.g.,* Torchilin, Eur J Pharm Sci 11Suppl 2:S81 (2000) and Frankel et al., Clin Cancer Res 6:326 (2000). An alternative to the above is to convert these toxins into prodrugs and then selectively release them in the diseased tissue.

### Prodrugs

The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide containing prodrugs, 5-fluorocytosine and other 5-fluorouridine prodrugs which can be converted by the enzyme of the conjugate into the more active cytotoxic free drug. Examples of cytotoxic drugs that can be derivatized into a prodrug form for use in this invention include, but are not limited to, etoposide, temposide, adriamycin, daunomycin, carminomycin, aminopterin, dactinomycin, mitomycins, cis-platinum and cis-platinum analogues, bleomycins, esperamicins (*see* U.S. Pat. No. 4,675,187), 5-fluorouracil, melphalan, other related nitrogen mustards, and derivatives thereof. *See, e.g.,* U.S. Pat. No. 4,975,278.

In one embodiment of the invention, the pre-targeted enzyme is an alkaline phosphatase (AP) that converts a 4'-phosphate derivative of the epipodophyl-lotoxin glucosides into an active anti-cancer drug. Such derivatives include etoposide-4'-phosphate, etoposide- 4'-thiophosphate and teniposide-4'-phosphate. Other embodiments of the invention may include phosphate derivatives of these glucosides wherein the phosphate moiety is placed at other hydroxyl groups on the glucosides. In another embodiment, however, the phosphate derivative used as a pro-drug in this invention is etoposide-4'-phosphate or etoposide-4'-thiophosphate. The targeted AP removes the phosphate group from the prodrug, releasing an active antitumor agent. The mitomycin phosphate prodrug of this embodiment may be an N⁷-C₁₋₈ alkyl phosphate derivative of mitomycin C or porfiromycin, or pharmaceutically acceptable salts thereof. N⁷ refers to the nitrogen atom attached to the 7-position of the mitosane nucleus of the parent drug. According to another embodiment, the derivative used is 7-(2'-aminoethylphosphate)mitomycin ("MOP"). Alternatively, the MOP compound may be termed, 9a-methoxy-7-[[(phosphonooxy)ethyl]amino]mitosane disodium salt. Other embodiments of the invention may include the use pf N⁷-alkyl mitomycin phosphorothioates as prodrugs.

In still another embodiment of the invention, a penicillin amidase enzyme can be used as the pre-targeted enzyme, which converts a novel adriamycin prodrug into the active antitumor drug, adriamycin. In another embodiment, the penicillin amidase is a penicillin V amidase ("PVA") isolated from *Fusarium oxysporum* that hydrolyzes phenoxyacetyl amide bonds. The prodrug utilized can be N-(p-hydroxyphenoxyacetyl)adriamycin ("APO"), which is hydrolyzed by the amidase to release the potent antitumor agent, adriamycin

The present invention also comprises, for example, the use of the adriamycin prodrug, N-(p-hydroxyphenoxyacetyl)adriamycin and other related adriamycin prodrugs that can be derivatized in substantially the same manner. For example, use of the prodrug N-(phenoxyacetyl) adriamycin is also within the scope of the invention. In addition, it is to be understood that the adriamycin prodrugs of this invention include other N-hydroxyphenoxyacetyl derivatives of adriamycin, *e.g.*, substituted at different positions of the phenyl ring, as well as N-phenoxyacetyl derivatives containing substituents on the phenyl ring other than the hydroxyl group described herein.

Furthermore, the present embodiment encompasses the use of other amidases, such as penicillin G amidase, as the pre-targeted enzyme as well as other prodrugs correspondingly derivatized such that the particular amidase can hydrolyze that prodrug to an active antitumor form. For example, when a penicillin G amidase is used as the pretargeted enzyme, the prodrug should contain a phenylacetylamide group (as opposed to the phenoxyacetylamide group of APO) because penicillin G amidases hydrolyze this type of amide bond (*see, e.g.,* A. L. Margolin et al., Biochim. Biophys Acta. 616, pp. 283-89 (1980)). Thus, other prodrugs of the invention include N-(p-hydroxyphenylacetyl) adriamycin, N-(phenylacetyl) adriamycin and other optionally substituted N-phenylacetyl derivatives of adriamycin.

It should also be understood that the present invention includes, but is not limited to, any prodrug derived by reacting the amine group of the parent drug with the carboxyl group of phenoxyacetic acid, phenylacetic acid or other related acids. Thus, prodrugs of anthracyclines other than adriamycin that are capable of being derivatized and acting in substantially the same manner as the adriamycin prodrugs described herein falls within the scope of this invention. For example, other prodrugs that can be produced and used in accordance with this invention include hydroxyphenoxyacetylamide derivatives, hydroxyphenylacetylamide derivatives, phenoxyacetylamide derivatives and phenylacetylamide derivatives of anthracyclines such as daunomycin and carminomycin. Other amine-containing drugs such as melphalan, mitomycin, aminopterin, bleomycin and dactinomycin can also be modified described herein to yield prodrugs of the invention.

Yet another embodiment of the invention involves a targeted enzyme form of the enzyme, cytosine deaminase ("CD"). The deaminase enzyme catalyzes the conversion of 5-fluorocytosine ("5-FC"), a compound lacking in antineoplastic activity, to the potent antitumor drug, 5-fluorouracil ("5-FU").

Disclosed herein is a method of combination chemotherapy using several prodrugs and a single targeted enzyme. According to this embodiment, a number of prodrugs are used that are all substrates for the same targeted enzyme. Thus, a particular targeted enzyme converts a number of prodrugs into cytotoxic form, resulting in increased antitumor activity at the tumor site.

According to another embodiment, a number of different targeted enzymes are used. Each targeted enzyme can be used to convert its respective prodrug or prodrugs into active form at the target tumor site.

Disclosed herein is the use of a number of targeted enzymes wherein the target bound by the enzymes varies, i.e., a number of targeted enzymes are used, each one binding specifically to a different target of interest. The catalytic activities of the targeted enzymes may be the same or may vary. This embodiment may be especially useful in situations where, for example, the amounts of the various targets on the surface of a tumor is unknown and one wants to be certain that sufficient enzyme is targeted to the tumor site. The use of a number of targeted enzymes recognizing different targets on the tumor increases the likelihood of obtaining sufficient enzyme at the tumor site for conversion of a prodrug or series ofprodrugs. Additionally, this embodiment is important for achieving a high degree of specificity for the tumor because the likelihood that normal tissue will possess all of the same tumor-associated antigens is small (*cf*., I. Hellstrom et al., "Monoclonal Antibodies To Two Determinants Of Melanoma-Antigen p97 Act Synergistically In Complement-Dependent Cytotoxicity", J. Immunol, 127 (No. 1),pp. 157-160(1981)).

In another embodiment, a targeted enzyme is used that binds to a plurality of targets on a diseased cell. In another embodiment, the targeted enzyme comprises a plurality of targeting sites, each of which binds to a different target on the diseased cell. The targeted enzyme binds relatively weakly to cells having fewer than all of the targets but relatively strongly to cells having all of the targets.

There is often a requirement for extending the blood circulation half-lives of pharmaceutical peptides, proteins, or small molecules. Typically short half-lives lasting minutes to hours require not only frequent, but also high, doses for therapeutic effect often so high that initial peak doses cause side effects. Extending the half-life of such therapeutics permits lower, less frequent, and therefore potentially safer doses, which arc cheaper to produce. Previously researchers have increased protein half-life by fusing them covalently to PEG, *see* U.S. Patent 5,711,944, human blood serum albumin, *see* U.S. Patent 5,766,883, orFc fragments, *see* WO 00/24782. In addition, nonspecific targeting of drugs to human serum albumin has been accomplished by chemical coupling drugs *in vivo. See* U.S. Patent 5,843,440. Furthermore, in the case of cancer drugs it has been proposed that high molecular weight drugs may localize in tumors due to enhanced permeability and retention. Therefore, improvement in the therapeutic index of a drug can be obtained by linking the drug to a protein or other high molecular weight polymer.

However, the prior art methods for stabilizing protein and peptide therapeutics or increasing the size of cancer therapeutics have several limitations. These methods suffer from the lack of specificity involved in chemical coupling. There is also an inherent limitation of C- and N-terminal fusions in the case of fusion peptides since only two sites of attachment arc possible. In addition, protein production of HSA conjugates can be problematic on a large scale. There is little or no release of covalently fused therapeutics so the pharmacodynamic properties of the therapeutic construct are not easily controlled. In addition, all of these methods substantially increase the time and effort required to identify stable therapeutics since they are not modular in nature.

In one embodiment, the present invention provides a method to selectively stabilize a therapeutic peptide, protein, or small molecule by non-covalently targeting the therapeutic site specifically to human serum albumin (HSA). Using selective targeting methods, peptide sequences that selectively bind to serum albumin with high affinity and high selectivity could be identified. Briefly, HSA-depleted blood is incubated with a library of molecules, preferably peptides. Peptides that do not bind to HSA- depleted blood are then incubated with immobilized HSA, washed extensively, and HSA binding peptides are then identified. Peptides are further optimized for use as a therapeutic, *e.g.,* to limit their immunological response, proteolytic susceptiblity in the blood, or ease of manufacture. Fusion of these small peptides to therapeutics of interest substantially increase the half-life or therapeutic index of the drug. Furthermore, the peptide drug conjugate can be much simpler to administer. Protease clip sites can be introduced between the HSA targeting peptide and the drug or therapeutic. When these HSA targeted drugs are administered in the blood, the drug conjugate selectively binds to HSA and could be released based upon the physically designed properties of the binding agent (kₒₙ & k_{off} in the blood) or by enzymatic cleavage or activation. This approach can be extended to targeting other long lived blood proteins including Fc fragments, α2-macroglobulin, steroids, and erythrocytes, for example.

The vasculature in cancer tissue exhibits a higher than normal diffusivity. *See* Yuan et al., Cancer Res 55:3752 (1995). Furthermore, the diffusivity of macromolecules in the interstitial space of tumors is relatively high compared to normal tissues. *See* Jain, Cancer Res 47:3039 (1987).

A recent review summarizes experimental results that demonstrate that the increased diffusivity of tumors can be exploited by designing macromolecular prodrugs in particular based an coupling to PEG. *See* Greenwald et al., Crit Rev Ther Drug Carrier Syst 17:101 (2000). However, these prodrugs rely for their activation either on chemical lability of the linker or on rather non-specific enzymes in the tumor site. This approach can be significantly enhanced by targeting a selective enzyme to the tumor site which can cleave the macromolecular part of the prodrug and thus release it. This approach allows for prodrugs with very low toxicity, due to their macromolecular pro-part which keeps the prodrug out of most tissues and prevents the prodrug from entering most cells. In addition, one can design the linker part to be very stable to prevent drug activation in unrelated tissues.

Disclosed herein is a method of treating a condition in subject comprising administering to the subject a targeted enzyme with β-lactamase activity and a prodrug. In another embodiment, the targeted enzyme is targeted to cancerous cell, tissue, tumor or organ. In another embodiment, the cancer is a melanoma or a carcinoma. In another embodiment, the prodrug is converted by the targeted enzyme into an active drug. In another embodiment, the active drug is an alkylating agent. In another embodiment, the prodrug is an anticancer nitrogen mustard prodrug. In another embodiment, the active drug is melphalan. In another embodiment, the prodrug is C-Mel. *See* Kerr et al., Bioconjugate Chem. 9:255-59 (1998). In another embodiment, the prodrug is vinca-cephalosporin or doxorubicin cephalosporin. *See* Bagshawe et al., Current Opinion in Irnmunology, 11:579-83 (1999). Other prodrug/enzyme combinations that can be used in the present invention include, but are not limited to, those found in U.S. Patent No. 4,975,278 and Melton et al., Enzyme-Prodrug Strategies for Cancer Therapy Kluwer Academic/Plenum Publishers, New York (1999).

The list of candidates for the pro-part of the prodrugs is extensive and diverse, and many, are well known to those of skill in the art.

### NUCLEIC ACIDS AND METHODS OF EXPRESSING TARGETED ENZYMES

In another aspect, the present invention provides a nucleic acid encoding a targeted enzyme. The nucleic acid can be, for example, a DNA or an RNA. The present invention also provides a plasmid comprising a nucleic acid encoding a targeted enzyme. The plasmid can be, for example, an expression plasmid that allows expression of the targeted enzyme in a host cell or organism, or *in vitro.* The expression vector can allow expression of the targeted enzyme in, for example, a bacterial cell. The bacterial cell can be, for example, an *E. coli* cell.

Because of the redundancy in the genetic code, typically a large number of DNA sequences encode any given amino acid sequence and are, in this sense, equivalent. As described below, it may be desirable to select one or another equivalent DNA sequences for use in a expression vector, based on the preferred codon usage of the host cell into which the expression vector will be inserted. The present invention is intended to encompass all DNA sequences that encode the targeted enzyme.

Production of the targeted enzyme of the invention can be carried out using a recombinant expression clone. The construction of the recombinant expression clone, the transformation of a host cell with the expression clone, and the culture of the transformed host cell under conditions which promote expression, can be carried out in a variety of ways using techniques of molecular biology well understood in the art. Methods for each of these steps are described in general below. Preferred methods are described in detail in the examples.

An operable expression clone is constructed by placing the coding sequence in operable linkage with a suitable control sequences in an expression vector. The vector can be designed to replicate autonomously in the host cell or to integrate into the chromosomal DNA of the host cell. The resulting clone is used to transform a suitable host, and the transformed host is cultured under conditions suitable for expression of the coding sequence. The expressed targeted enzyme is isolated from the medium or from the cells, although recovery and purification of the targeted enzyme may not be necessary in some instances.

Construction of suitable clones containing the coding sequence and a suitable control sequence employs standard ligation and restriction techniques that are well understood in the art. In general, isolated plasmids, DNA sequences, or synthesized oligonucleotides are cleaved, modified, and religated in the form desired. Suitable restriction sites can, if not normally available, be added to the ends of the coding sequence so as to facilitate construction of an expression clone.

Site-specific DNA cleavage is performed by treating with a suitable restriction enzyme (or enzymes) under conditions that are generally understood in the art and specified by the manufacturers of commercially available restriction enzymes. *See, e.g.,* product catalogs from Amersham (Arlington Heights, IL), Roche Molecular Biochemicals (Indianapolis, IN), and New England Biolabs (Beverly, MA). In general, about 1 µg of plasmid or other DNA is cleaved by one unit of enzyme in about 20µl of buffer solution; in the examples below, an excess of restriction enzyme is generally used to ensure complete digestion of the DNA. Incubation times of about one to two hours at a temperature which is optimal for the particular enzyme are typical. After each incubation, protein is removed by extraction with phenol and chloroform; this extraction can be followed by ether extraction and recovery of the DNA from aqueous fractions by precipitation with ethanol. If desired, size separation of the cleaved fragments may be performed by polyacrylamide gel or agarose gel electrophoresis using standard techniques. *See, e.g.,* Maxam et al., 1980, Methods in Enzymology 65:499-560.

Restriction enzyme-cleaved DNA fragments with single-strand "overhanging" termini can be made blunt-ended (double-strand ends) by, for example, treating with the large fragment of *E*. *coli_*DNA polymerase I (Klenow) in the presence of the four deoxynucleoside triphosphates (dNTPs) using incubation times of about 15 to 25 minutes at 20°C to 25°C in 50 mM Tris, pH 7.6, 50 mM NaCl, 10 mM MgCl₂, 10 mM DTT, and 5 to 10 µM dNTPs. The Klenow fragment fills in at 5' protruding ends, but chews back protruding 3' single strands, even though the four dNTPs are present. If desired, selective repair can be performed by supplying one or more selected dNTPs, within the limitations dictated by the nature of the protruding ends. After treatment with Klenow, the mixture is extracted with phenol/chloroform and ethanol precipitated. Similar results can be achieved using S1 nuclease, because treatment under appropriate conditions with S 1 nuclease results in hydrolysis of any single-stranded portion of a nucleic acid.

Ligations can be performed, for example, in 15-30 µl volumes under the following standard conditions and temperatures: 20 mM Tris-Cl, pH 7.5, 10 mM MgCl₂, 10 mM DTT, 33 µg/ml BSA, 10-50 mM NaCI, and either 40 µM ATP and 0.01-0.02 (Weiss) units T4 DNA ligase at 0°C (for ligation of fragments with complementary single-stranded ends) or 1mM ATP and 0.3-0.6 units T4 DNA ligase at 14°C (for "blunt end" ligation). Intermolecular ligations of fragments with complementary ends are usually performed at 33-100 µg/ml total DNA concentrations (5-100 nM total ends concentration). Intermolecular blunt end ligations (usually employing a 20-30 fold molar excess of linkers, optionally) are performed at 1 µM total ends concentration.

In vector construction, the vector fragment is commonly treated with bacterial or calf intestinal alkaline phosphatase (BAP or CIAP) to remove the 5' phosphate and prevent religation and reconstruction of the vector. BAP and CIAP digestion conditions are well known in the art, and published protocols usually accompany the commercially available BAP and CIAP enzymes. To recover the nucleic acid fragments, the preparation is extracted with phenol-chloroform and ethanol precipitated to remove the phosphatase and purify the DNA. Alternatively, religation of unwanted vector fragments can be prevented by restriction enzyme digestion before or after ligation, if appropriate restriction sites are available.

Correct ligations for plasmid construction can be confirmed using any suitable method known in the art. For example, correct ligations for plasmid construction can be confirmed by first transforming a suitable host, such as *E. coli* strain DG101 (ATCC 47043) or *E. coli* strain DG116 (ATCC 53606), with the ligation mixture. Successful transformants are selected by ampicillin, tetracycline or other antibiotic resistance or sensitivity or by using other markers, depending on the mode of plasmid construction, as is understood in the art. Plasmids from the transformants are then prepared according to the method of Clewell et al., 1969, Proc. Natl. Acad. Sci. USA 62:1159, optionally following chloramphenicol amplification. *See* Clewell, 1972, J. Bacteriol. 110:667. Alternatively, plasmid DNA can be prepared using the "Base-Acid" extraction method at page 11 of the Bethesda Research Laboratories publication Focus 5 (2), and very pure plasmid DNA can be obtained by replacing steps 12 through 17 of the protocol with CsCl/ethidium bromide ultracentrifugation of the DNA. As another alternative, a commercially available plasmid DNA isolation kit, *e.g.,* HISPEEDJ, QIAFILTERJ and QIAGEN7 plasmid DNA isolation kits (Qiagen, Valencia CA) can be employed following the protocols supplied by the vendor. The isolated DNA can be analyzed by, for example, restriction enzyme digestion and/or sequenced by the dideoxy method of Sanger et al., 1977, Proc. Natl. Acad. Sci. USA 74:5463, as further described by Messing et al., 1981, Nuc. Acids Res. 9:309, or by the method of Maxam et al., 1980, Methods in Enzymology 65:499.

The control sequences, expression vectors, and transformation methods are dependent on the type of host cell used to express the gene. Generally, procaryotic, yeast, insect, or mammalian cells are used as hosts. Procaryotic hosts are in general the most efficient and convenient for the production of recombinant proteins and are therefore preferred for the expression of the protein.

The procaryote most frequently used to express recombinant proteins is *E. coli.* However, microbial strains other than *E. coli* can also be used, such as bacilli, for example *Bacillus subtilis,* various species *of Pseudomonas* and *Salmonella,* and other bacterial strains. In such procaryotic systems, plasmid vectors that contain replication sites and control sequences derived from the host or a species compatible with the host are typically used.

For expression of constructions under control of most bacterial promoters, *E. coli* K12 strain MM294, obtained from the *E. coli* Genetic Stock Center under GCSC #6135, can be used as the host. For expression vectors with the P_{L}N_{RBS} or P_{L} T7_{RBS} control sequence, *E. coli* K12 strain MC1000 lambda lysogen, N₇N₅₃cI857 SusP₈₀, ATCC 39531, may be used. *E. coli* DG116 , which was deposited with the ATCC (ATCC 53606) on April 7, 1987, and *E*. *coli* KB2, which was deposited with the ATCC (ATCC 53075) on March 29, 1985, are also useful host cells. For M13 phage recombinants, *E. coli* strains susceptible to phage infection, such as *E. coli* K12 strain DG98 (ATCC 39768), are employed. The DG98 strain was deposited with the ATCC on July 13, 1984.

For example, *E. coli* is typically transformed using derivatives of pBR322, described by Bolivar et al., 1977, Gene 2:95. Plasmid pBR322 contains genes for ampicillin and tetracycline resistance. These drug resistance markers can be either retained or destroyed in constructing the desired vector and so help to detect the presence of a desired recombinant. Commonly used procaryotic control sequences, i.e., a promoter for transcription initiation, optionally with an operator, along with a ribosome binding site sequence, include the β-lactamase (penicillinase) and lactose (lac) promoter systems, *see* Chang et al., 1977, Nature 198:1056, the tryptophan (trp) promoter system, *see* Goeddel et al., 1980, Nuc. Acids Res. 8:4057, and the lambda-derived P_{L} promoter, *see* Shimatake et al., 1981, Nature 292:128, and gene N ribosome binding site (N_{RBS}). A portable control system cassette is set forth in U.S. Patent No. 4,711,845, issued December 8, 1987. This cassette comprises a P_{L} promoter operably linked to the N_{RBS} in turn positioned upstream of a third DNA sequence having at least one restriction site that permits cleavage within six base pairs 3' of the N_{RBS} sequence. Also useful is the phosphatase A (phoA) system described by Chang et al., in European Patent Publication No. 196,864, published October 8, 1986. However, any available promoter system compatible with procaryotes can be used to construct a expression vector of the invention.

In addition to bacteria, eucaryotic microbes, such as yeast, can also be used as recombinant host cells. Laboratory strains of *Saccharomyces cerevisiae,* Baker's yeast, are most often used, although a number of other strains are commonly available. While vectors employing the two micron origin of replication are common, *see* Broach, 1983, Meth. Enz. 101:307, other plasmid vectors suitable for yeast expression are known. *See, e.g.,* Stinchcomb et al., 1979, Nature 282:39; Tschempe et al., 1980, Gene 10:157; and Clarke et al., 1983, Meth. Enz. 101:300. Control sequences for yeast vectors include promoters for the synthesis of glycolytic enzymes. *See* Hess et. al., 1968, J. Adv. Enzyme Reg. 7:149; Holland et al., 1978, Biotechnology 17:4900; and Holland et al., 1981, J. Biol. Chem. 256:1385. Additional promoters known in the art include the promoter for 3-phosphoglycerate kinase, *see* Hitzeman et al., 1980, J. Biol. Chem. 255:2073, and those for other glycolytic enzymes, such as glyceraldehyde 3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other promoters that have the additional advantage of transcription controlled by growth conditions are the promoter regions for alcohol dehydrogenase 2, isocytochrome C, acid phosphatase, degradative enzymes associated with nitrogen metabolism, and enzymes responsible for maltose and galactose utilization (Holland, *supra).*

Terminator sequences may also be used to enhance expression when placed at the 3' end of the coding sequence. Such terminators are found in the 3' untranslated region following the coding sequences in yeast-derived genes. Any vector containing a yeast-compatible promoter, origin of replication, and other control sequences is suitable for use in constructing yeast expression vectors.

The coding sequence can also be expressed in eucaryotic host cell cultures derived from multicellular organisms. *See, e.g.,* Tissue Culture, Academic Press, Cruz and Patterson, editors (1973). Useful host cell lines include COS-7, COS-A2, CV-1, murine cells such as murine myelomas N51 and VERO, HeLa cells, and Chinese hamster ovary (CHO) cells. Expression vectors for such cells ordinarily include promoters and control sequences compatible with mammalian cells such as, for example, the commonly used early and late promoters from Simian Virus 40 (SV 40), *see* Fiers et al., 1978, Nature 273:113, or other viral promoters such as those derived from polyoma, adenovirus 2, bovine papilloma virus (BPV), or avian sarcoma viruses, or immunoglobulin promoters and heat shock promoters. A system for expressing DNA in mammalian systems using a BPV vector system is disclosed in United States Patent No. 4,419,446. A modification of this system is described in U.S. Patent No. 4,601,978. General aspects of mammalian cell host system transformations have been described by Axel, U.S. Patent No. 4,399,216. "Enhancer" regions are also important in optimizing expression; these are, generally, sequences found upstream of the promoter region. Origins of replication may be obtained, if needed, from viral sources. However, integration into the chromosome is a common mechanism for DNA replication in eucaryotes.

Plant cells can also be used as hosts, and control sequences compatible with plant cells, such as the nopaline synthase promoter and polyadenylation signal sequences, *see* Depicker et al., 1982, J. Mol. Appl. Gen. 1:561, are available. Expression systems employing insect cells utilizing the control systems provided by baculovirus vectors have also been described. *See* Miller et al., in Genetic Engineering (1986), Setlow et al., eds., Plenum Publishing, Vol. 8, pp. 277-97. Insect cell-based expression can be accomplished in *Spodoptera frugipeida.* These systems are also successful in producing recombinant enzymes.

Depending on the host cell used, transformation is done using standard techniques appropriate to such cells. The calcium treatment employing calcium chloride, as described by Cohen, 1972, Proc. Natl. Acad. Sci. USA 69:2110 is used for procaryotes or other cells that contain substantial cell wall barriers. Infection with *Agrobacterium tumefaciens, see* Shaw et al., 1983, Gene 23:315, is used for certain plant cells. For mammalian cells, the calcium phosphate precipitation method of Grahamet al., 1978, Virology 52:546 is preferred. Transformations into yeast are carried out according to the method of Van Solingen et al., 1977, J. Bact. 130:946, and Hsiao et al., 1979, Proc. Natl. Acad. Sci. USA 76:3829.
It may be desirable to modify the sequence of the DNA encoding the targeted enzyme of the invention to provide, for example, a sequence more compatible with the codon usage of the host cell without modifying the amino acid sequence of the encoded protein. Such modifications to the initial 5-6 codons may improve expression efficiency. DNA sequences which have been modified to improve expression efficiency, but which encode the same amino acid sequence, are considered to be equivalent and encompassed by the present invention.

A variety of site-specific primer-directed mutagenesis methods are available and well-known in the art. *See, e.g.,* Sambrook et al., Molecular Cloning: A Laboratory Manual, Cold Spring Harbor, 1989, second edition, chapter 15.51, "Oligonucleotide-mediated mutagenesis," which is incorporated herein by reference. The polymerase chain reaction (PCR) can be used to perform site-specific mutagenesis. In another technique now standard in the art, a synthetic oligonucleotide encoding the desired mutation is used as a primer to direct synthesis of a complementary nucleic acid sequence contained in a single-stranded vector, such as pBSM13+ derivatives, that serves as a template for construction of the extension product of the mutagenizing primer. The mutagenized DNA is transformed into a host bacterium, and cultures of the transformed bacteria are plated and identified. The identification of modified vectors may involve transfer of the DNA of selected transformants to a nitrocellulose filter or other membrane and the "lifts" hybridized with kinased synthetic mutagenic primer at a temperature that permits hybridization of an exact match to the modified sequence but prevents hybridization with the original unmutagenized strand. Transformants that contain DNA that hybridizes with the probe are then cultured (the sequence of the DNA is generally confirmed by sequence analysis) and serve as a reservoir of the modified DNA.

Once the protein has been expressed in a recombinant host cell, purification of the protein may be desired. A variety of purification procedures can be used to purify the targeted enzymes of the invention.

For long-term stability, the purified targeted enzyme must be stored in a buffer that contains one or more non-ionic polymeric detergents. Such detergents are generally those that have a molecular weight in the range of approximately 100 to 250,00 preferably about 4,000 to 200,000 daltons and stabilize the enzyme at a pH of from about 3.5 to about 9.5, preferably from about 4 to 8.5. Examples of such detergents include those specified on pages 295-298 ofMcCutcheon's Emulsifiers & Detergents, North American edition (1983), published by the McCutcheon Division of MC Publishing Co., 175 Rock Road, Glen Rock, NJ (USA), the entire disclosure of which is incorporated herein by reference. Preferably, the detergents are selected from the group comprising ethoxylated fatty alcohol ethers and lauryl ethers, ethoxylated alkyl phenols, octylphenoxy polyethoxy ethanol compounds, modified oxyethylated and/or oxypropylated straight-chain alcohols, polyethylene glycol monooleate compounds, polysorbate compounds, and phenolic fatty alcohol ethers. More particularly preferred are Tween 20J, a polyoxyethylated (20) sorbitan monolaurate from ICI Americas Inc. (Wilmington, DE), and IconolJ NP-40, an ethoxylated alkyl phenol (nonyl) from BASF Wyandotte Corp. (Parsippany, NJ).

### METHODS OF MAKING TARGETED ENZYMES

In one aspect, the present invention provides methods of making the targeted enzymes of the invention. Any suitable method can be used.

In one embodiment of the invention, a targeted enzyme is made by modifying a variation-tolerant sequence of a pre-targeted enzyme and selecting the modified enzyme if it binds to a target and has catalytic activity while bound to the target. In another embodiment, an iterative approach is used wherein a modified enzyme that has catalytic activity while bound to target is further modified in the variant sequence and further selected if it has increased binding to the target, increased catalytic activity, or shows an improvement in another property. The cycle is repeated until an enzyme having a desired set of characteristics is obtained. In another embodiment, the pre-targeted enzyme has two or more varation-tolerant sequences that are modified. In another embodiment, the pre-targeted enzyme has three or more variation-tolerant sequences that are modified. In another embodiment, the pre-targeted enzyme has four or more variation-tolerant sequences that are modified.

In another embodiment of the invention, a variation-tolerant sequence of a pre-targeted enzyme is replaced with a repertoire of variant sequences, forming a repertoire of modified enzymes, and a modified enzyme is selected from the repertoire of modified enzymes if it has catalytic activity while bound to a target. In another embodiment, an iterative approach is used wherein a modified enzyme that has catalytic activity while bound to target is further modified in its variant sequence and further selected if it has increased binding to the target, increased catalytic activity, or shows an improvement in another property. The cycle is repeated until an enzyme having a desired set of characteristics is obtained.

In another embodiment, a first variant sequence corresponding to a first variation-tolerant sequence of a pre-targeted enzyme is combined with a second variant sequence corresponding to a second variation-tolerant sequence of the pre-targeted enzyme to create a modified enzyme comprising the first variant sequence and the second variant sequence, and the modified enzyme is selected if it has catalytic activity while bound to a target. In another embodiment, an iterative approach is used wherein a modified enzyme that has catalytic activity while bound to the target is further modified in its first and/or its second variant sequence and further selected if it has increased binding to the target, increased catalytic activity, or shows an improvement in another property. The cycle is repeated until an enzyme having a desired set of characteristics is obtained.

In another embodiment, a first repertoire of variant sequences corresponding to a first variation-tolerant sequence in a pre-targeted enzyme is combined with a second repertoire of variant sequences corresponding to a second variation-tolerant sequence of the pre-targeted enzyme to produce a repertoire of modified enzymes comprising a variant sequence from the first repertoire and a variant sequence from the second repertoire, and a modified enzyme is selected from the repertoire of modified enzymes if it has catalytic activity while bound to a target. In another embodiment, an iterative approach is used wherein a modified enzyme that has catalytic activity while bound to the target is further modified in its first and/or its second variant sequence and further selected if it has increased binding to the target, increased catalytic activity, or shows an improvement in another property. The cycle is repeated until an enzyme having a desired set of characteristics is obtained.

In another embodiment, a first repertoire of variant sequences corresponding to a first variation-tolerant sequence in a pre-targeted enzyme is combined with a second repertoire of variant sequences corresponding to a second variation-tolerant sequence of the pre-targeted enzyme and a third repertoire of variant sequences corresponding to a third variation-tolerant sequence of the pre-targeted enzyme to produce a repertoire of modified enzymes comprising a variant sequence from the first repertoire, a variant sequence from the second repertoire and a variant sequence from the third repertoire, and a modified enzyme is selected from the repertoire of modified enzymes if it has catalytic activity while bound to a target. In another embodiment, an iterative approach is used wherein a modified enzyme that has catalytic activity while bound to the target is further modified in one or more of its variant sequences and further selected if it has increased binding to the target, increased catalytic activity, or shows an improvement in another property. The cycle is repeated until an enzyme having a desired set of characteristics is obtained.

In another embodiment, a first repertoire of variant sequences corresponding to a first variation-tolerant sequence in a pre-targeted enzyme is combined with a second repertoire of variant sequences corresponding to a second variation-tolerant sequence of the pre-targeted enzyme, a third repertoire of variant sequences corresponding to a third variation-tolerant sequence of the pre-targeted enzyme and a fourth repertoire of variant sequences corresponding to a fourth variation-tolerant sequence of the pre-targeted enzyme to produce a repertoire of modified enzymes comprising a variant sequence from the first repertoire, a variant sequence from the second repertoire, a variant sequence from the third repertoire and a variant sequence from the fourth repertoire, and a modified enzyme is selected from the repertoire of modified enzymes if it has catalytic activity while bound to a target. In another embodiment, an iterative approach is used wherein a modified enzyme that has catalytic activity while bound to the target is further modified in one or more of its variant sequences and further selected if it has increased binding to the target, increased catalytic activity, or shows an improvement in another property. The cycle is repeated until an enzyme having a desired set of characteristics is obtained.

The number of variant sequences that can be combined in one modified enzyme is limited only by the number of variation-tolerant sequences that the corresponding pre-targeted enzyme possesses.

In one embodiment, the enzymatic activity of the pre-targeted enzyme is used to select modified enzymes that are at least partially functional and, therefore, relatively structurally unaffected by the modification. For example, modified pre-targeted enzymes that confer antibiotic resistance to a cell can be expressed in the cell, and the cell exposed to the antibiotic. Resistance to the antibiotic indicates that the modification does not inactivate the enzyme. Similarly, a modified pre-targeted enzyme that metabolizes a necessary nutrient can be expressed in a cell that requires that nutrient. Growth in the absence of the nutrient indicates that the modified enzyme does not inactivate the enzyme. More generally, any pre-targeted enzyme that confers a detectable or selectable phenotype to a cell can be used to select modified pre-targeted enzymes that have not been inactivated by the modification. Cell-free or *in vitro* selection or detection systems also can be used, for example, processing of a fluorogenic or chromogenic substrate by the modified pre-targeted enzyme.

In one embodiment, a two-step approach is used to generate the targeted enzyme. In the first step, a peptide sequence is inserted into an enzyme. A library of modified enzymes can be generated, each having the peptide sequence inserted into a different site of the enzyme, using, for example, transposon mutagenesis. The library of modified enzymes is screened to identify modified enzymes that retain enzymatic activity. This allows the identification of one or more sites in the enzyme that will tolerate the insertion of a peptide sequence. In the second step, the inserted peptide sequence of a modified enzyme identified as retaining enzymatic activity in the first step is replaced by a peptide that binds to a target. A second, smaller library of modified enzymes can thus be formed, each modified enzyme in this library having a binding peptide in a position known to tolerate insertion of a peptide. In another embodiment, binding peptides are inserted into a plurality of sites identified in the unmodified enzyme as tolerating an insertion. The inserted peptides can be the same, or they can be different. In another embodiment, the inserted peptides together comprise a binding domain. In another embodiment, the inserted peptides each comprise their own binding domain, thus imparting to the modified enzyme binding sites for a plurality of targets.

In another embodiment, the present invention provides a method of making a targeted enzyme that is a milieu-dependent targeted enzyme.

In another embodiment, the present invention provides a method of making a targeted enzyme that is a multifunctional polypeptide application.

In another embodiment, the present invention provides a method of making a targeted enzyme comprising an affinity maturation step or steps.

Several workers have proposed grafting recognition elements from one protein enzyme onto another enzyme for improved (or modified) function. *See* Smith er al., J Biol Chem 270:30486 (1995). However, it is often suggested that efficient *in vivo* targeting cannot by accomplished with significant effect by proteins as small as single, recombinant V-domains with a molecular weight of about 15 kD. Random libraries of peptides have been generated on various protein scaffolds including protease inhibitors, *see* Roberts et al., Gene 121:9 (1992), and GFP. Furthermore, it is generally assumed that the sites for such loop libraries are quite restricted based on the overall fold of the protein and it is often required to have a three-dimensional model of such a protein to construct and screen useful libraries. *See* U.S. Patent 6,025,485. The rules for locating such replacement loops are not well defined. Furthermore it is often assumed that large binding fragments such as Fab fragments are required for tight binding in tumor targeting applications. *See* Hudson, Curr Opin Biotechnol. 9(4):395 (1998). Phage display of folded proteins is often difficult and generating large libraries for phage displayed proteins can be problematic. The present invention provides methods of rapidly generating targeted enzymes without the requirement for three-dimensional structural information, and the libraries generated by phage can be cloned rapidly into a variety of different enzyme systems without the need to optimize library generation for each enzyme.

In one embodiment, the present invention provides a method of generating on a single enzyme scaffold for therapeutic effect tight binding, targeted and efficient enzymes smaller than 60 kD, and preferably smaller than 30 kD. The flexibility of the present therapeutic system can be formatted to be effective at nanomolar doses or less due to the catalytic nature of the targeted enzyme. Furthermore the circulating half-life can be customized for rapid clearance in ADEPT or TEPT strategies for example. The smaller size of such agents would provide novel methods of delivery such as inhalation that are problematic for larger molecules.

In one embodiment of the invention, the generation of targeted enzymes involves the steps of
1) Screening a library of peptides (displayed on phage for example) for affinity to cell specific targets including tumor antigens or other cell surface markers, using selective targeting methods.
2) PCR amplification of tight binding phage peptides and using Type II restriction enzymes to clone these sub-libraries into any protein of interest
3) Screening these much smaller targeted enzyme libraries on a single clone level (10² to 10⁴) for function such as prodrug activation in a cell based assay.

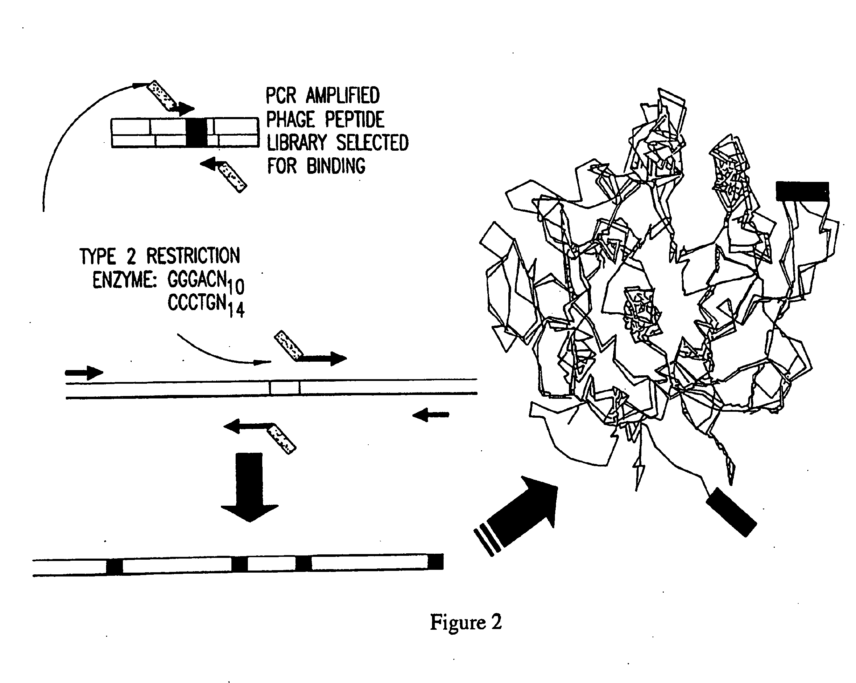

It may be assumed that peptide sequences that bind to a target in the context of pIII phage displayed peptides will also bind to the target in the context of loops in the enzyme. Although this is a radical assumption since the peptide has a free amino terminus in the phage and its conformation is therefore presumably able to adopt many more conformations, avidity in the context of the multicopy pIII system may allow tight binders, *e.g.*, peptides identified by *in vivo* phage display. *See* Arap et al., Science 279:377 (1998). Cloning strategies can be developed that allow construction of sublibraries with appropriate restriction sites such that only 4-5 libraries will have to be constructed in the enzyme to screen for function. This approach requires the use of type II restriction enzyme cloning to introduce appropriate libraries (Figure 2). The inventors postulate the introduction of additional mutational variability in the oligo design may improve the expression of loop targeted variants. This method takes advantage of the fact that a key step in selective targeting using phage peptide libraries relies on a PCR step to amplify target bound phage so that PCR primers can be designed as a part of the targeting strategy to clone directly into a protein of interest. Thus, problems of constructing phage protein libraries directly are alleviated.

Flexible loops for insertion and replacement can be based on criteria well known in the art. For example, in subtilisin from *Bacillus lentus,* loop insertions can be identified from, *e.g.,* :
1. alignment of the sequence with thermal B-factors
2. conservancy indices across a family
3. ¹⁵N-¹H NOE correlation times
4. substrate binding grooves/clefts near active site residues (so as to not occlude substrate binding completely.

Alternatively, the enzyme library could be generated by standard molecular biology protocols either directly or using display technologies and screened for binding affinity to the target of interest using selective targeting methods. Once tight binding sequences are identified, the enzyme can be optimized for function and binding in an iterative fashion.

### Variant sequence repertoires

In one embodiment, the variant sequences in the repertoire are chosen to have one or more desired traits, *e.g.*:A
· a targeted enzyme comprising the variant sequence adopts a conformation that is homologous to that of the pre-targeted enzymeA
· a targeted enzyme comprising the variant sequence retains catalytic activity
· a targeted enzyme comprising the variant sequence retains stability, *e.g.*, protease stabilityA
· the variant sequences in the repertoire have diverse chemical properties and/or shapesA
· the variant sequences have low immunogenicityA
· the variant sequences have known liquid chromatography/mass spectroscopy (LC/MS) profiles, which simplifies the identification and/or characterization of individual variant sequences in a recombinant library or in subgroups of library members.

A library of protein mutants should contain at least one member with desirable and identifiable properties. One can increase the odds of finding a desired clone by increasing the library size. However, the size of a library can be limited by a variety of factors like transformation efficiency or the ability to screen or select. A more efficient way of increasing the odds of finding a desired clone is to increase the hit density of a library, *i.e.,* the fraction of useful clones in the library.

Recombining repertoires of variant sequences that have been pre-selected reduces the fraction of unstable variants in a recombinant library. In general, proteins vary in their tolerance to substitutions with residues close to the active site or in the conserved center of a protein being less tolerated than residues in outside loops. However, even outside residues of a protein that show little evolutionary conservation may not be freely substituted without some loss of protein stability. If one simultaneously replaces multiple residues of a protein, a significant fraction of the mutants may have impaired expression, secretion, stability or catalytic activity compared to the wildtype protein. *See* Axe, J Mol Biol 301:585 (2000). By recombining a plurality of segments, each of which in an otherwise wildtype protein has been found to result in a fully functional or nearly fully functional protein, then one significantly reduces the fraction of unstable, non-expressing or inactive mutants in a library. This is particularly the case if the various recombined segments do not directly interact with each other in the correctly folded protein.

Furthermore, by recombining variant sequence repertoires one gains control over the structural and chemical diversity in the recombinant library. For instance, it has been observed that certain amino acids like Tyr and Asn are more abundant in the variable loops of natural antibodies as compared to their abundance in other proteins. It has been speculated these and some other amino acids are particularly suitable for recognition and discrimination. Similarly, one can affect the abundance of charged and hydrophobic residues in the variable segments by increasing the number of charged or hydrophobic residues in a segment to increase chemical and structural diversity.

Typical random libraries contain many very similar clones. Consequently, if a library contains a clone with a desired property then it is likely to contain many other clones with similar functional and structural properties. This may actually confound the identification of desirable clones. An ideal library contains just a sufficient number of clones with desirable properties and few similar clones, i.e., it has a steep fitness distribution. In such a library one can frequently identify desirable clones by pooling sublibraries and measuring their properties. By using preselected variable segments, which differ widely in their properties one can create such libraries with "non-smooth" fitness distributions.

### Generation of variant sequence repertoires

In one embodiment of the invention, the repertoires are derived from human sequences. This reduces the potential to elicit an immune response. In addition, one can inspect known three-dimensional structures and synthesize all variant sequences that apparently can be accomodated by a variation-tolerant sequence of a pre-targeted enzyme. In another embodiment, one can replace a variation-tolerant sequence in a pre-targeted enzyme with a fully randomized or partially randomized sequence. Subsequently, one can screen and select for retention of enzyme function and stability and any other trait of importance.

Alternatively, one can sequence the functional mutants and choose variant sequences of the repertoire based on their sequence considering one or more criteria as discussed above. This would enable one to create repertoires and not rely on purely random sequences. For instance one can avoid duplication of variant sequences, avoid variant sequences that have equal mass but different structure, which would be difficult to identify via mass spectroscopy, or choose variant sequences that differ widely in amino acid composition to maximize the diversity in the library.

### Location of variant sequences in the enzyme

The variant sequences can be placed anywhere in the structure of the pre-targeted enzyme. Of particular interest are regions that can tolerate modification, and/or binding of a target to the modified region, without undesirably affecting the catalytic activity of the enzyme.

A targeting site can comprise one or more variant sequences. In another embodiment, the targeting site comprises several variant sequences. In another embodiment, each of the variant sequences is separated from its neighboring variant sequences by one or more constant segments in the primary sequence of the enzyme, but is close to each of the other variant sequences in the folded protein. This arrangement will simplify recombination as one can introduce recombination sites into the constant segments. Furthermore, such an arrangement reduces the chance of direct interaction between the different variable segments.

Variation-tolerant sequences can be, for example, single amino acids, or can sequences that are less than about 100, 90, 80, 70, 60, 50, 40, 30, 20, 10 or 5 amino acid residues in length. Variant sequences can be, for example, between zero and about 50 amino acid residues. In another embodiment, a variant sequence ranges from about zero to about 20, zero to ten, or three to 20 amino acid residues in length. "Zero" amino acid residues refers to a situation where a variation-tolerant sequence has been deleted.

Potential variation-tolerant sequences and targeting sites can be identified by, *e.g.*, comparing sequence alignments of homologous genes. Sequence regions that show a low degree of conservation are more likely to accommodate a variety of different segments compared to highly conserved regions of the sequence. Of particular interest are regions where natural homologs of a protein have insertions or deletions relative to each other.

Potential variation-tolerant sequences and targeting sites also can be chosen, *e.g.*, based on the known or predicted three-dimensional structure of the pre-targeted enzyme or its homologs. For instance one can align the three-dimensional structures of several homologous proteins and identify regions in the structure that show significant variability in the side chains or in the conformation of the peptide backbone. Alternatively, one can identify regions of the structure that form a groove that can or could accommodate a target *(i.e.,* a concave targeting sites). In other cases it may be advantageous to identify a region or regions that protrude away from the protein *(i.e.,* a convex targeting sites).

Solvent accessible loops also are potential variation-tolerant sequences in a pre-targeted enzyme. Solvent accessible loops can be identified, for example, based on their sequence and their location in the sequence of a pre-targeted enzyme or by examination of the known or predicted three-dimensional structure of the pre-targeted enzyme.

Placement of variant sequences in β-lactamase: In another embodiment the present invention provides a targeted β-lactamase (BLA) enzyme, and methods of making and using targeted BLA enzymes, particularly in combination with a prodrug. BLA and tumor-specific antibody fragments have shown promising results in experiments testing the targeted release of cancer drugs. *See* Siemers et al., Bioconjug Chem 8:510 (1997). Inspection of the available crystal structure reveals a number of loops that are candidates for variation-tolerant sequences. Of particular interest, but by no means of only interest, are the following areas of the protein, which are surface accessible and not part of secondary structure elements: Q23-P26, A50-P56, G81-R105, G116-A127, P140-T146, L184-K193, Y203-S212, E241-D245, N275-A280, A294-K309.

### Construction of variant sequence repertoires

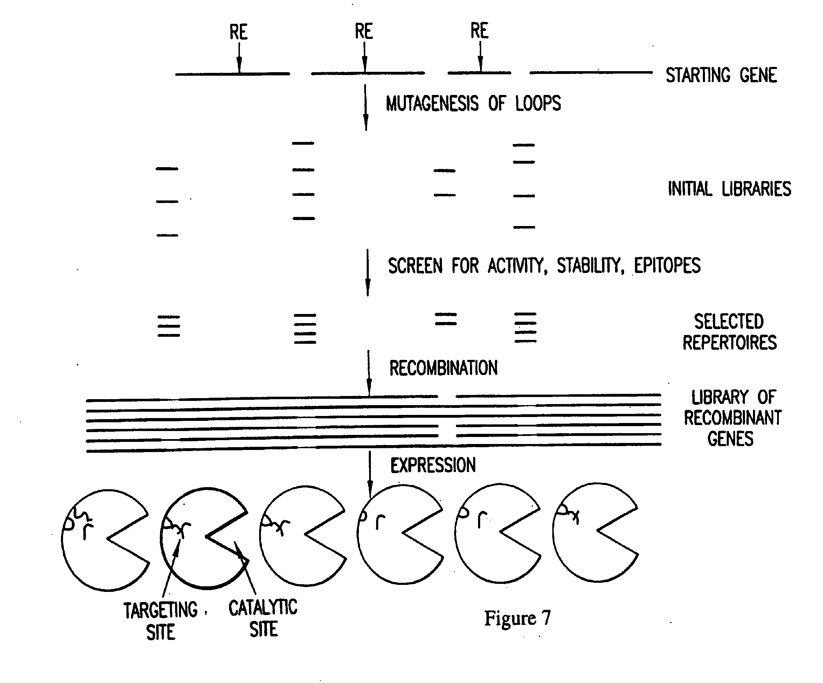

Figure 7 outlines the overall process of generating variant sequence repertoires, recombining them, and generating a large plurality of enzyme variant which differ in the amino acid sequences that make up the targeting site of the enzyme. The resulting mixture of enzyme variants has to be searched to identify variants that bind the target of interest. This can be done, for example, by screening, mass spectroscopy, or phage display. One of skill in the art knows many methods for creating libraries of recombined variant sequences, including, but not limited to, those methods described below.

Assembly of multiple restriction or PCR fragments: One isolates mixtures of nucleic acids that code for each variant sequence repertoire. These nucleic acids can be prepared by, *e.g.*, PCR or by digestion of plasmid mixtures with restriction enzymes. In another embodiment the nucleic acids are generated by digestion of plasmids with hapaxomers. Then one can mix the variant sequence repertoires and assemble full-length plasmids via ligation. Alternatively, one can isolate the individual variant sequences from each clone in the variant sequence repertoires and then mix them to create the library. This process requires the handling of many DNA samples but it allows one to control the relative abundance of each variant sequence in the library.

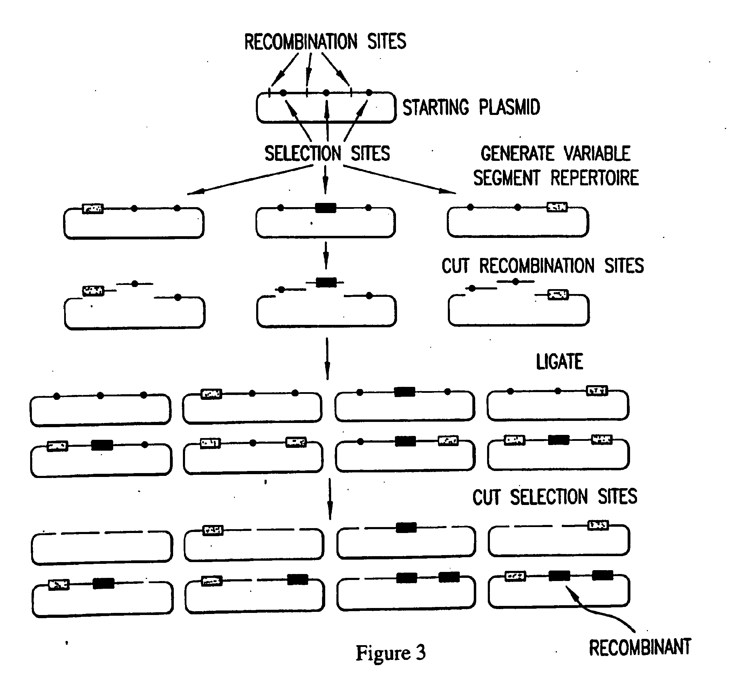

Phoenix mutagenesis: Phoenix mutagenesis has been described as an approach to introduce mutations into a plasmid. *See* Berger et al., Anal Biochem 214:571 (1993). One can digest and reassemble a plasmid with high efficiency when using endonucleases that generate non-palindromic overhangs, *i.e,* hapaxomers. In the present invention, the procedure is modified to allow for the efficient recombination of variant sequence repertoires as illustrated in Figure 3. The starting plasmid will be designed such that the constant segments, which separate the variation-tolerant sequences, contain at least one recombination site that can be cleaved by a hapaxomer (indicated by vertical line) and each variation-tolerant sequence contains at least one unique restriction site (selection sites, indicated by circle). All recombination sites can be recognized by the same hapaxomer as long as the resulting overhangs differ between all recombination sites. Once the variant sequence repertoires have been generated the plasmids coding for the different repertoires are mixed and digested at their recombination sites. The resulting fragments can be ligated. Because all recombination sites have different overhangs most of the re-ligated plasmids will contain the respective sequences in the same order as the starting plasmid. Subsequently, the ligation products can be cleaved at the selection sites. As a result, all ligation products that carry a wild type version of one or more variation-tolerant sequences will be cut into one or more linear fragments. Linear DNA molecules transform E. *coli* with a greatly reduced efficiency. Only ligation products wherein each variation-tolerant sequence originates from one of the variant sequence repertoires will remain circular and will transform E. *coli* with high efficiency.

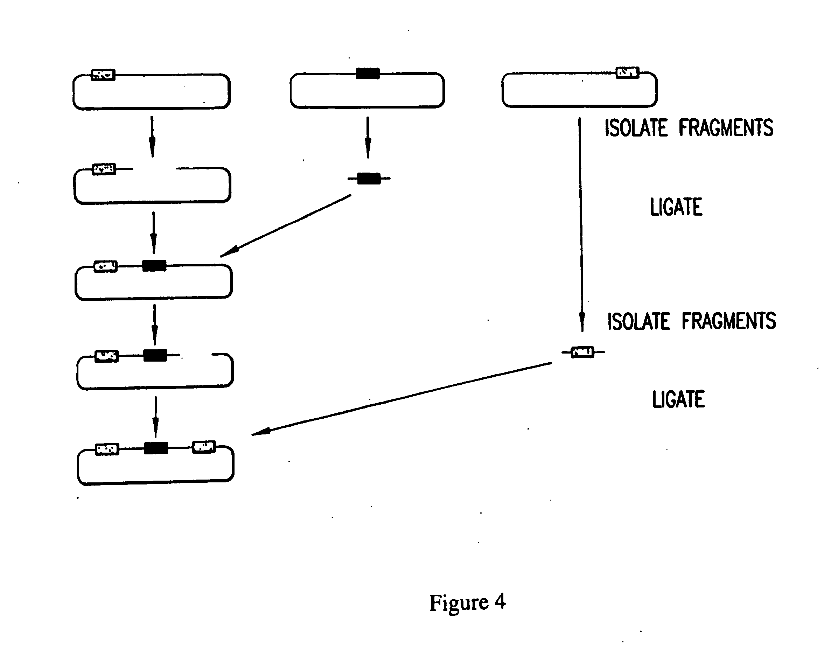

Library generation using conventional restriction enzyme cloning: After the variant sequence repertoires have been generated, regions that include the variant sequences can be cloned from one repertoire into another using conventional cloning methods. Recombining three or more repertoires requires the ligation of three or more fragments. This is inefficient when conventional restriction enzymes are used as the fragments can ligate in various order and in both orientations. However, one can increase the fraction of correctly assembled plasmids by recombining the variable segments in an iterative process which includes multiple two-piece ligations. This process is illustrated in Figure 4.

Other recombination methods: The individual variant sequence repertoires can be recombined using any of the available random recombination methods. Another way to recombine is to mix the plasmids encoding the various variant sequence repertoires and subject the mix to PCR using primers that sit outside of all variable segments. It is known that recombination occurs during conventional PCR. The frequency of recombination can be increased by applying very short extension times as described in Meyerhans et al., Nucleic Acids Res. 18:1687 (1990).

### Identification of modified enzymes that bind a target

From the library one can produce a mixture of the protein of interest containing different combinations of variant sequences. Optionally, the mixture can be purified. Variants of the protein that bind to the target can be enriched by passing the mixture over a column or other device carrying the immobilized target. Alternatively, the mixture can be incubated with the target to bind variants of interest. In another embodiment, the mixture is passed over an affinity column with the immobilized target and subsequently, the column is washed to remove variants with weak or moderate affinity for the target. To monitor the process the column can be washed with a solution containing a chromogenic or fluorogenic substrate and optionally a reversible inhibitor to monitor the amount of bound enzyme. This enables one to choose an appropriate washing duration.

It is possible to remove library members with undesired affinities for antitargets. Antitargets are molecules or structures that the final protein should not bind to. This allows one to identify variants that bind to the target with high selectivity. The removal of variant that bind to antitargets can be accomplished by incubating the library or an enriched sub-library with the antitarget. The antitarget can be immobilized to facilitate the process. If the target is bound to a carrier (*e.g*., resin, column, plastic or beads) during the affinity enrichment of binders then that carrier is likely to constitute an antitarget.

The identity of the enriched variants can be determined using any known method. For example, the identify can be determined using mass spectrometry. This may require the elution of the bound protein or one can directly analyze the bound material. The identity of the bound protein also can be determined using a combination of liquid chromatography and mass spectrometry. To simplify the latter analysis one can determine the LC/MS profile of the members of the variable segment repertoires. The MS or LC/MS analysis can be preceded by a proteolytic or chemical degradation step and the identity of the bound variants will then be deduced from the identity of the fragmentation products.

Screening for binders via random pooling: The library can be split into a number of pools. All these pools can be assayed for their contents of binding variants. This measurement can be performed similar to ELISA using microtiter plates that have been coated with the target protein. As a result one determines the population or the populations that contain the strongest binders. Subsequently, the positive populations can be further divided and screened until individual clones can be identified which can then be sequenced.

An alternative method of creating subpopulations is to individually construct the subpopulation such that all members of a subpopulation have one variable segment in common. By identifying the subpopulation that contains the best binder one will automatically have determined the nature of one variable fragment of the best binding variant. This deconvolution process can be repeated until the nature of all variable segments has been determined. This deconvolution strategy can be particularly useful if the binding assay has a relatively low throughput.

Phage or other display: A variety of methods have been described where protein libraries can be expressed on the surface of phage, cells, or ribosomes. These methods have in common that all library members carry the encoding DNA with them which can simplify the subsequent identification of binding variants.

In one embodiment of the present invention, a targeted_-lactamase is creating by cloning a large population of β-lactamase mutants into the phagemid vector pCB04. The plasmids can then be introduced into the XL-1 blue cells through electroporation. After super-infection with helper phage, such as M13K07, the XL-1 blue cells will produce infectious phage particles with β-lactamase-pIII (phage minor coat protein) fusion protein on the surface and the corresponding pCB04 plasmid inside of the phage particle.

The phage library can then be used to select specific binders for the targets. The method of bio-panning has been previously described in the literature (Barbas et al., Phage Display: A laboratory Manual Cold Spring Harbor Laboratory Press (2001)). Briefly, the phage library is first incubated with anti-targets (anything other than the intended target) to deplete binders to the anti-targets. After the depletion step, the resulting library is incubated with the targets. The unbound phage particles are washed away with buffer, and the bound phage particles are recovered by either acid elution or protease digestion (Ward et al., J. Immunol Methods, 1996, 189:73-82, Smith, Science, 1985 228: p. 1315-7, Smith et al., J Biol Chem, 1994 269: 32788-95, Clackson, et al., Nature, 1991 352: 624-28). The phage elution is then used to infect fresh XL-1 blue cells, followed by helper phage super-infection to amplify the library. The secondary library is used for a second round of bio-panning. The same process can be reiterated for multiple times until a specific binding phage clone is identified.

Once a library has been enriched for binders it can be transferred (by transformation or transfection) into a non-permissive host like TOP10 cells (Invitrogen). In non-permissive hosts translation of the lactamase will stop after the His6 sequence. The resulting enriched library can be subjected to a high throughput screen to identify individual clones with affinity for the target of interest.

### METHODS OF USING TARGETED ENZYMES

From the following, it will be clear to one of skill in the art that the targeted enzymes of this invention have many uses. For example, the enzymes can be used in the targeted release of prodrugs into tissues that carry a particular marker (*e.g.*, an antigen or receptor). Alternatively, the enzymes can be included in an analytical reagent similar to enzyme-antibody conjugates but with increased stability and diffusion and lower cost. The enzymes can also be used as surface catalysts, for example, a targeted laccase. Other uses include, *e.g.,* targeted generation of a compound (*e.g.,* H₂O₂ from glucose) and the targeted destruction of compounds (*e.g.*, a metabolite or signalling molecule from a particular tissue).

### PHARMACEUTICAL COMPOSITIONS

The targeted enzymes, nucleic acids encoding them, and prodrugs (also referred to herein as "active compounds") described herein can be incorporated into pharmaceutical compositions suitable for administration. Such compositions typically comprise the active compound and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

Described herein are methods for preparing pharmaceutical compositions for modulating the expression or activity of a targeted enzyme, prodrug (or its corresponding active drug) or nucleic acid of interest. Such methods comprise formulating a pharmaceutically acceptable carrier with an agent which modulates expression or activity of an active compound of interest. Such compositions can further include additional active agents. Thus, described herein are methods for preparing a pharmaceutical composition by formulating a pharmaceutically acceptable carrier with an agent that modulates expression or activity of a targeted enzyme, prodrug (or its corresponding active drug) or nucleic acid of interest and one or more additional active compounds.

A pharmaceutical composition is formulated to be compatible with its intended route of administration. Examples of routes of administration include parenteral, *e.g.*, intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose, pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, Cremophor ELJ (BASF; Parsippany, NJ) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressurized container or dispenser which contains a suitable propellant, *e.g.,* a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

As defined herein, a therapeutically effective amount of a targeted enzyme (*i.e.*, an effective dosage) is the amount of the targeted enzyme that is administered to a subject to produce a desired therapeutic effect in the subject. In the case of targeted enzymes to be used as part of targeted enzyme prodrug therapy applications, a therapeutically effective amount of the targeted enzyme is an amount sufficient to convert enough prodrug to active drug that a symptom of the disorder being treated is ameliorated.

Typically, the amount of targeted enzyme to be delivered to a subject will depend on a number of factors, including, for example, the route of administration, the activity of the targeted enzyme, the degree to which it is specifically targeted to the desired cells, tissues or organs of the subject, the length of time required to clear the non-specifically bound targeted enzyme from the subject, the desired therapeutic effect, the body mass of the subject, the age of the subject, the general health of the subject, the sex of the subject, the diet of the subject, the subject's immune response to the targeted enzyme, other medications or treatments being administered to the subject, the severity of the disease and the previous or future anticipated course of treatment.

For applications in which a prodrug also is administered, other factors affecting the determination of a therapeutically effective dose will include, for example, the amount of prodrug administered, the activity of the prodrug and its corresponding active drug, and the side effects or toxicities of the prodrug and the active drug.

Examples of ranges of mass of targeted enzyme/mass of subject include, for example, from about 0.001 to 30 mg/kg body weight, from about 0.01 to 25 mg/kg body weight, from about 0.1 to 20 mg/kg body weight, and from about 1 to 10 mg/kg, 2 to 9 mg/kg, 3 to 8 mg/kg, 4 to 7 mg/kg, or 5 to 6 mg/kg body weight.

In a particular example, a subject is treated with a targeted enzyme in the range of between about 0.1 to 20 mg/kg body weight, one time per week for between about 1 to 10 weeks, preferably between 2 to 8 weeks, more preferably between about 3 to 7 weeks, and even more preferably for about 4, 5, or 6 weeks. It will also be appreciated that the effective dosage of targeted enzyme may increase or decrease over the course of a particular treatment. Changes in dosage may result and become apparent from the results of diagnostic assays as described herein.

Administration of targeted enzyme may be systemic. Administration of targeted enzyme may be at or near the target to be bound.

A prodrug may also be administered to the subject. It is understood that appropriate doses of prodrugs depend upon a number of factors within the ken of the ordinarily skilled physician; veterinarian, or researcher. The dose(s) of the prodrug will depend, for example, on the same factors provided above as factors affecting the effective dose of the targeted enzyme. Exemplary doses include milligram or microgram amounts of the prodrug per kilogram of subject or sample weight (*e.g*., about 1 microgram per kilogram to about 500 milligrams per kilogram, about 100 micrograms per kilogram to about 5 milligrams per kilogram, or about 1 microgram per kilogram to about 50 micrograms per kilogram. It is furthermore understood that appropriate doses of a prodrug depend upon the potency of the prodrug with respect to the desired therapeutic effect. When one or more of these prodrugs is to be administered to an animal (*e.g*., a human), a physician, veterinarian, or researcher may, for example, prescribe a relatively low dose at first, subsequently increasing the dose until an appropriate response is obtained.

The timing of administration of the prodrug is another important factor to be considered. Preferably, the targeted enzyme is administered to the subject, then the prodrug is administered. More preferably, the time between the administration of the targeted enzyme and administration of the prodrug is sufficient to allow the prodrug to accumulate at its target site by binding to its target, and to allow unbound targeted enzyme to be cleared from the non-targeted portions of the subject's body. Most preferably, the ratio of target-bound targeted enzyme to unbound targeted enzyme in the subject's body will be at or near its maximum when the prodrug is administered. The time necessary after administration of the targeted enzyme to reach this point is called the clearing time. The clearing time can be determined or approximated in an experimental system by, for example, administering a detectable targeted enzyme (*e.g.*, a radiolabeled or fluorescently labeled targeted enzyme) to a subject and simultaneously measuring the amount of enzyme at the target site and at a non-targeted control site at timed intervals. For some prodrugs, particularly those whose counterpart active drugs are highly toxic, it may be more important to ensure that the levels of unbound targeted enzyme in the subject's system are below a certain threshold. This too can be determined experimentally, as described above.

Administration of the prodrug may be systemic. Administration of the prodrug may be at or near the target to be bound.

The pharmaceutical compositions can be included in a container, pack, dispenser or kit together with instructions for administration.

### EXAMPLES

The following examples are submitted for illustrative purposes only and should not be interpreted as limiting the invention in any way.

### Example 1: Selection of variable loops in β-lactamase (BLA)

This example demonstrates that variation-tolerant sequences in a β-lactamase can be identified and replaced with repertoires of variant sequences.

The p99 β-lactamase of *E. cloacae* (pdb accession # IBLS) has the sequence as illustrated in Figure I with the 20 amino acid residue pro-sequence deleted. This structure was inspected manually to identify residues that appeared to be on the surface and not involved in defined secondary structure and these residues are in bold. Active site residues are marked with *. Loops at amino acid residues 116 - 127, and 295 - 306 are in the vicinity of the active site. The structure was compared to a close homologue IGCE (69% homology) and there was no structural divergence at 1.5Å. The structure was also compared to a remote homologue 1 PTE (20% homology). The regions that were structurally unconserved are marked in italic5. Various insertions and deletions are allowed based on this homology.

Loop Modeling: The variable loops of an antibody (1SM3) to a tumor antigen peptide were modeled onto p99. This was unsuccessful due to the differing topology of the two molecules. P99 was then inspected for potential loop variable and loop insertion sites based on the approximate distances apart and structural motifs of 1SM3 heavy chain. The antibody CDRs all form connecting strands between β-sheets. The distance between the 3 loops in 1SM3.H are 4 - 9Å and 6 - 10Å, depending where the measurements are made.

The following loops were picked as possible variation-tolerant sequences in P99 (*see* Figure 1):
Loop A: Between residue Y34 and K37. Twelve residues of the 14 from CDR2 of 1SM3 were modeled in. The modeling indicated that 5 B 12 residues be engineered into this region.
LoopB: Between N302 and S311. Nine residues of the 10 from the extended CDR1 of 1 SM3 were modeled in. The modeling indicated that 7-10 residues be engineered into this region (*i.e.* minimal resultant loop length change). Residues 297 - 302 (with the exception of 298 which has a buried side-chain) were also indicated to be amenable to change.
Loop C: Between residues P330 and Q333. Six residues of the 7 from CDR3 of 1SM3 were modeled in. The modeling indicated that 5 - 8 residues be engineered into this region.

Two other extended regions are on the same face as Loops A, B and C that are amenable to change: Loop D, between residue E241 and L248, and Loop E, between residues M273 and A280. It is indicated that 6-10 residues be engineered into these regions.

Loops A, B, and C interact (~8-10Å), A, C, and D interact (14 Å without insertion into D), and B, C, and E interact (10 Å without insertion into D). Residues 279-309 are deleted in the homologous (dipeptidase) structure 1PTE.

Construction of a Synthetic BLA Gene: The plasmid pK1841 was constructed from pK184 *(see* Jobling et al. (1990) Nucleic Acids Res 18: 5315-6) by deleting its lacZ gene and introducing *Eco*RI and *Sal*I restriction sites using a PCR-based method. A portion of pK184 was amplified using the primers:

### 1841F:

### 1841R:

Two µl of each primer (25 µM) were combined with 10 µl 10x pfu buffer, 3 µl dNTP (10 mM), 2 µl pK184, 2 µl pfu TURBOJ and 80 µl H₂O (all reagents and enzymes from Stratagene, La Jolla, CA). The reaction was run through 16 cycles, wherein each cycle consisted of 30s at 95E C, 1 min at 55E C and 6 min at 68E C. Then 1 µl of *Dpn*I (Roche, Indianapolis, IN) was added to the PCR products to digest template DNA. Five µl of the resulting mix was used to transform 50 µl of chemical competent TOP 10 cells (Invitrogen, Carlsbad, CA) and the transformation plated on LA+50ppm Kan plates. The plates were incubated at 37E C overnight. Eight colonies were picked and plasmids isolated using a Qiagen miniprep kit (Qiagen, Valencia, CA). The isolated plasmids were run on 1.2% agarose e-gel (Invitrogen) in parallel with pK184 and two of them were confirmed by sequencing. These were named pK1841.

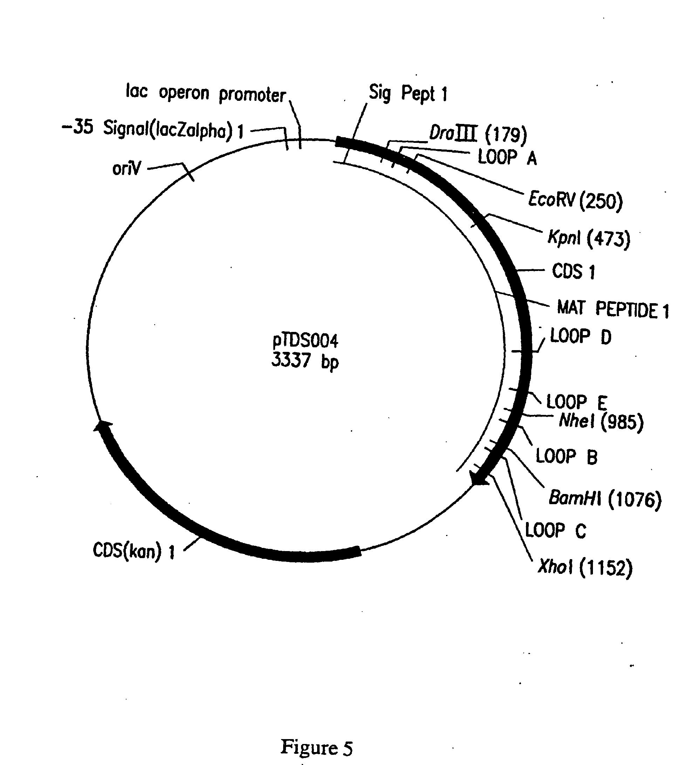

pTDS004 (Figure 5) was constructed by subcloning a synthetic AmpC gene from pPCRSCRIPTJ (Aptagen, Herndon, VA) into pK1841. The synthetic AmpC gene encodes the amino acid sequence of the *E. cloacae* P99 ampC gene, but it has unique restriction sites between the variable loops. In particular, type IIS enzymes were chosen which generate non-palindromic overhangs. No amino acid changes were introduced.

In separate reactions, 2 µg each of pK1841 and pPCRSCRIPTJ-AmpC were digested with 20 units of *Eco*RI and *Sal*I (Roche) in 50 µl at 37E C for two hours. Digests were run on 1.2% e-gel, and a 2.1 kb fragment from pK1841 and a 1.2 kb fragment from the pPCRSCRIPTJ AmpC gene were gel purified using the Qiagen gel purification kit. One-hundred µg of digested vector pK 1841 was ligated with 120 ng insert from pPCRSCRIPTJ-AmpC using Takara ligase (Panvera, Madison, WI) at 16E C overnight. Five µl of ligation mix was used to transform 50 µl chemical competent TOP 10 cells (Invitrogen), and plated on LA+50ppm Kan and LA + 50ppm Kan + 0.5ppm cefotaxime (CTX, Sigma, St. Louis, MO). The plates were incubated at 37E C overnight. Six colonies were picked from LA + 50ppm Kan plates, and plasmids were isolated using a Qiagen miniprep kit. *Hind*III and *Bam*HI were used to digest plasmid to determine which colonies had the correct plasmid construction. A typical digest was done using 0.2 µg plasmid and 2.5 units of each enzyme in a volume of 20 µl and incubating at 37E C for 1 hr. Correct plasmids gave bands of 2.3 kb and 1 kb fragment on an e-gel. Two apparently correct plasmids were confirmed by sequencing and named pTDS004.

pTDS004 contains a P_{lac} promoter and the native ampC promoter in front of the ampC coding sequence. As a control an equivalent plasmid was constructed carrying the wild-type nucleotide sequence of *E. cloacae* ampC. When grown in LB medium strains carrying both plasmids produced similar amounts of nitrocefin activity, which indicates that the synthetic gene is fully functional.

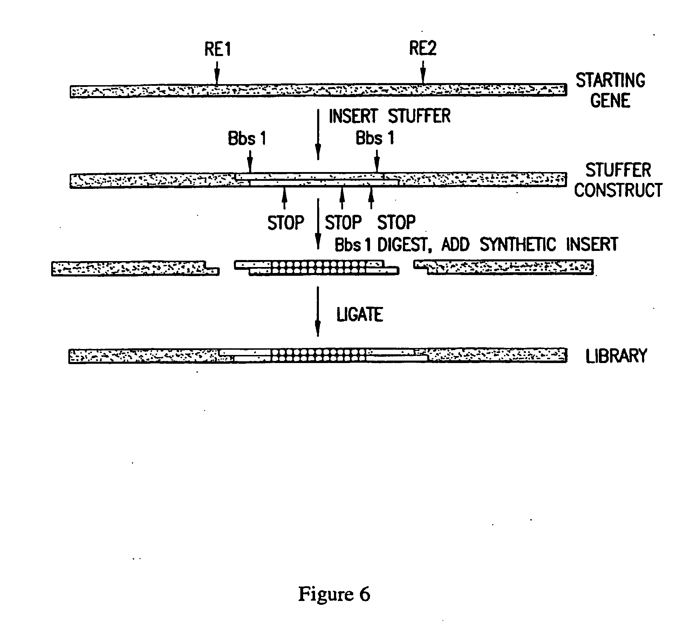

A two-step cloning strategy was developed which allows randomization of individual loops while minimizing the fraction of unmutated vector in the resulting populations. In the first step, a stuffer sequence was introduced that contained at least one stop codon and two *Bbs* I sites. The stuffer sequence used should provide restriction sites and lead to inactivation of the gene via, for example, frame shifts or stop codons. In the second step, the stuffer was cut with *Bbs* I and a synthetic cassette containing partially randomized oligonucleotides was inserted. The process is illustrated in Figure 6 and this scheme was used to modify loops A, B, C, and D. In all cases between 10⁴ and 10⁷ transformants were obtained.

Oligonucleotides: The following oligonucleotides were used to modify each loop. In addition to the standard nucleotide abbreviations, N denotes an equimolar mix of A, C, G and T; D denotes an equimolar mix of A, G, and T; H denotes an equimolar mix of A, C, and T; S denotes an equimolar mix of C and G.

### Loop A

### Mutagenic primers for constructing pME20P (A8):

### LoopA-A118-F:

### LoopA-A118-R:

### Loop B

### LoopB Stuffer:

### Mutagenic primers for constructing pAL14P (B8):

### LoopB-A118-F:

### LoopB-all8-R:

### Mutagenic primers for constructing pAL16P (B14):

### LoopB-All14-F:

### LoopB-A1114-R:

### Mutagenic primers for constructing pAL18P (B14 focused):

### LB_6K7:

### LB_anneal1:

### LB_anneal2:

### Loop D

### Loop D stuffer:

### LDstuff1:

### LDstuff2:

### LDstuff3:

### Mutagenic primers for constructing pME28P (D6):

### LDa116f:

### LDall6r:

### Mutagenic primers for constructing pME29P (D10):

### LDall10f:

### LDall10r:

### Libraries were constructed as follows:

### Construction of pME20P (Primary Library)

The plasmid pTDS004 was cut with the enzymes *Dra*III and *Eco*RV*,* and the vector fragment (3266 bp) was gel purified from a 1% agarose gel. Two complimentary oligos (LA_Stuf1 and LA_Stuf2, below) were annealed together, which contain *Bbs*I sites for cloning. Once annealed the oligos have ends compatible with *Dra*III and *EcoR*V (blunt) ends. 12.5 µg of each oligo was combined and the volume was brought up to 50 µl with Tris pH 8.5. The mixture was heated at 95E C for 5 minutes in a heat block, then the heat block was turned off and the mixture was allowed to cool down to room temperature.

### Oligonucleotide sequences:

### LA_Stufl/LA_Stuf2:

The gel purified vector (3.2 kb) was ligated to the annealed insert (approximately 84bp) in a 1:5 vector: insert molar ratio. 90 ng of vector and 9.5 ng of insert were used (99.5ng total). The vector and insert mixture was brought up to 10 µl using Tris pH 8.5, 10 µl of Takara Solution I (Panvera, Madison, WI) was added and the mixture was annealed at 16E C for four hrs in a MJ research PCR machine (Waltham, MA). A vector-only control was set up the same way using the 3.2 kb fragment and Tris pH 8.5 up to ten µl and adding ten µl of Takara Solution I. Ligation reactions were purified using the DNA Clean & Concentrator kit (Zymo Research, Orange, CA). DNA was eluted from columns in two spins, using six µl of water each time (10-12 µl total). Five µl of purified ligation was transformed into 50 µl of Top 10 electrocompetent cells (Invitrogen, Carlsbad, CA) and recovered in 250 µl SOC for onehr. The same was done for the control. Half of transformation was plated on large LA + 50ppm Kan plate, the other half on LA + 0.5ppm CTX. No colonies were expected to grow on CTX because the insert should disrupt the gene. Plates were incubated overnight at 37E C. Four colonies were picked from LA + 50ppm Kan plates and grown overnight in five ml LB + 50ppm Kan. Miniprep DNA was made from the cultures. Pure DNA from each clone was digested with *Bbs*I (2 sites contained in insert) to identify the correct construct. All eight clones contained the insert of interest, one was confirmed by sequencing, and this construct was named pME 17.

### Library construction:

2.5 µg of pME17 was 20-fold overdigested with ten µl *Bbs*I in a 100 µl reaction, creating one 3267 bp fragment and one 75 bp fragment. The 3.2kb fragment was gel purified form a 1% agarose gel using the Qiagen purification kit. Library insert of annealed oligonucleotides was prepared exactly as described above.

### Oligonucleotide sequences:

### LA_Lib_A1181

A 100 ng ligation was set up in a 1:5 vector: insert molar ratio using 96 ng vector (3.2kb) and 12 ng insert (approximtely90 bp). DNA was mixed together and brought up to 10 µl using Tris pH 8.5. A vector alone control was also set up substituting Tris for insert volume. Ten µl of Takara Solution I was added, and reactions incubated overnight at 16E C in MJ research machine. Overnight ligations were purified with DNA Clean & Concentrator kit and DNA was eluted in two spins, 6 µl water each (10-12.) Five µl (approximately 27 ng) of each purified ligation was transformed into 50 µl Top 10 electrocompetent cells, and recovered in 250 µl SOC for 1 hour. Transformations for both library and control were plated undiluted (50 µl, or 1/6 transformation volume), diluted 1/10, and diluted 1/100 on both LA + 50ppm Kan and LA + 0.5ppm CTX large plates. The transformation mixture was spread using 10-15 glass beads per plate. Plates incubated overnight at 37E C. The total number of colony forming units obtained was 2.6x10⁴ for LA( 50 ppm kan) and 2.5x10⁴ for LA(0.5 ppm CTX). Since one transformation yielded approximately 30,000 active colonies (on LA + 50ppm Kan + 0.5ppm CTX plates) this process was scaled up so four transformations were performed to yield approximately 100,000 colonies on Kan + CTX plates. The 22 resulting LA + 50ppm Kan + 0.5ppm CTX plates from the four transformations were scraped using 2ml LB + 50ppm Kan per plate and a cell scraper. Total diversity was 2.0 E +05. Scraped colonies from each plate were pooled together, and 36ml total volume was recovered. Optical density was measured at OD₆₀₀ and 15ml of 50% glycerol was added to pooled colonies for a final 15% glycerol concentration. Two ml aliquots were frozen at -80E C.

### Construction of pAL16P (primary library):

### Construction of pTDS004BS, B loop stuffer plasmid:

pTDS004BS was constructed using the same method as for as pME17, the A loop stuffer, with the following modifications:

*Nhe*I and *Bam*HI was used to cut pTDS004 and a 3246bp fragment was gel purified.

The two complementary stuffer oligos are (74bp each):

### LB_stuf1/LB_stuf2:

### Construction of pAL16P (B loop Library):

The method of constructing pAL16P using two oligonucleotides was the same as the construction of pME20P except the complementary two oligonucleotides used for insert are: LB_Al16-1/LB_Al16-2:

The total number of colony forming units obtained was 4.7x10⁵ for LA(50 ppm kan) and 3.1x10⁵ for LA(0.5 ppm CTX).

For the construction of pAL16P we also tested a method where the inserted region is comprised of three oligos. The 3 oligos are:
LB_All6-1 d (above)
LB_anneall:
LB_anneal2:

Oligos LB_anneal 1 and LB_anneal2 can anneal with the ends of oligo LB_A116-1. In the annealing reaction, 1.5 fold more LB_anneal1 and LB_anneal2 were used relative to LB_A116-1.

After ligation, Klenow fragment and dNTPs were added to the ligation mixture to fill in the 42bp gap on the plasmid at 37E C for two hrs. This approach resulted in about twofold more transformants compared to the protocol where only two oligos were used for insertion.

The resulting library was grown on LA plates containing 0.5 ppm CTX to select variants that exhibited BLA activity. Nintey-six clones were randomly chosen and submitted for DNA sequencing. Eighty-nine clones gave interpretable sequences. Eighty-seven clones exhibited sequences that were expected to be in the library. Two clones had frame shifts, which were likely the result of sequencing errors.

The sequences below represent an example of 10 sequences obtained from library pAL16P. The 14 random positions of the B loop are highlighted. The first line of the alignment shows the sequence of the wild-type BLA.

### Construction of pAL18P (focused B Loop Library):

pAL18P is a B loop library with 14 amino acids XZXZXZKZXZXZXZ, where X represents F, I, V, S, T, A, Y, N or D and Z represents V,A,E,G,L,P,Q, or R. The construction of pAL18P was similar as pAL16P by starting with the same stuffer plasmid pTDS004BS. However, the synthetic insert was encompassed the following three oligonucleotides:
LB_6K7:
LB_anneal 1 :
LB_anneal2:

During ligation the oligonucleotide LB_6K7 was used in 5 fold excess relative to the cut vector and the oligonucleotides LB_anneal1 and LB_anneal2 were used in 7.5 fold excess relative to the cut vector.

After ligation, Klenow (Roche, Indianapolis, IN) and dNTPs (Roche, Indianapolis, IN) were added in concentrations recommended by the manufacturer in order to fill in the 42 bp gap in the plasmid at 37E C for two hrs. Subsequently, the DNA was purified and transformed into TOP10 cell as described for library pME20P. The total number of active clones was 2.4e5 on LA+0.5ppm CTX.

### Construction of pME27P (recombined library):

Two ml of frozen pME20P library was grown in 100m1 of LB + 50ppm Kan in a 1 liter flask and shaken at 37E C for 4 hours. The same was done for the pAL16P library. DNA was purified using Qiagen miniprep kit, and five µg of each library was digested with *Bgl*I and *Dra*III simultaneously overnight at 37E C. Digest produces two bands, one 2.6 kb, the other 660 bp. The 2.6 kb piece was taken from Loop B library, and the 660 bp piece was taken from the Loop A library.

A second digest was performed on the loop B library with enzymes located within the 660 bp piece in case of incomplete digestion, eliminating possible background from linear DNA. Both *Mlu*I and *Sph*I were added to the digest and incubated overnight at 37E C. Digests were run out on a 1% gel and 660 bp fragment from Loop A library and 2.6 kb fragment from Loop B library were gel purified using a Qiagen gel purification kit. DNA was eluted in 50 µl water. Using the 660 bp band from the Loop A library, and the 2.6 kb band from the Loop B library, the two fragments were ligated together in a 1:4 vector : insert ratio. 145 ng of 2640 bp fragment and 36.25 ng of 660 bp fragment were combined and the volume was brought up to 10 µl with Tris pH 8.5. A vector only control (2.6 kb fragment) was set up in the same manner, substituting Tris for insert volume. 10 µl of Takara Solution one was added to each mixture, and ligations were incubated at 16E C overnight in MJ PCR machine. Overnight ligations were purified using the DNA Clean & Concentrator kit. DNA was eluted in two spins, eight µl each spin (14-16 µl). Five µl (30 ng) of both library and control ligations was transformed into 50 µl of Top 10 electrocompetent cells, recovered in 250 µl SOC, shaken at 37E C for one hour. Both transformations were plated 50 µl (1/6 transformation) straight, 10-1, and 10-2 on large LA + 1 OppmKan and LA +0.5ppm CTX and incubated overnight at 37E C. The total number of colony forming units obtained was 6x10⁵ for LA(50 ppm kan) and 6.6x10⁴ for LA(0.5 ppm CTX).

### Construction of pME30P (re-recombined library):

The recombination as described for pME27P resulted in a significant number of clones not resistant to CTX indicating that some of the recombinants did not yield a fully functional enzyme. Therefore, the plasmid mixture encoding pME27P was cleaved and re-ligated to generate novel combinations between the variant sequences contained in pME27. The process of re-recombination is very efficient because there was no need to purify the plasmid fragments after digestion, which avoids loss, and the molar ratio between the restriction fragments is exactly one to one which favors complete re-ligation. This example re-recombined two variant segment repertoires but the process can be applied for a larger number of variant segments.

One ml of frozen pME27P library was thawed and grown between two 250ml LB + 10ppm Kan cultures in 1L shake flasks for 4-5 hours at 37E C. Cultures were spun down, and pellets used for Qiagen maxiprep to obtain pure library DNA. 250ng of library DNA was digested with *Dra*III and *Bgl*I (same enzymes used to create pME27P) in a 20 µl reaction. A control was also set up using the same amount of DNA, but to which ligase was not added. Both digests were incubated at 37E C overnight. Five µl of the reactions were run on a 1.2% agarose e-gel to confirm digestion, then enzymes were heat inactivated at 65E C for 20 min. To the remaining 15 µl of the digests, 15 µl of Takara ligase Solution I was added to one digest, and 15 µl Tris pH 8.5 added to the control. Reactions were incubated overnight in MJ PCR machine at 16E C. Overnight ligations were selected again by digesting with *Nhe* I and *Eco*RV*,* because these sites should be destroyed when either A or B library fragment are both present in the vector. This step eliminates wild-type background. Ligations were digested at 37E C for 3.5 hours. Ligations were purified using the DNA Clean & Concentrator kit. DNA was eluted in two spins, eight µl each spin (14-16 µl.) The library and control were both transformed by adding five µl (22ng) to 50 µl of Top 10 electrocompetent cells, recovering in 250 µl SOC for one hr, and 100 µl (1/6 of transformation) of 10-land 10-2 dilutions were plated on large LA + 0.2ppm CTX. 20 µl (1/30) was plated on small LA + 10ppm Kan plates. All plates incubated overnight at 37E C. Remaining transformation was frozen down at -80E C with 50% glycerol. The total number of colony forming units obtained was 1.5x10⁶ for LA(50 ppm kan) and 1.6x10⁶ for LA(0.5 ppm CTX).

Modifications of loops A, B or D led to a large fraction of variants that still conferred resistance to cefotaxime (5-50%). Modification of loop C led to inactive variants. Table 2 lists some of the constructed loop libraries.

**TABLE 2**

| name | Randomization (d) | parent | % functional (c) | number of functional transformants |
|---|---|---|---|---|
| PAL14P | B8 | pTDS004 | ca 70 | |
| pAL16P | B14 | pTDS004 | ca 30 | 3x10⁵ |
| pAL18P | B14 focused (a) | pTDS004 | ca 70 | 3x10⁵ |
| pME20P | A8 | pTDS004 | 5-10 | 1x10⁵ |
| pME27P | A8+B14 (b) | pTDS004 | 11-33 | 1.2x10⁵ |
| pME28P | D6 | pTDS004 | 37 | 2x10⁵ |
| pME29P | D10 | pTDS004 | 40 | |
| pCB04-AL8 | A8 | pCB04 | 6 | 1x10⁵ |
| pME30 | A8 + B14 (e) | pTDS004 | 99 | 1.6x 10⁶ |

(a) This library contains limited diversity. Some positions allow only 8 different amino acids and other positions allow 9 amino acids. Position 7 is lysine only. This library facilitates sequencing of enriched clones by mass spectrometry.
(b) Two libraries, pAL16P and pME20P, were recombined. This library contains variants which differ from each other in 22 positions.
(c) The percentage of total clones that have a functional β-lactamase gene was determined either by isolating random clones and testing growth on ctx-agar or by plating libraries on agar containing an antibiotic that selects for the presence of the vector and in parallel on agar containing the same antibiotic and ctx.
(d) This column indicates the randomized variation-tolerant sequence (loop A, B, D) and how many positions were randomized (indicated by the number following the loop designation) .
(e) This library was made by re-recombining loops A and B from pME27P.

Ninety-six clones from most libraries were sequenced to validate the mutagenesis procedure and to identify bias which could result from the oligonucleotide synthesis, the cloning procedure, and antibiotic selection. Greater than 500 clones from various libraries were sequenced, and it was observed that between 50-95% of the sequences conformed with the expected randomization scheme.

Recombining variation-tolerant sequence A and B repertoires yielded between 6 and 33% functional genes. Ten variants were randomly isolated from this population and it was confirmed that 9 of the 10 variants contained variant sequences in both the A and B positions. This is evidence that the generation of libraries of variants containing several variant sequences can be achieved.

### Example 2 - Expression and Purification of BLA.

This example demonstrates that milligram quantities of targeted β-lactamase BLA) molecules made according to the invention can be expressed and purified.

Enzyme production was tested from 10 BLA variants that were chosen from the libraries pAL14P and pME20P. Some of the variants result in low BLA production at 37E C. This may be caused by proteolytic degradation. All clones produced at least 50% activity compared to the wild-type strain when the variants were grown at 25E C. Therefore most mutants, which confer ctx resistance, can produce sufficient enzyme for further analysis and to identify desired targeting characteristics.

### Example 3: Affinity Enrichment of Streptavidin-binding BLA Variants

This example demonstrates that the methods of the invention can be used to created targeted β-lactamase enzymes that retain catalytic activity.

### Preparation of Samples

Library Production: A 250 ml flasks filled with Terrific Broth (12 g/l bactotryptone, 24 g/l bacto yeast extract, 4 ml glycerol, 17 mM KH₂PO₄, and 72 mM K₂HPO₄) + 50 ppm Kanamycin, was inoculated with a scraping of a frozen stock of the pAL16P1 library, serially diluted 1/26 and 1/676, and grown at 25E C, shaking at 280 rpm. Multiple dilutions were done to ensure proper harvest time at the initiation of stationary phase. Optical density was measured at 600 nm at 18 hours (measured 23.8). The remaining volume (~21 ml) was harvested by centrifuging at 7k rpm (~4k gravity) for 20 minutes and the supernatant fraction decanted. The pellet was resuspended in 4 ml buffer A (20% sucrose (m/v), 200 mM triethaolamine, 100 mM EDTA, pH = 7) and rotated for 20 mintues at 4E C to begin osmotic shock of the periplasmic space. The sample was centrifuged again at 7k rpm and the supernatant fraction decanted. The pellet was resuspended in 4 ml buffer B (20 mM triethanolamine, 0.5 M NaCl, pH = 7) and rotated for 20 minutes. The supernatant fraction was collected.

The wild type β-lactamase was produced using the same protocol.

Library Purification: An affinity-based purification was used. The chosen resin is specific to the active site of β-lactamases.

A five ml column of p-aminophenylboronic acid linked to an agarose resin (Sigma Chemical Co., St. Louis, MO, Cat. No. A 8530) using a 14 cm, 20 ml max bed polypropylene BIO-RADJ column (Bio-Rad, Hercules, CA, Cat. No. 732-1010).

The column was filled and packed with the supplied porous frit to ~3.5 ml. The column was conditioned with 10 ml 1 M NaCl, 0.5 M Sorbitol, pH = 7, then 10 ml 0.5 M Borate, pH = 7, and finally 10 ml 20 mM triethanolamine, 0.5 M NaCl, pH = 7. The column was stored in this buffer. The fluid reservoir was drained prior to purification.

3.5 ml of the periplasmic product was loaded onto the column, which was then allowed to drain completely. The column was washed with 3.5 ml of 20 mM triethanolamine, 0.5 M NaCl, pH = 7 loading buffer completely drained. Bound protein was eluted with 3.5 ml 0.5 M Borate and fractions were collected. The column was reconditioned with an additional volume of the same buffer. Finally, >5 ml 20 mM triethanolamine, 0.5 M NaCl, pH = 7 loading buffer was flowed through the column, which was then stored.

The concentration of each of the collected fractions was determined in the elution fraction and the β-lactamase activity measured using the nitrocefin substrate (Oxoid BR0063A) in the standard protocol: A substrate solution containing PBS (phosphate buffered saline); 1.25 g/l n-octyl-β-D-glucopyranoside, 100 mg/l nitrocefin and 1 g/l DMSO (dimethylsulfoxide) was used. The reaction was monitored at 25E C in microtiter plates containing a total volume of 210 µl per well. The absorbance at 486 nm was monitored using a Molecular Devices (Sunnyvale, CA) plate reader. The assay was calibrated using a purified sample of BLA and based on its absorbance at 280 nm. In this assay, one unit of activity is defined as the amount of BLA activity that produces a rate of 1 mO.D.₂₈₀/min. Wild-type BLA from *E. cloacae* has a specific activity of 3.6 U/ng protein.

The library and the purified wild-type β-lactamase stock were run on a PAGE-gel to visualize purity. Total yield was ~100 µg purified library.

The library was then tested for binding to streptavidin, a molecule not bound by wild type β-lactamase.

Equipment: Two Amersham Pharmacia HiTrap Streptavidin HP, 1 ml columns (Cat. No. 17-5112-01) with the included fittings were used. A Rainin DYNAMAXJ peristaltic pump (RP-1) (Rainin, Emeryville, CA) with a multi-channel head was used to drive both colums with appropriate gauge Rainin tubing for the desired flow rate. Two Pharmacia circular fraction collectors (Frac-100) were used for sample collection.

Method: The chromatography apparatus was constructed such that there would be minimal delay between sample injection and column loading and with negligible post-column dead-space. A valve was inserted to switch between sample loading and flow. 500 µl of a 21 mg/l stock of both the purified pAL16P1 library and WT β-lactamase were injected into the flow line at approximately 1 ml/min for a total loading amount of 10.5 µg, followed by the sample with 500 µl of the running buffer from the same syringe. The valve was reset the system run at ~ 1 ml/hr (1.89 rpm in this system). A one ml fraction was collected each hour. Fractions were assayed for β-lactamase activity using the nitrocefin substrate. 300 µl of the first ten fractions were serially diluted at 0.4x from 1 to 1.7e-5. A 180 µl sample was assayed with 20 µl substrate (1.8 mg/ml (3.5 mM) in 0.125% n-Octyl-beta-D-glucopyranoside in Phosphate-buffered Saline). Samples past fraction nine had no detectable activity.

Results: The activity values were calculated relative to the activity of the samples that were loaded into the column and are given in Table 3.

**TABLE 3**

| Fraction | WT | pAL16P1 |
|---|---|---|
| 1 | 4.18% | 1.87% |
| 2 | 36.56% | 17.83% |
| 3 | 21.31% | 18.88% |
| 4 | 1.49% | 8.85% |
| 5 | 0.04% | 6.44% |
| 6 | 0.00% | 1.14% |
| 7 | 0.00% | 0.43% |
| 8 | 0.00% | 0.02% |
| 9 | 0.00% | 0.00% |

These results demonstrate that the library of modified β-lactamase enzymes encoded by pAL16P1 comprises targeted enzymes that, unlike wild type β-lactamase, bind to a target, streptavidin, and so elute in later fractions than the wild type β-lactamase, yet retain their catalytic activity.

### Construction of a β-lactamase Phage Library:

The following example describes the successful generation of a BLA phage library comprising BLA enzymes modified within variation tolerant sequences.

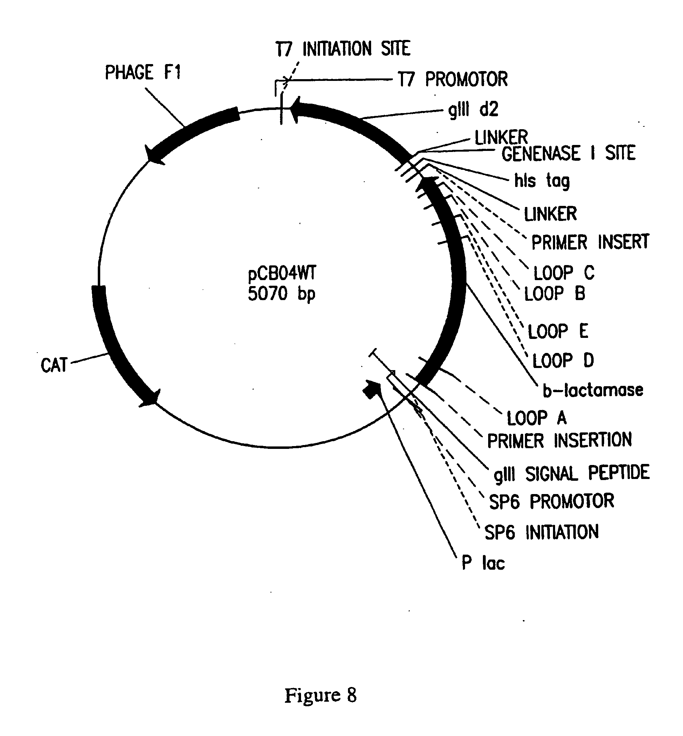

A gene encoding the p99 β-lactamase was subcloned into phagemid vector pCB04 to create pCB04WT. *See* Figure 8. pCB04 was used to make the PCB04-BL14 library as follows:

A synthetic BLA gene containing the B-loop stuffer fragment was cloned into pCB04 between the *Spe*I and *Ava*I sites. The clone was digested with *Bbs*I, and the vector fragment was purified by gel electrophoresis. The library was generated as described above for the pAL16P library. The ligated DNA was then purified and used to transform XL-1F' blue cells.

A fraction of the transformed cells was plated onto agar plates containing either five mg/ml CMP or 5 mg/ml CMP + 0.1 mg/ml CTX. The percentage of active clones was similar to that of pAL16P library. The diversity of the library was calculated based on the total number of active clones on the 5 mg/ml CMP + 0.1 mg/ml CTX plate.

The rest of the transformed cells were cultured for 6 hr at 37E C in the presence of five mg/ml CMP, 10 mg/ml tetracycline, and 0.1 mg/ml CTX with shaking. The cell density was determined by spectrometer (OD₆₀₀). The cells were then infected with 10 times more M13K07 helper phage (Invitrogen) and incubated for 30 min at 37E C without shaking. The total culture volume was then brought up to 250 ml with fresh LB media. The final antibiotic concentration was also adjusted to 5 mg/ml CMP, 10 mg/ml tetracycline, and 0.1 mg/ml CTX. The culture was incubated at 23E C for 48 hr with shaking. The phage preparation and subsequent titering were done using the protocol of Barbas et al., Phage Display: A Laboratory Manual, 2001, Cold Spring Harbor Laboratory Press.

## Claims

1. A method of making a targeted enzyme, comprising
a) selecting a polynucleotide encoding a modified enzyme, the modified enzyme having a peptide inserted into it relative to a corresponding unmodified enzyme, wherein the unmodified enzyme and the selected modified enzyme have a measurable enzymatic activity, and
b) replacing the nucleotides encoding the inserted peptide in the polynucleotide with an oligonucleotide encoding a targeting peptide that binds a target, wherein the enzyme with the targeting peptide has the measurable enzymatic activity and binds the target,
c) expressing the targeted enzyme from the polynucleotide in a protein expression system.

2. The method of claim 1, wherein the target is a molecule associated with the outer surface of a cell.

3. The method of claim 2, wherein the molecule is selected from the group consisting of a protein, a peptide, a nucleic acid, a carbohydrate, a lipid, a polysaccharide, a glycoprotein, a hormone, a receptor, an antigen, an antibody, a toxic substance, a metabolite, an inhibitor, a drug, a dye, a nutrient, a growth factor and a molecule which is part of the extracellular matrix.

4. The method of claim 2 or claim 3, wherein the cell is a diseased cell.

5. The method of claim 4, wherein the diseased cell is a cancer cell or an infected cell.

6. The method of any one of the preceding claims, further comprising making the polynucleotide of step (a) by inserting an oligonucleotide encoding said peptide into a polynucleotide encoding said corresponding unmodified enzyme.

7. The method of claim 6, wherein the oligonucleotide encoding said peptide is inserted into the polynucleotide encoding said corresponding unmodified enzyme at a random position.

8. The method of claims 1 to 5, wherein the polynucleotide encoding the modified enzyme having a measurable enzymatic activity is selected from a library of polynucleotides encoding modified enzymes, said library comprising polynucleotides encoding modified enzymes that have an inserted peptide that a corresponding unmodified enzyme does not have, and said unmodified enzyme having the measurable enzymatic activity.

9. The method of claim 8 further comprising making the library of polynucleotides encoding the modified enzymes by inserting an oligonucleotide encoding a peptide into the polynucleotide encoding the unmodified enzyme.

10. The method of claims 1 to 5, further comprising repeating steps (a) and (b) such that an enzyme is produced that has the measurable enzymatic activity and has a plurality of targeting peptides.

11. The method of claim 10, wherein two or more of the plurality of targeting peptides bind the same target.

12. The method of claim 10, wherein two or more of the plurality of targeting peptides bind different targets.

13. A method of making a polynucleotide encoding a targeted enzyme, the method comprising
a) selecting a polynucleotide encoding a modified enzyme, the modified enzyme having a peptide inserted into it relative to a corresponding unmodified enzyme, wherein the unmodified enzyme and the selected modified enzyme have a measurable enzymatic activity, and
b) replacing the nucleotides encoding the inserted peptide in the polynucleotide with an oligonucleotide encoding a targeting peptide that binds a target, wherein the enzyme with the targeting peptide has the measurable enzymatic activity and binds the target.

14. The method of claim 13, wherein the target is a molecule associated with the outer surface of a cell.

15. The method of claim 14, wherein the molecule is selected from the group consisting of a protein, a peptide, a nucleic acid, a carbohydrate, a lipid, a polysaccharide, a glycoprotein, a hormone, a receptor, an antigen, an antibody, a toxic substance, a metabolite, an inhibitor, a drug, a dye, a nutrient, a growth factor and a molecule which is part of the extracellular matrix.

16. The method of claim 14 or claim 15, wherein the cell is a diseased cell.

17. The method of claim 16, wherein the diseased cell is a cancer cell or an infected cell.

## Patentansprüche

1. Verfahren zur Herstellung eines zielgerichteten Enzyms, umfassend:
a) das Selektieren eines Polynukleotids, das ein modifiziertes Enzym codiert, wobei in das modifizierte Enzym im Vergleich zu einem entsprechenden nicht modifizierten Enzyms ein Peptid eingefügt ist, wobei das nicht modifizierte Enzym und das selektierte modifizierte Enzym eine messbare enzymatische Aktivität aufweisen, und
b) das Ersetzen der Nukleotide, die das in das Polynukleotid inserierte Peptid codieren, mit einem Oligonukleotid, welches ein Targeting-Peptid codiert, das ein Ziel bindet, wobei das Enzym mit dem Targeting-Peptid die messbare enzymatische Aktivität besitzt und das Ziel bindet,
c) das Exprimieren des zielgerichteten Enzyms von dem Polynukleotid in einem Protein-Expressionssystem.

2. Verfahren gemäß Anspruch 1, wobei das Ziel ein Molekül ist, welches mit der Außenoberfläche einer Zelle assoziiert ist.

3. Verfahren gemäß Anspruch 2, wobei das Molekül aus der Gruppe gewählt ist, die aus einem Protein, einem Peptid, einer Nukleinsäure, einem Kohlenhydrat, einem Lipid, einem Polysaccharid, einem Glykoprotein, einem Hormon, einem Rezeptor, einem Antigen, einem Antikörper, einer toxischen Substanz, einem Metabolit, einem Inhibitor, einem Arzneistoff, einem Farbstoff, einem Nährstoff, einem Wachstumsfaktor und einem Molekül, das Teil der extrazellulären Matrix ist, besteht.

4. Verfahren gemäß Anspruch 2 oder Anspruch 3, wobei die Zelle eine erkrankte Zelle ist.

5. Verfahren gemäß Anspruch 4, wobei die erkrankte Zelle einer Krebszelle oder eine infizierte Zelle ist.

6. Verfahren gemäß mindestens einem der vorangehenden Ansprüche, ferner umfassend die Herstellung des Polynukleotids von Schritt (a) durch Einfügen eines Oligonukleotids, das das Peptid codiert, in ein Polynukleotid, das das entsprechende nicht modifizierte Enzym codiert.

7. Verfahren gemäß Anspruch 6, wobei das Oligonukleotid, das das Peptid codiert, in das Polynukleotid, das das entsprechende nicht modifizierte Enzym codiert, an einer zufälligen Position eingefügt wird.

8. Verfahren gemäß Anspruch 1 bis 5, wobei das Polynukleotid, das das modifizierte Enzym mit einer messbaren enzymatischen Aktivität codiert, aus einer Bibliothek von Polynukleotiden, die modifizierte Enzyme codieren, gewählt wird, wobei die Bibliothek Polynukleotide umfasst, welche modifizierte Enzyme codieren, die ein eingefügtes Peptid aufweisen, das ein entsprechendes nicht modifiziertes Enzym nicht hat, und wobei das nicht modifizierte Enzym die messbare enzymatische Aktivität besitzt.

9. Verfahren gemäß Anspruch 8, ferner umfassend die Herstellung der Bibliothek von Polynukleotiden, welche die modifizierten Enzyme codieren, durch Einfügen eines Oligonukleotids, das ein Peptid codiert, in das Polynukleotid, welches das nicht modifizierte Enzym codiert.

10. Verfahren gemäß den Ansprüchen 1 bis 5, ferner umfassend die Wiederholung der Schritte (a) und (b) dergestalt, das ein Enzym hergestellt wird, welches die messbare Enzymaktivität aufweist und eine Vielzahl von Targeting-Peptiden besitzt.

11. Verfahren gemäß Anspruch 10, wobei zwei oder mehrere der Vielzahl von Targeting-Peptiden das gleiche Ziel binden.

12. Verfahren gemäß Anspruch 10, wobei zwei oder mehrere der Vielzahl von Targeting-Peptiden unterschiedliche Ziele binden.

13. Verfahren zur Herstellung eines Polynukleotids, das ein zielgerichtetes Enzym codiert, wobei das Verfahren folgendes umfasst:
a) das Selektieren eines Polynukleotids, das ein modifiziertes Enzym codiert, wobei in das modifizierte Enzym im Vergleich zu einem entsprechenden nicht modifizierten Enzyms ein Peptid eingefügt ist, wobei das nicht modifizierte Enzym und das selektierte modifizierte Enzym eine messbare enzymatische Aktivität aufweisen, und
b) das Ersetzen der Nukleotide, die das in das Polynukleotid inserierte Peptid codieren, mit einem Oligonukleotid, welches ein Targeting-Peptid codiert, das ein Ziel bindet, wobei das Enzym mit dem Targeting-Peptid die messbare enzymatische Aktivität besitzt und das Ziel bindet

14. Verfahren gemäß Anspruch 13, wobei das Ziel ein Molekül ist, welches mit der Außenoberfläche einer Zelle assoziiert ist.

15. Verfahren gemäß Anspruch 14, wobei das Molekül aus der Gruppe gewählt ist, die aus einem Protein, einem Peptid, einer Nukleinsäure, einem Kohlenhydrat, einem Lipid, einem Polysaccharid, einem Glykoprotein, einem Hormon, einem Rezeptor, einem Antigen, einem Antikörper, einer toxischen Substanz, einem Metabolit, einem Inhibitor, einem Arzneistoff, einem Farbstoff, einem Nährstoff, einem Wachstumsfaktor und einem Molekül, das Teil der extrazellulären Matrix ist, besteht.

16. Verfahren gemäß Anspruch 14 oder Anspruch 15, wobei die Zelle eine erkrankte Zelle ist.

17. Verfahren gemäß Anspruch 16, wobei die erkrankte Zelle einer Krebszelle oder eine infizierte Zelle ist.

## Revendications

1. Procédé de fabrication d'une enzyme ciblée, comprenant :
a) la sélection d'un polynucléotide codant pour une enzyme modifiée, l'enzyme modifiée ayant un peptide inséré dans ledit polynucléotide par rapport à une enzyme non-modifiée correspondante, dans laquelle l'enzyme non-modifiée et l'enzyme modifiée sélectionnée ont une activité enzymatique mesurable, et
b) le remplacement des nucléotides codant pour le peptide inséré dans le polynucléotide par un oligonucléotide codant pour un peptide ciblant qui se lie à une cible, dans lequel l'enzyme avec le peptide de ciblage a l'activité enzymatique mesurable et se lie à la cible,
c) 1' expression de l'enzyme ciblée du polynucléotide dans un système d'expression des protéines.

2. Procédé de le revendication 1, dans lequel la cible est une molécule associée à la surface extérieure d'une cellule.

3. Procédé de la revendication 2, dans lequel la molécule est sélectionnée dans le groupe consistant en une protéine, un peptide, un acide nucléique, un glucide, un lipide, un polysaccharide, une glycoprotéine, une hormone, un récepteur, un antigène, un anticorps, une substance toxique, un métabolite, un inhibiteur, un médicament, un colorant, un nutriment, un facteur de croissance et une molécule qui fait partie de la matrice extracellulaire.

4. Procédé de la revendication 2 ou de la revendication 3, dans lequel 1a cellule est une cellule malade.

5. Procédé de la revendication 4, dans lequel la cellule malade est une cellule cancéreuse ou une cellule infectée.

6. Procédé de l'une quelconque des revendications précédentes, comprenant en outre 1a fabrication d'un polynucléotide de l'étape (a) par insertion d'un oligonucléotide codant pour ledit peptide dans un polynucléotide codant pour ladite enzyme non-modifiée correspondante.

7. Procédé de la revendication 6, dans lequel l'oligonucléotide codant pour ledit peptide est inséré dans le polynucléotide codant pour ladite enzyme non-modifiée correspondante à une position aléatoire.

8. Procédé des revendications 1 à 5, dans lequel le polynucléotide codant pour l'enzyme modifiée ayant une activité enzymatique mesurable est sélectionnée dans une bibliothèque de polynucléotides codant pour les enzymes modifiées, ladite bibliothèque comprenant les polynucléotides codant pour les enzymes modifiées qui ont un peptide inséré, qu'une enzyme non-modifiée correspondante n'a pas, et ladite enzyme non-modifiée ayant l'activité enzymatique mesurable.

9. Procédé de la revendication 8 comprenant en outre la fabrication de la bibliothèque de polynucléotides codant pour les enzymes modifiées par insertion d'un oligonucléotide codant pour un peptide dans le polynucléotide codant pour l'enzyme non-modifiée.

10. Procédé des revendications 1 à 5, comprenant en outre la répétition des étapes (a) et (b) de telle façon qu'une enzyme est produite, laquelle possède l'activité enzymatique mesurable et possède une pluralité de peptides de ciblage.

11. Procédé de la revendication 10, dans lequel deux peptides de ciblage ou plus de la pluralité de peptides de ciblage se lient à la même cible.

12. Procédé de la revendication 10, dans lequel deux peptides de ciblage ou plus de la pluralité de peptides de ciblage se lient à de cibles différentes.

13. Procédé de fabrication d'un polynucléotide codant pour une enzyme ciblée, le procédé comprenant :
a) la sélection d'un polynucléotide codant pour une enzyme modifiée, l'enzyme modifiée ayant un peptide inséré dans ledit polynucléotide par rapport à une enzyme non-modifiée correspondante, dans laquelle l'enzyme non-modifiée et l'enzyme modifiée sélectionnée ont une activité enzymatique mesurable, et
b) le remplacement des nucléotides codant pour le peptide inséré dans le polynucléotide par un oligonucléotide codant pour un peptide de ciblage qui se lie à une cible, dans lequel l'enzyme avec le peptide de ciblage a l'activité enzymatique mesurable et se lie à la cible.

14. Procédé de la revendication 13, dans lequel la cible est une molécule associée à la surface extérieure d'une cellule.

15. Procédé de la revendication 14, dans lequel la molécule est sélectionnée dans le groupe consistant en une protéine, un peptide, un acide nucléique, un glucide, un lipide, un polysaccharide, une glycoprotéine, une hormone, un récepteur, un antigène, un anticorps, une substance toxique, un métabolite, un inhibiteur, un médicament, un colorant, un nutriment, un facteur de croissance et une molécule qui fait partie de la matrice extracellulaire.

16. Procédé de la revendication 14 ou de la revendication 15, dans lequel la cellule est une cellule malade.

17. Procédé de 1a revendication 16, dans lequel la cellule malade est une cellule cancéreuse ou une cellule infectée.


### SEQUENCE LISTING

<110> Genencor International, Inc.
<120> Methods and Compositions for Grafting
   Functional Loops into a Protein
<130> SJK/FP6265169
<140> EP 03724564.4
   <141> 2002-06-12
<150> PCT/US03/15017
   <151> 2002-06-12
<150> US 10/170,387
   <151> 2002-06-12
<150> US 10/022,073
   <151> 2001-12-13
<150> US 10/022,097
   <151> 2001-12-13
<150> US 60/255,774
   <151> 2000-12-14
<150> US 60/279,609
   <151> 2001-03-28
<150> US 60/348,570
   <151> 2001-10-26
<160> 36
<170> FastSEQ for Windows Version 4.0
<210> 1
   <211> 8
   <212> PRT
   <213> Artificial Sequence
<220>
   <223> targeted beta-lactamase
<400> 1
   Arg Leu Tyr Ala Asn Ala Ser Ile
   1 5
<210> 2
   <211> 4
   <212> PRT
   <213> Artificial Sequence
<220>
   <223> targeted beta-lactamase
<221> VARIANT
   <222> (1)...(4)
   <223> Xaa = Any Amino Acid
<400> 2
   Lys Thr Xaa Ser
   1
<210> 3
   <211> 8
   <212> PRT
   <213> Artificial Sequence
<220>
   <223> targeted beta-lactamase
<400> 3
   Val His Lys Thr Gly Ser Thr Gly
   1 5
<210> 4
   <211> 50
   <212> DNA
   <213> Artificial Sequence
<220>
   <223> primer
<400> 4
   gggcccggac atccaaagct tgtcgacagg aagcggaaca cgtagaaagc 50
<210> 5
   <211> 50
   <212> DNA
   <213> Artificial Sequence
<220>
   <223> primer
<400> 5
   aagctttgga tgtccgggcc cgaattcgtg tgaaattgtt atccgctcac 50
<210> 6
   <211> 88
   <212> DNA
   <213> Artificial Sequence
<220>
   <223> primer
<221> misc_feature
   <222> (1)...(88)
   <223> n = A,T,C or G
<400> 6
   ttccaggcat ggcggtggcc gttatttatn nsnnsnnsnn snnsnnsnns nnsaaaccgc 60
   actattacac atttggcaag gccgacat 88
<210> 7
   <211> 88
   <212> DNA
   <213> Artificial Sequence
<220>
   <223> primer
<221> misc_feature
   <222> (1)...(88)
   <223> n = A,T,C or G
<400> 7
   cgcgatgtcg gccttgccaa atgtgtaata gtgcggttts nnsnnsnnsn nsnnsnnsnn 60
   snnataaata acggccaccg ccatgcct 88
<210> 8
   <211> 74
   <212> DNA
   <213> Artificial Sequence
<220>
   <223> primer
<400> 8
   ctaggtcttc tactagttta attgtcttag tcgtagctcc atctgcagtt gaagactctc 60
   tactggcggg tttg 74
<210> 9
   <211> 77
   <212> DNA
   <213> Artificial Sequence
<220>
   <223> primer
<221> misc_feature
   <222> (1)...(77)
   <223> n = A,T,C or G
<400> 9
   cgcttgcgcc gttgcccgtg gcagaagtga atnnsnnsnn snnsnnsnns nnsnnstcct 60
   gggtccataa aactggc 77
<210> 10
   <211> 77
   <212> DNA
   <213> Artificial Sequence
<220>
   <223> primer
<221> misc_feature
   <222> (1)...(77)
   <223> n = A,T,C or G
<400> 10
   tagagccagt tttatggacc caggasnnsn nsnnsnnsnn snnsnnsnna ttcacttctg 60
   ccacgggcaa cggcgca 77
<210> 11
   <211> 95
   <212> DNA
   <213> Artificial Sequence
<220>
   <223> primer
<221> misc_feature
   <222> (1)...(95)
   <223> n = A,T,C or G
<400> 11
   cgcttgcgcc gttgcccgtg gcagaagtga atnnsnnsnn snnsnnsnns nnsnnsnnsn 60
   nsnnsnnsnn snnstcctgg gtccataaaa ctggc 95
<210> 12
   <211> 95
   <212> DNA
   <213> Artificial Sequence
<220>
   <223> primer
<221> misc_feature
   <222> (1) ... (95)
   <223> n = A,T,C or G
<400> 12
   tagagccagt tttatggacc caggasnnsn nsnnsnnsnn snnsnnsnns nnsnnsnnsn 60
   nsnnsnnatt cacttctgcc acgggcaacg gcgca 95
<210> 13
   <211> 95
   <212> DNA
   <213> Artificial Sequence
<220>
   <223> primer
<221> misc_feature
   <222> (1) ... (95)
   <223> n = A,T,C or G
<400> 13
   gcgttgcgcc gttgcccgtg gcagaagtga atsngdhcsn gdhcsngdhc aagdhcsngd 60
   hcsngdhcsn gdhctcctgg gtccataaaa ctggc 95
<210> 14
   <211> 28
   <212> DNA
   <213> Artificial Sequence
<220>
   <223> primer
<400> 14
   attcacttct gccacgggca acggcgca 28
<210> 15
   <211> 25
   <212> DNA
   <213> Artificial Sequence
<220>
   <223> primer
<400> 15
   tagagccagt tttatggacc cagga 25
<210> 16
   <211> 84
   <212> DNA
   <213> Artificial Sequence
<220>
   <223> primer
<400> 16
   tggcccgcgg ccgctaattg tcttaggcgg atgccatgtg cagtactaga agacggcgta 60
   tcgggtcaat gtatcagggt ctcg 84
<210> 17
   <211> 26
   <212> DNA
   <213> Artificial Sequence
<220>
   <223> primer
<400> 17
   agacaattag cggccgcggg ccatgt 26
<210> 18
   <211> 28
   <212> DNA
   <213> Artificial Sequence
<220>
   <223> primer
<400> 18
   cagccgagac cctgatacat tgacccga 28
<210> 19
   <211> 68
   <212> DNA
   <213> Artificial Sequence
<220>
   <223> primer
<221> misc_feature
   <222> (1) ... (68)
   <223> n = A,T,C or G
<400> 19
   tggccccgga gnnsnnsnns nnsnnsnnsc ttaagcaggg catcgcgctg gcgcagtcgc      60
   gctactgg                                                              68
<210> 20
   <211> 75
   <212> DNA
   <213> Artificial Sequence
<220>
   <223> primer
<221> misc_feature
   <222> (1) ... (75)
   <223> n = A,T,C or G
<400> 20
   tacgccagta gcgcgactgc gccagcgcga tgccctgctt aagsnnsnns nnsnnsnnsn      60
   nctccggggc catgt                                                      75
<210> 21
   <211> 80
   <212> DNA
   <213> Artificial Sequence
<220>
   <223> primer
<221> misc_feature
   <222> (1) ... (80)
   <223> n = A,T,C or G
<400> 21
   tggccccgga gnnsnnsnns nnsnnsnnsn nsnnsnnsnn scttaagcag ggcatcgcgc      60
   tggcgcagtc gcgctactgg                                                 80
<210> 22
   <211> 87
   <212> DNA
   <213> Artificial Sequence
<220>
   <223> primer
<221> misc_feature
   <222> (1) ... (87)
   <223> n = A,T,C or G
<400> 22
   tacgccagta gcgcgactgc gccagcgcga tgccctgctt aagsnnsnns nnsnnsnnsn      60
   nsnnsnnsnn snnctccggg gccatgt                                           87
<210> 23
   <211> 76
   <212> DNA
   <213> Artificial Sequence
<220>
   <223> synthetic oligonucleotide
<400> 23
   gtgttccagg tcttctacta gtttaattgt cttaggcgga tgccatgtgc tcgtagctcc      60
   atctgcagtt gaagac                                                       76
<210> 24
   <211> 36
   <212> PRT
   <213> Artificial Sequence
<220>
   <223> beta-lactamase wild-type
<221> VARIANT
   <222> (1)...(36)
   <223> Xaa = Any Amino Acid
<400> 24
   Lys Val Ala Leu Ala Pro Leu Pro Val Ala Glu Val Asn Pro Pro Ala
    1               5                  10                  15
   Pro Pro Val Lys Ala Ser Trp Val His Lys Thr Gly Ser Thr Gly Gly
               20                  25                  30
   Phe Gly Ser Xaa
           35
<210> 25
   <211> 42
   <212> PRT
   <213> Artificial Sequence
<220>
   <223> library sequence
<221> VARIANT
   <222> (1)...(42)
   <223> Xaa = Any Amino Acid
<400> 25
   Lys Val Ala Leu Ala Pro Leu Pro Val Ala Glu Val Asn Glu Tyr Asp
   1 5 10 15
   Arg Arg Leu Asp Ala Ser Leu Cys Phe Val Lys Ser Trp Val His Lys
   20 25 30
   Thr Gly Ser Thr Gly Gly Phe Gly Ser Xaa
   35 40
<210> 26
   <211> 42
   <212> PRT
   <213> Artificial Sequence
<220>
   <223> library sequence
<221> VARIANT
   <222> (1) ... (42)
   <223> Xaa = Any Amino Acid
<400> 26
   Lys Val Ala Leu Ala Pro Leu Pro Val Ala Glu Val Asn Glu Gln Gln
   1 5 10 15
   Glu Glu Glu Ala Gly Thr Ser Lys Val Gly Pro Ser Trp Val His Lys
   20 25 30
   Thr Gly Ser Thr Gly Gly Phe Gly Ser Xaa
   35 40
<210> 27
   <211> 42
   <212> PRT
   <213> Artificial Sequence
<220>
   <223> library sequence
<221> VARIANT
   <222> (1)...(42)
   <223> Xaa = Any Amino Acid
<400> 27
   Lys Val Ala Leu Ala Pro Leu Pro Val Ala Glu Val Asn Gln Gly Thr
   1 5 10 15
   Glu Leu Arg Phe Lys Leu Lys Leu Lys Arg Glu Ser Trp Val His Lys
   20 25 30
   Thr Gly Ser Thr Gly Gly Phe Gly Ser Xaa
   35 40
<210> 28
   <211> 42
   <212> PRT
   <213> Artificial Sequence
<220>
   <223> library sequence
<221> VARIANT
   <222> (1)...(42)
   <223> Xaa = Any Amino Acid
<400> 28
   Lys Val Ala Leu Ala Pro Leu Pro Val Ala Glu Val Asn Arg Gly Leu
    1               5                  10                  15
   Pro Thr Trp Thr Ala Leu Val Glu Lys Pro Gly Ser Trp Val His Lys
               20                  25                  30
   Thr Gly Ser Thr Gly Gly Phe Gly Ser Xaa
           35                  40
<210> 29
   <211> 42
   <212> PRT
   <213> Artificial Sequence
<220>
   <223> library sequence
<221> VARIANT
   <222> (1)...(42)
   <223> Xaa = Any Amino Acid
<400> 29
   Lys Val Ala Leu Ala Pro Leu Pro Val Ala Glu Val Asn Ala Ile Arg
    1               5                  10                  15
   Val Asp Leu Gly Pro Ser Ser Arg Ser Arg Arg Ser Trp Val His Lys
               20                  25                  30
   Thr Gly Ser Thr Gly Gly Phe Gly Ser Xaa
           35                  40
<210> 30
   <211> 42
   <212> PRT
   <213> Artificial Sequence
<220>
   <223> library sequence
<221> VARIANT
   <222> (1) ... (42)
   <223> Xaa = Any Amino Acid
<400> 30
   Lys Val Ala Leu Ala Pro Leu Pro Val Ala Glu Val Asn Ala Thr Asn
    1               5                  10                  15
   Thr Thr Ser Asp Glu Val Val Gly Thr Gln Lys Ser Trp Val His Lys
               20                  25                  30
   Thr Gly Ser Thr Gly Gly Phe Gly Ser Xaa
           35                  40
<210> 31
   <211> 42
   <212> PRT
   <213> Artificial Sequence
<220>
   <223> library sequence
<221> VARIANT
   <222> (1) ... (42)
   <223> Xaa = Any Amino Acid
<400> 31
   Lys Val Ala Leu Ala Pro Leu Pro Val Ala Glu Val Asn Tyr Thr Ser
    1               5                  10                  15
   Val Gly Ala Gly Trp Arg Ala Gln Ala Val Gly Ser Trp Val His Lys
               20                  25                  30
   Thr Gly Ser Thr Gly Gly Phe Gly Ser Xaa
           35                  40
<210> 32
   <211> 42
   <212> PRT
   <213> Artificial Sequence
<220>
   <223> library sequence
<221> VARIANT
   <222> (1) ... (42)
   <223> Xaa = Any Amino Acid
<400> 32
   Lys Val Ala Leu Ala Pro Leu Pro Val Ala Glu Val Asn Gly His Arg
    1               5                  10                  15
   Val Val Pro Ser Tyr Leu Val Arg His Asp Ser Ser Trp Val His Lys
               20                  25                  30
   Thr Gly Ser Thr Gly Gly Phe Gly Ser Xaa
           35                  40
<210> 33
   <211> 42
   <212> PRT
   <213> Artificial Sequence
<220>
   <223> library sequence
<221> VARIANT
   <222> (1) ... (42)
   <223> Xaa = Any Amino Acid
<400> 33
   Lys Val Ala Leu Ala Pro Leu Pro Val Ala Glu Val Asn Gln Thr Leu
    1              5                  10                  15
   Asn Thr Ser Thr Ile Met Pro Arg Ser Pro His Ser Trp Val His Lys
               20                  25                  30
   Thr Gly Ser Thr Gly Gly Phe Gly Ser Xaa
           35                  40
<210> 34
   <211> 42
   <212> PRT
   <213> Artificial Sequence
<220>
   <223> library sequence
<221> VARIANT
   <222> (1)...(42)
   <223> Xaa = Any Amino Acid
<400> 34
   Lys Val Ala Leu Ala Pro Leu Pro Val Ala Glu Val Asn Gly Gly Arg
    1              5                  10                  15
   Lys Asp Gly Trp Pro Arg Gln Gly Lys Glu Gly Ser Trp Val His Lys
               20                  25                  30
   Thr Gly Ser Thr Gly Gly Phe Gly Ser Xaa
           35                  40
<210> 35
   <211> 361
   <212> PRT
   <213> Enterobacter cloacae
<400> 35
   Thr Pro Val Ser Glu Lys Gln Leu Ala Glu Val Val Ala Asn Thr Ile
   1 5 10 15
   Thr Pro Leu Met Lys Ala Gln Ser Val Pro Gly Met Ala Val Ala Val
   20 25 30
   Ile Tyr Gln Gly Lys Pro His Tyr Tyr Thr Phe Gly Lys Ala Asp Ile
   35 40 45
   Ala Ala Asn Lys Pro Val Thr Pro Gln Thr Leu Phe Glu Leu Gly Ser
   50 55 60
   Ile Ser Lys Thr Phe Thr Gly Val Leu Gly Gly Asp Ala Ile Ala Arg
   65 70 75 80
   Gly Glu Ile Ser Leu Asp Asp Ala Val Thr Arg Tyr Trp Pro Gln Leu
   85 90 95
   Thr Gly Lys Gln Trp Gln Gly Ile Arg Met Leu Asp Leu Ala Thr Tyr
   100 105 110
   Thr Ala Gly Gly Leu Pro Leu Gln Val Pro Asp Glu Val Thr Asp Asn
   115 120 125
   Ala Ser Leu Leu Arg Phe Tyr Gln Asn Trp Gln Pro Gln Trp Lys Pro
   130 135 140
   Gly Thr Thr Arg Leu Tyr Ala Asn Ala Ser Ile Leu Gly Phe Gly Ala
   145 150 155 160
   Leu Ala Val Lys Pro Ser Gly Met Pro Tyr Glu Gln Ala Met Thr Thr
   165 170 175
   Arg Val Leu Lys Pro Leu Lys Leu Asp His Thr Trp Ile Asn Val Pro
   180 185 190
   Lys Ala Glu Glu Ala His Tyr Ala Trp Gly Tyr Arg Asp Gly Lys Ala
   195 200 205
   Val Arg Val Ser Pro Gly Met Leu Asp Ala Gln Ala Tyr Gly Val Lys
   210 215 220
   Thr Asn Val Gln Asp Met Ala Asn Trp Val Met Ala Asn Met Ala Pro
   225 230 235 240
   Glu Asn Val Ala Asp Ala Ser Leu Lys Gln Gly Ile Ala Leu Ala Gln
   245 250 255
   Ser Arg Tyr Trp Arg Ile Gly Ser Met Tyr Gln Gly Leu Gly Trp Glu
   260 265 270
   Met Leu Asn Trp Pro Val Glu Ala Asn Thr Val Val Glu Gly Ser Asp
   275 280 285
   Ser Lys Val Ala Leu Ala Pro Leu Pro Val Ala Glu Val Asn Pro Pro
   290 295 300
   Ala Pro Pro Val Lys Ala Ser Trp Val His Lys Thr Gly Ser Thr Gly
   305 310 315 320
   Gly Phe Gly Ser Tyr Val Ala Phe Ile Pro Glu Lys Gln Ile Gly Ile
   325 330 335
   Val Met Leu Ala Asn Thr Ser Tyr Pro Asn Pro Ala Arg Val Glu Ala
   340 345 350
   Ala Tyr His Ile Leu Glu Ala Leu Gln
   355 360
<210> 36
   <211> 14
   <212> PRT
   <213> Artificial Sequence
<220>
   <223> loop library
<221> VARIANT
   <222> 1, 3, 5, 9, 11, 13
   <223> Xaa = Phe, Ile, Val, Ser, Thr, Ala, Tyr, Asn or Asp
<221> VARIANT
   <222> 2, 4, 6, 8, 10, 12, 14
   <223> Xaa = Val, Ala, Glu, Gly, Leu, Pro, Gln or Arg
<400> 36
   Xaa Xaa Xaa Xaa Xaa Xaa Lys Xaa Xaa Xaa Xaa Xaa Xaa Xaa
    1               5                  10